# EUROPEAN PATENT APPLICATION

(11) **EP 4 825 871 A1**
(43) Date of publication of application: **21.10.2026**
(21) Application number: 25170845.9
(22) Date of filing: 15.04.2025
(51) Int. Cl.: C07K 16/08, C07K 1/22, C07K 16/18, C07K 16/28, C07K 17/00, C12N 7/02

(54) **ALKALINE-STABLE ANTIBODIES**

(71) Applicant: Cytiva BioProcess R&D AB, 751 84 Uppsala (SE)
(72) Inventor: JONSSON, Andreas, 751 84 Uppsala (SE); LANDMARK, Fredrika, 751 84 Uppsala (SE); TIDEHOLM, Ellen, 751 84 Uppsala (SE)
(74) Representative: Spjuth, Nora Sofia

(57) **Abstract**

The disclosure provides a polypeptide, in particular a single domain antibody (sdAb) such as a VHH, comprising an antibody heavy chain variable domain (VH) variant comprising a plurality of antigen-binding regions and a framework comprising framework region 1, framework region 2 and framework region 3 (FWR3), wherein the VH variant has an amino acid sequence in which criterion viii) is fulfilled, and in addition at least eight of the following criteria i) to xi) are fulfilled:
i) the residue in Kabat position 19 is R, S, K or T;
ii) the residue in Kabat position 23 is T, V, A or S;
iii) the residue in Kabat position 24 is I, V, A, R, N, D, Q, E, H, L, K, F, T, Y or S;
iv) the residue in Kabat position 40 is selected from R, I, T or K;
v) the residue in Kabat position 43 is R, K, Q, E or G;
vi) the residue in Kabat position 44 is E, Q, A, D or G;
vii) the residue in Kabat position 45 is R, I or L;
viii) the residue in Kabat position 71 is F, H, K, Q, Y or R;
ix) the residue in Kabat position 76 is N or Q;
x) the residue in Kabat position 79 is Y, W or F; and
xi) the residue in Kabat position 89 is V, or I.

The VH variants have improved properties, such as enhanced binding properties (selectivity, affinity) and in particular alkaline stability, and are useful as a scaffold for an affinity binder for *in vitro* applications, such as detection, analysis or separation of an analyte based on affinity interactions.

## Description

### TECHNICAL FIELD

The present invention relates to polypeptides based on antibody heavy chain variable domains (VH) and their use as a scaffold for affinity ligands, and to adsorbent materials comprising such polypeptides. The invention also relates to a method of affinity separation.

### BACKGROUND

Binding events between molecules based on affinity, where a ligand and a target entity interact in a "lock-key" fashion, are utilized in numerous therapeutic and non-therapeutic applications. Non-therapeutic applications include e.g. *in vitro* detection, analysis and separation of a target or analyte present in a sample.

An affinity ligand is capable of selectively and reversibly binding the target entity. General examples of interactions are e.g., enzyme-substrate interaction, biotin-avidin interaction, antibody-antigen interaction, etc. In many applications, the affinity ligand can be immobilized on a support and used for capture of the target entity, e.g. for the purpose of detection or purification.

Affinity chromatography is a specific mode of chromatography which utilizes the selectivity of the interaction between the ligand and the target entity. The affinity ligand is immobilized on a chromatographic support material, also referred to as the stationary phase. When contacted with a sample containing the target entity under binding conditions, the ligand selectively binds the target entity, while other species of the sample can be washed away. The captured target entity can then be eluted, typically by changing buffer conditions, such as conductivity or salt concentration, and/or pH. After subsequent cleaning and regeneration of the chromatography material, the chromatography material can be used for a new cycle of affinity purification. Affinity chromatography can potentially yield a target entity of very high purity.

Affinity chromatography is frequently used in the purification of biomolecules, including biopharmaceuticals and such as monoclonal antibodies, antibody fragments and recombinant proteins, as well as nucleic acids and viral particles for use in vaccine production or gene therapy, for instance.

Preparative chromatography, as well as many analytical applications using a support having the affinity ligand immobilized thereon, require comprehensive attention to definite removal of contaminants from the support between capture/purification cycles. Such contaminants can for example be non-eluted molecules adsorbed to the support or stationary phase, such as non-desired biomolecules or microorganisms, including for example proteins, carbohydrates, lipids, bacteria and viruses. In affinity chromatography, the removal of such contaminants from the support is usually performed after a first elution of the desired product in order to regenerate the stationary phase before subsequent use. Such removal usually involves a procedure known as cleaning-in-place (CIP), wherein agents capable of eluting contaminants from the stationary phase are used. One such class of agents often used is alkaline solutions that are passed over said stationary phase. At present the most extensively used cleaning and sanitizing agent is NaOH, and the concentration thereof can range from 0.05 M up to e.g. 1 M, depending on the degree and nature of contamination. This strategy is associated with exposing the stationary phase to solutions with pH values of about 13 and above. For many affinity chromatography materials containing proteinaceous affinity ligands such alkaline environment is a very harsh condition and consequently results in decreased capacities owing to instability of the ligand to the high pH involved.

Affinity reagents based on staphylococcal protein A (SpA), which is the most widely used affinity medium for isolation of immunoglobulins and their fragments, have been developed to better withstand alkaline conditions (see for example WO2003080655A1 and WO2016/079033A1). Meanwhile, there is a great need in the art for improved affinity ligands capable of binding to biomolecules other than immunoglobulins.

### SUMMARY OF THE INVENTION

It is an object of the invention to overcome or at least partly alleviate drawbacks of the prior art. Accordingly, it is an object of the invention to provide a scaffold useful for development of new affinity ligands, which scaffold give rise to enhanced properties of the affinity ligand, such as improved binding kinetics and selectivity to target, increased alkaline stability, increased thermal stability, enhanced expression, solubility and purification when manufacturing the ligand, as well as enhanced elution properties when using the ligand for target separation purposes.

These and other objects are achieved by a polypeptide comprising a heavy chain variable domain (VH) variant in which at least one framework region, such as framework region 3 (FWR3), is modified to provide improved properties, such as affinity and alkaline stability.

In a first aspect, the invention provides a polypeptide comprising a heavy chain variable domain (VH) variant comprising a plurality of antigen-binding regions (flexible loops, i.e. CDRs) and a framework comprising framework region 1 (FWR1), framework region 2 (FWR2) and framework region 3 (FWR3), wherein the VH variant has an amino acid sequence comprising a plurality of mutations compared to other framework sequences, for example an existing framework, such as i) a native camelid framework as defined in any one of SEQ ID NO: 157, SEQ ID NO: 155 or SEQ ID NO: 157, or ii) a synthetic framework as defined in any one of SEQ ID NO: 72 or SEQ ID NO:73, in which at least eight of the following criteria i) to xi) are fulfilled:
i) the residue in Kabat position 19 is R, S, K or T;
ii) the residue in Kabat position 23 is T, V, A or S, preferably T;
iii) the residue in Kabat position 24 is I, V, A, R, N, D, Q, E, H, L, K, F, T, Y or S;
iv) the residue in Kabat position 40 is selected from R, I, T or K, preferably R;
v) the residue in Kabat position 43 is R, K, Q, E or G, preferably R or K;
vi) the residue in Kabat position 44 is E, Q, A, D or G;
vii) the residue in Kabat position 45 is R, I or L, preferably R;
viii) the residue in Kabat position 71 is F, H, K, Q, Y or R;
ix) the residue in Kabat position 76 is N or Q;
x) the residue in Kabat position 79 is Y, W or F; and
xi) the residue in Kabat position 89 is V or I.

Optionally at least nine, such as ten, such as all, of the criteria i) to xi) are fulfilled. In some embodiments, the residue in Kabat position 71 is not R.

In a second aspect, there is provided a polypeptide comprising a heavy chain variable domain (VH) variant, comprising a plurality of antigen-binding regions and a framework comprising framework region 1 (FWR1), framework region 2 (FWR2) and framework region 3 (FWR3), wherein the VH variant has an amino acid sequence comprising a plurality of mutations compared to other framework sequences, such as i) a native camelid framework as defined in any one of SEQ ID NO: 152, SEQ ID NO: 155 or SEQ ID NO: 157, or ii) a synthetic framework as defined in any one of SEQ ID NO: 72 or SEQ ID NO:73 in which at least five of the following criteria i) to vii) are fulfilled:
i) the residue in Kabat position 24 is I, V, A, R, N, D, Q, E, H, L, K, F, T, Y or S;
ii) the residue in Kabat position 40 is selected from R, I, K, or T, preferably R;
iii) the residue in Kabat position 45 is R, I or L, preferably R;
iv) the residue in Kabat position 71 is F, H, K, Q, Y or R;
v) the residue in Kabat position 76 is N or Q;
vi) the residue in Kabat position 79 is Y, W or F; and
vii) the residue in Kabat position 89 is V or I.
Optionally, at least six, or all seven criteria i)-vii) may be fulfilled. In some embodiments, the residue in Kabat position 71 is not R.

A polypeptide of the present disclosure may satisfy at least eight of the criteria i)-xi) of the first aspect and at the same time at least five of the criteria i)-vii) of the second aspect.

In some embodiments, the residue in Kabat position 71 is F, H, K, Q, or Y and/or the residue in Kabat position 24 is I, V, A, R, N, D, Q, E, H, L, K, F, T, Y or S.

In the following, all references to a polypeptide of the present disclosure refers to both the first aspect and the second aspect alike, unless otherwise provided.

Optionally, each of FWR1, FWR2 and FWR3 may have at least 80 % identity to the amino acid sequence of a corresponding framework region of SEQ ID NO: 80 wherein for the purpose of determining sequence identity, the Kabat positions 14, 19, 23, 24, 40, 43, 44, 45,47, 60, 71, 76, 79, 82b and 89, and optionally position 6, are omitted.

The VH variant may be a single-domain antibody (sdAb) variant.

Furthermore, the amino acid sequence of the VH variant may also fulfil any one, such as more than one, such as all, of the following criteria:
the residue in Kabat position 6 is Q or E;
the residue in Kabat position 14 is A, S or T;
the residue in Kabat position 47 is F or L, preferably F;
the residue in Kabat position 60 is A, T, N, Q or S, preferably A or S;
the residue in Kabat position 82b is N, S, T or Q.

In particular, the amino acid sequence of the VH variant may comprise a phenylalanine (F) or leucine (L) residue in Kabat position 47. However, even in embodiments where the residue in Kabat position 47 is not limited to F or L, it is preferred that it is not G, S, T or Y.

In another aspect, there invention provides a multimeric polypeptide comprising at least two moieties, each moiety being a VH variant as defined herein, said moieties optionally being joined by a peptide linker.

In yet another aspect, a fusion protein is provided, comprising at least one polypeptide or multimer as defined herein, and at least one further polypeptide moiety.

In a further aspect the invention provides an adsorbent material comprising said polypeptide, multimer or fusion protein coupled to a solid support. The solid support may be selected from the group consisting of a particle, a bead, a fiber, a fibrous membrane, a filter, a sheet, a porous monolith, a chip, a plate and a well. The support may be a chromatography matrix.

In further aspects, the invention provides the use of said polypeptide, multimer or fusion protein as affinity ligand for capturing a target entity, as well as the use of the aforementioned adsorbent material for the separation of a target entity from other components of a sample. The target entity is an entity for which the VH variant has an affinity. Provided is also a method of separation, comprising the steps of:
(a) Providing an adsorbent material as described herein, wherein the VH variant of the polypeptide has a binding affinity for a target entity,
(b) Contacting the adsorbent material with a liquid sample comprising said target entity under conditions allowing the target entity to bind to the polypeptide,
(c) Optionally washing the adsorbent material,
(d) Eluting the target entity from the adsorbent material, and
(e) Cleaning the adsorbent material with a cleaning liquid.

The cleaning liquid is typically alkaline and may comprise from 0.05 to 0.5 M NaOH. Steps (a)-(e) may be repeated at least 10 times.

Advantageously, the affinity optimized and alkaline stable VH variant of the polypeptide can retain a high target binding capacity also after repeated or extended exposure to alkaline conditions.

### BRIEF DESCRIPTION OF THE DRAWINGS

These and other aspects of the present invention will now be described in more detail, with reference to the appended drawings showing embodiment(s) of the invention, in which:
Fig. 1 is a schematic illustration of the arrangement of framework regions and complementarity determining regions of a single-domain antibody.
Fig. 2a-c are schematic illustrations of various structures of polypeptides according to the present disclosure.
Fig. 3a-c are schematic illustrations of a fusion protein comprising at least one polypeptide as disclosed herein fused to another polypeptide moiety.
Fig. 4 is a graph showing the result of the alkaline stability study of polypeptides described in Example 2A.
Fig. 5 is a graph showing the result of the alkaline stability study of polypeptides described in Example 2B.
Fig. 6a-c show the target binding responses obtained for increasing number of cycles involving NaOH exposure for polypeptides capable of binding AAV9 (Fig. 6a), GFP (Fig. 6b) or EGFR (Fig. 6c), respectively (Example 4A). The responses were normalized to the response of the first cycle.
Fig. 7a-c show target binding responses obtained for increasing number of cycles involving NaOH exposure for fusion proteins capable of binding AAV9 (Fig. 7a), GFP (Fig. 7b) or EGFR (Fig. 7c), respectively (Example 4B). The responses were normalized to the response of the first cycle.
Fig. 8 is a graph showing the alkaline stability of exemplary polypeptides (Example 5).
Fig. 9 is a graph showing the alkaline stability of exemplary polypeptides and a commercially available affinity ligand (Example 6).
Fig. 10a is a chromatogram showing the elution peak from a chromatography column using a fusion protein according to embodiments of the present invention as affinity ligand immobilized on the chromatography matrix. Fig. 10b is a closer view of the elution peak.
Fig. 11 is a photograph of an SDS-PAGE gel showing the protein content of the eluate fractions from the chromatography run of Example 7.
Fig. 12 is a graph plotting the dynamic binding capacity as evaluated in Example 7.
Fig. 13 is a graph plotting the alkaline stabilities evaluated in Example 8.
Fig. 14 is a graph plotting and binding affinity and alkaline stability on a SA-chip of purified and biotinylated constructs according to Example 12.
Fig. 15 shows binding sensorgram of 250 nM GFP (15a) and graph of alkaline stability measured ZVH3 (15b) for GFP binding polypeptides according to Example 13.
Fig.16 shows binding sensorgram of 200 nM EGFR (16a), graph of alkaline stability ZVH3 measured (16b) and graph of alkaline stability target molecule measured (16c) for EGFR binding polypeptides according to Example 13.
Fig. 17 shows binding sensorgram of 2e12 VP/ml AAV9 (17a), graph of alkaline stability ZVH3 measured (17b) and graph of alkaline stability target molecule measured (17c) for AAV9 binding polypeptides according to Example 13.
Fig. 18 illustrates a tertiary structure of a single domain antibody of the present disclosure.

As illustrated in the figures, some features may be exaggerated for illustrative purposes and, thus, are provided to illustrate the general structures of embodiments of the present invention. Like reference numerals refer to like elements throughout.

### DEFINITIONS

As used herein, the terms "peptide" and "polypeptide" are used synonymously herein to refer to compounds formed of sequences of amino acids, without restriction as to size. "Protein" may be used to refer to the larger compounds of this class. Amino acid sequences are written left to right in the direction from the amino (N) to the carboxy I terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L ), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V). "Peptides" include any oligopeptide, polypeptide, gene product, expression product, or protein. A peptide is comprised of consecutive amino acids and encompasses naturally occurring or synthetic molecules. In addition, as used herein, the term "peptide" refers to amino acids joined to each other by peptide bonds or modified peptide bonds, e.g., peptide isosteres, etc. and may contain modified amino acids other than the 20 gene-encoded amino acids. The peptides can be modified by either natural processes, such as post-translational processing, or by chemical modification techniques which are well known in the art.

A "single-chain polypeptide" herein refers to a polypeptide that is formed of a single amino acid sequence, in which the amino acid residues are connected via peptide bonds. A single-chain polypeptide may, upon folding, additionally form other internal bonds such as disulfide bonds.

The expression "antigen-binding polypeptide" generally refers to a polypeptide that possesses at least one binding region, and typically at least two, such as three binding regions, such that the polypeptide has a binding affinity for a molecule (referred to as antigen or "target", i.e. "target-binding"). An antigen-binding polypeptide may have any protein structure, as long as there is a binding affinity for the antigen. However, some classes of antigen-binding polypeptides that have frequently been exploited and used as a basis for engineered antigen-binding polypeptides often have a scaffold or framework structure that is generally conserved between antigen-binding polypeptides of the same class, but which bind to different antigens. The binding regions of an antigen-binding molecule can typically be engineered or evolved to bind to a particular antigen, while the scaffold or framework structure remains essentially the same. Non-limiting examples of antigen-binding polypeptides include natural or engineered (i) antibodies, such as monoclonal antibodies, (ii) antibody fragments that contain the light chain and/or heavy chain variable region(s) with complementarity determining regions (CDRs), such as Fab (fragment antigen-binding), Fv (variable fragment), scFab (single chain antigen-binding fragment) and scFv (single chain variable fragment), iii) single-domain antibodies, and iv) polypeptides having a scaffold derived from bacteria, such as immunoglobulin-binding bacterial proteins (such as *Finegoldia magna* protein L, or staphylococcal protein A and protein G) or domains thereof, including the wild types as well as variants in which one or more of the antigen binding regions have been engineered.

The expression "single-domain polypeptide" or "single-domain amino acid sequence" refers to a polypeptide which forms an individual protein domain, and which does not comprise any other individual protein domain. Examples of single-domain polypeptides include antibody fragments such as heavy chain variable domain (VH) and light chain variable domain (VL), single-domain antibodies (sdAb), albumin binding domain (ABD) and polypeptides derived from a bacterial protein domain, such as the A, B, C, D, E or Z domain of SpA, or protein domains of protein L. Single-domain antibodies are particularly contemplated. A single-domain polypeptide typically consists of a single-chain polypeptide. A single-domain polypeptide is in itself monomeric, but it may form part of a multimer, as described elsewhere herein. A single-domain polypeptide may be joined to another polypeptide, for example via a peptide bond or via a disulfide bond. Preferably, in the context of the present disclosure, an antigen-binding polypeptide may consist of a single polypeptide chain.

The term "single-domain antibody" refers to a variable domain which is or is derived from a heavy chain variable domain (VH) of an antibody devoid of light chains. Hence, a single domain antibody lacks antibody light chains entirely, including the light chain variable domain (VL). A single-domain antibody may also lack such structural features that are needed for a functional VH/VL interaction, and which are present in, e.g., the conventional VH of IgG1. Hence, a single domain antibody does not form part of a dimeric structure with an antibody light chain variable domain. Single-domain antibodies include antibodies naturally devoid of antibody light chains, such as sdAb derived from IgG2 or IgG3 of camelids (*Camelidae*), e.g. dromedary, camel, llama and alpaca, which are also referred to as VHH (heavy chain variable domain of a heavy chain antibody). VHH typically refers to the single domain of the antibody, while the single-domain antibody itself is referred to as VH, although VH and VHH sometimes are used interchangeably. Herein is included for reference polypeptides having such native frameworks, such as an AAV9-binding sdAb of SEQ ID NO: 152 (vh342), a GFP-binding sdAb of SEQ ID NO: 155 (vh60) and an EGFR-binding sdAb of SEQ ID NO 157 (vh166). Single domain antibodies naturally devoid of light chains also include so-called VNARs (variable new antigen receptors), which are antibodies derived from cartilaginous fishes, such as sharks. Alternatively, single domain antibodies may be synthetic variants based on an amino acid sequence of an antibody heavy chain variable domain from a species that naturally does not produce sdAb (e.g. cow, rat, mouse or rabbit) and which are modified e.g. with respect to amino acids in the natural VH/VL interaction of such antibodies, so as to mimic as an sdAb. Such modification may involve substitution of amino acids of the VH/VL interface region to increase the hydrophilicity or solubility.

Generally, terms such as "polypeptide", "protein", "antibody", "single-domain polypeptide", "single-domain antibody", etc. also include synthetic variants that are recombinantly produced and whose amino acid sequence may have been modified in relation to the amino acid sequence of a naturally occurring counterpart. Where such modified variants are particularly intended, the term "variant" may also be used. The term "wild type" or "wt" refers to the typical naturally occurring form, which may also be referred to as "native" or "non-modified" when compared to modified variants. Single-domain antibody variants of the present invention are synthetic, non-naturally occurring amino acid sequences.

Complementary determining regions (CDRs) are hypervariable regions of an antibody or part thereof which participate in binding to a target epitope. The CDRs are typically defined by their separate amino acid sequences, although in an antibody, the CDRs together form a three-dimensional binding site for the target antigen. A variable domain of an antibody heavy chain (VH) has three CDRs, referred to as CDR1, CDR2 and CDR3, as positioned from the N terminus of the polypeptide chain. The sequence length of CDRs may vary, and CDR1, CDR2 and especially CDR3 of a VH may be of different lengths.

The portions of an antibody VH not forming the CDRs are referred to as framework regions. The framework regions are typically four and referred to as framework region (FW) 1 to 4, as counted from the N terminus. The framework regions are responsible for the general secondary and tertiary structure of the domain and thus for positioning and orientation of the CDR regions. The framework regions may be referred to as a scaffold structure. Although part of the variable domain, the framework regions are less variable than the CDRs. Generally, amino acid sequence variations that do not significantly alter the secondary or tertiary structure of the framework may however be tolerated. Some parts or amino acid positions of the framework regions may be conserved. The general stability of the framework regions allows for a high degree of variation in the CDRs.

As used herein, the term "ligand" is a molecule that has a known or unknown affinity for a given entity. The term "ligand" may herein be used interchangeably with the terms "affinity ligand", "selective binding molecule", "selective binding partner", "capturing molecule" and "capturing agent". "Affinity ligand" refers to a moiety or molecule that binds reversibly and selectively or preferentially with high affinity to a target entity through a specific interaction with a binding site of the component. In the context of the present invention, an affinity ligand is a polypeptide, and may also be referred to as an "affinity protein". An affinity ligand may be immobilized to a solid support such as a resin.

The term "target entity" herein refers to an entity that forms a specific binding partner to the ligand, and may also be referred to as an "analyte". The analytes or target entities of interest according to the present disclosure may be any type of molecule suitable for affinity purification using the sdAbs of the present disclosure. Such analytes include viral particles, such as adeno-associated virus vectors, AAVs, in particular AAV9 and AAV8 vectors. Other target entities may be antibodies, such as the kappa (κ) and lambda (λ) light chains, or receptors, enzymes, small molecules, modifications on proteins etc., as disclosed herein.

As used herein, "affinity" in the context of polypeptides denotes the strength of interaction between two molecules, wherein at least one molecule is a polypeptide. The interaction is selective, i.e. discriminates between the affinity binding partner and other molecules present. Binding affinity is also expressed as the dissociation equilibrium constant (KD). In some aspects of the present disclosure, the binding affinity of the antigen-binding polypeptides to a target (antigen) is enhanced. The enhanced binding affinity may also increase the selectivity for the target, i.e. the preference of the binding polypeptide to the target compared to other molecules. The term "specificity", sometimes referred to as "selectivity," of the binding polypeptide for a target refers to a binding polypeptide that will bind to the target with high affinity, but typically not to other molecules, or with low affinity. A selective or specific binding polypeptide will not, or to a low extent, cross-react with other targets than the intended target. Thus, by binding "specifically" it is meant that the binding polypeptide binds to its target (e.g. AAV8 or AAV9 capsids) in a manner that can be distinguished from binding to non-target molecules, more particularly that the binding protein binds its target with greater binding affinity than with which it binds other molecules. That is, the binding polypeptide does not bind to other, non-target, molecules, or does not do so to an appreciable or significant degree, or binds with lower affinity to such other molecules than with which it binds its intended target.

The enhanced frameworks as presented herein are shown to affect binding properties, such as binding kinetics. With the term "binding kinetics" is referred to how quickly the sdAb binds to its target and the speed at which it dissociates from it. Binding kinetics measure the on-rate (kₒₙ) and the off-rate for the interaction (k_{off}) and from the binding kinetics the dissociation equilibrium constant (KD) can also be calculated.

As used herein, the term "binding capacity" refers to the capability of a ligand to bind the target molecules when the ligand is immobilized on a surface. The binding capacity of a ligand can differ depending on the type of surface on which it is immobilized, as well as ligand density. For the purpose of the present invention, the binding capacity can be measured by surface plasmon resonance (SPR) technology using e.g. a Biacore instrument as described in the present examples. The binding capacity for a certain concentration of analyte is influenced by the density of immobilized ligands on the surface. In the context of the present invention the binding capacity is preferably determined at a ligand density of at least 3000 response units (RU), such as in the range of from 3000-4000 RU.

The term "dynamic binding capacity" (abbreviated "DBC") of a chromatography column in the context of protein purification is the binding capacity under operating conditions, i.e., in a packed affinity chromatography column during sample application. The DBC of a chromatography resin is expressed as the amount of analyte that binds to the resin under given flow conditions before a significant breakthrough of unbound analyte occurs. DBC is determined by loading a sample containing a known concentration of the analyte and monitoring the flow-through. The analyte will bind to the resin to a certain break point before unbound analyte will flow through the column. The DBC can be determined on the breakthrough curve at a loss of, for example, 10% analyte. This is referred to as the QB10% value. A sample is applied to a chromatography resin column during a specific residence time and the dynamic binding capacity for each resin is calculated at 10% of the breakthrough capacity i.e., the amount of analyte sample that is loaded onto the column until the concentration of analyte in the column effluent is 10% of the analyte sample concentration in the feed. If the dynamic binding capacity for each resin is calculated at 80% of the breakthrough capacity, this is referred to as the QB80% value. The DBS may also be enhanced by the polypeptides of the disclosure.

The term "alkaline stability" as used herein, refers to a property of an antigen-binding polypeptide which relates to its ability to withstand alkali exposure without deleterious effect on structure and/or function of the polypeptide. For polypeptides capable of binding a target entity, the alkaline stability is determined based on the affinity for the target entity after exposure to NaOH. The alkaline stability of an antigen-binding polypeptide can be evaluated immobilizing the antigen-binding polypeptide on a support and measuring the target binding capacity before and after one or more cycles of exposure to NaOH, using methods described in the Examples below. In particular the evaluation can be made on an SPR chip onto which the antigen-binding polypeptide is immobilized by covalent coupling with thiol, N-hydroxysuccinimide, streptavidin-biotin binding, or another coupling that is alkali resistant in itself, at a ligand density corresponding to at least 1000 RU, and preferably at least 3000 RU, and for a predetermined analyte concentration. Often, the first alkaline cleaning cycle may have a unique, large impact on binding capacity, e.g. due to removal of non-covalently bound antigen-binding polypeptide from the support surface, and therefore the binding capacity is sometimes represented as normalized to the binding capacity measured after the first cycle of alkali exposure.

For example, "improved alkaline stability" can mean that an antigen-binding polypeptide can withstand a higher number of alkaline cleaning cycles, or the same number of cycles but with harsher alkali conditions, without a deterioration in target binding capacity. Alternatively, "improved alkaline stability" can mean that the polypeptide will retain a higher percentage of the binding capacity after an equal number of cleaning cycles using the same conditions. The alkaline stability may be particularly relevant for a polypeptide intended to be immobilized on a solid support. The alkali treatment may involve contact or incubation with 0.05-1 M NaOH, such as 0.1-0.5 M or 0.3-0.5 M NaOH for a time period of 5-15 minutes, such as 10 minutes (600 seconds) or about 10 minutes. Said treatment may be for example at 22 +/- 2 °C. An antigen-binding polypeptide showing improved alkaline stability as defined above and below may be referred to as "alkaline stable" or "alkali stable".

In the present context, an antigen-binding polypeptide is considered to be alkaline stable if, after at least 12 cleaning cycles, such as after 15 cleaning cycles, preferably after 20 cleaning cycles, the immobilized antigen-binding polypeptide retains at least 50 % of the target binding capacity compared to its binding capacity after the first cleaning cycle (i.e. disregarding the binding capacity of the first cycle, prior to the first alkali exposure event), where a cleaning cycle involves 600 s of exposure to at least 0.3 M NaOH, such as at least 0.5 M NaOH.

The enhanced properties of the antigen-binding polypeptides of the present disclosure, having enhanced framework regions, may relate to a number of properties, such as increased levels of expression when expressed in a bacterial host, increased ease of expression, enhanced elution properties, such as being eluted under mild pH conditions, and increased solubility.

The term "solid support" herein refers to a non-aqueous matrix of a solid phase material. Suitable solid phase materials include, but are not limited to, glass, silica (e.g., silica gel), polysaccharides (e.g., a polysaccharide matrix) such as agarose and cellulose, organic polymers such as polyacrylamide, methyl methacrylate, and polystyrenedivinylbenzene copolymers. The solid phase can be of porous or nonporous character and can be compressible or incompressible. For example, the solid phase can be a polymeric matrix or an agarose particle or bead. Preferred solid support materials will be physically and chemically resilient to the conditions employed in a purification process including pumping and cross-flow filtration, and temperatures, pH, and other aspects of the liquids employed.

The term "surface" herein means all external surfaces of a solid structure. In the case of a porous support, such as a bead used e.g. in the field of chromatography, the term "surface" includes outer surfaces as well as pore surfaces. In the case of a fibrous membrane, the term "surface" encompasses the external surface of individual fibers.

The term "separation matrix" is used herein to denote a material comprising a solid support to which one or more ligand(s) have been coupled. The ligand(s) are capable of binding target entities herein also called analytes, which are to be separated from their surroundings, e.g. a liquid sample, and/or which are to be separated from other components present in the liquid sample. A separation matrix may be employed in various contexts, including analytical assays and purification of target for analytical or preparative purposes. The type of support may be selected depending on the intended use.

A separation matrix may further comprise a compound which couples the ligand(s) to the support. The terms "spacer", "extender", and "surface extender" may be used to describe such a compound, as further described herein. The term "resin" is sometimes used for a separation matrix in this field. The terms "chromatography material" and "chromatography matrix" are used herein to denote a type of separation matrix. An affinity separation matrix is a separation matrix where the ligand is an affinity ligand.

The term "spacer" refers to a peptide or other chemical linkage or element that extends the structure of an entity. A spacer attached to a polypeptide, in particular an amino acid spacer, may be provided at the N terminus or the C terminus of the polypeptide. A spacer may connect a polypeptide to a solid support. Suitable spacers for coupling a polypeptide to a support may generally be any spacer used in the art to connect peptides, proteins or other organic molecules to a support. A spacer may also serve to connect two polypeptides or polypeptide domains. A spacer connecting two polypeptide moieties may also be referred to as a linker.

The terms "multimer" and "multimeric protein" as used herein refer to a protein comprising at least two repeating units of the single-domain antibody disclosed herein. Hence, the single domain antibody may be regarded as a monomeric polypeptide unit, that may be combined into a multimeric protein, such as a dimer, a trimer, a tetramer, a pentamer etc. The single-domain antibody units of a multimer may be identical, or may differ slightly from one another with regard to their amino acid sequence. In addition to said single-domain antibody units, a multimer may contain one or more further polypeptide unit(s), such as a stabilizing polypeptide unit as described herein, which is not a single-domain antibody. Such a further polypeptide unit may be present in a single copy or at multiple copies in the multimer.

Within a multimer, individual polypeptide units may be linked via peptide bonds, typically via a linker peptide. Thus, a multimeric protein may be formed of a single polypeptide chain.

The term "fusion protein" refers to a protein formed of two or more than two separate proteins (fusion partners) produced by recombinant protein expression as a single polypeptide containing both fusion partners. The fusion partners may be joined sequentially one after the other. The genes encoding the respective proteins are joined at the genetic level. The fusion partners typically do not naturally occur as fused to each other. A fusion protein may contain additional amino acids sequences, such as linker in between the fusion partners.

As used herein a "linker" refers to a peptide or other chemical linkage that functions to link otherwise independent functional domains. A linker may be located between two polypeptide units, monomers or domains, such as between a ligand (e.g., an sdAb) and another polypeptide component containing an otherwise independent functional or structural domain, or between two ligands. Suitable linkers for coupling two or more linked units may generally be any linker used in the art to link peptides, proteins or other organic molecules. A linker peptide may be a stretch of amino acids preferably ranging from 1 to 20 amino acids, such as 2-15, such as 2-8, 2-4 or 4-12 amino acids.

The term "% identity" or "% sequence identity", as used throughout the disclosure, may for example be calculated as follows. The query sequence is aligned to the target sequence using the CLUSTAL W algorithm (Thompson et al, Nucleic Acids Research, 22: 4673-4680 (1994)). A comparison is made over the window corresponding to the shortest of the aligned sequences. The shortest of the aligned sequences may in some instances be the target sequence. In other instances, the query sequence may constitute the shortest of the aligned sequences. The amino acid residues at each position are compared and the percentage of positions in the query sequence that have identical correspondences in the target sequence is reported as % identity. Where a certain amino acid position is to be omitted when determining the % sequence identity, the sequence identity will be determined based on a shorter sequence. For example, in a FWR2 sequence having a total of 14 amino acid residues, where the Kabat positions 40, 43, 44, 45, and 47 are to be omitted from a sequence identity determination, these amino acids are deleted from the query sentence and from the target sentence such that the sequences are to be compared over a sequence of 9 amino acids instead.

### DETAILED DESCRIPTION

The present inventors have found that the framework regions (the "scaffold") of a heavy chain variable domain (VH) may affect the properties of the VH. For example, certain amino acid positions may affect the binding properties of the CDRs of the VH. The present inventors have also found that a heavy chain variable domain (VH) scaffold could be stabilized to render it particularly useful as a scaffold for an affinity binder for *in vitro* applications, such as detection, analysis or separation of an analyte based on affinity interactions. Thus, the framework is useful for presenting CDRs for the interaction to an analyte to be captured, e.g. for the purpose of detection or separation. In particular, the scaffold is useful for providing an affinity ligand for affinity capture, such as affinity chromatography. Thus, the polypeptide may be an antigen-binding polypeptide. However, it is contemplated that a stabilized VH scaffold (herein referred to as "framework") could be useful also where functional CDRs are not required. It is also shown herein that some framework positions also affect binding properties.

The present disclosure provides scaffold modifications that affect binding properties and improve alkaline stability of the polypeptide, meaning that the antigen binding functionality is less affected by exposure to alkaline conditions as compared to non-modified counterparts, such as naturally occurring single domain antibodies, sdAbs. Due to ease of expression and good affinity, use of sdAbs for affinity capture applications has gained interest. However, sdAbs are widely known for lacking alkaline stability, which is a great draw-back when it comes to affinity chromatography. Improved alkaline stability is a great benefit in the context of affinity chromatography, as the chromatography material is conventionally subjected to cleaning using an alkaline agent (often NaOH) between the purification cycles. Increased alkaline stability of the affinity ligand means that the chromatography material can be used for a higher number of purification cycles before binding capacity becomes unacceptably low.

Besides being able to affect the alkaline stability, it has been found that certain amino acid positions in the framework greatly affects the binding of the CDRs to the target antigen. The performance of a sdAb depends on both the CDRs and the framework, where the CDRs typically are the main contributors regarding interaction and binding while the framework contributes to the overall structure and stability. However, it has now been demonstrated that the CDRs may also affect alkaline stability in combination with the interaction, and in addition, that the framework comprises amino acid positions that are also very important for the interaction, and which could be combined with other protein properties.

The polypeptide of the present disclosure comprises a heavy chain variable domain (VH) variant, e.g. a single domain antibody variant, such as a VHH or VH variant, or a synthetic antibody fragment, such as single-chain variable fragment (scFv). The polypeptide of the present disclosure may thus be a single-chain polypeptide.

Single-domain antibodies, as well as the VH variants of the present disclosure, lack the light chains of full-size antibodies, and also lacks the heavy chain constant domains. Thus, VH variant of the polypeptide disclosed herein comprises only a heavy chain variable domain, including three complementarity determining regions (CDRs). Thus, the VH variant of the present disclosure contains no antibody light chain variable region or light chain CDRs. However, in embodiments where the polypeptide of the present disclosure comprises a single-chain variable fragment, it would comprise an antibody light chain variable domain in addition to the VH domain.

In general, single domain antibodies are easier to express in prokaryotic and eukaryotic cells as compared to conventional full-size antibodies, as well as Fab fragments or single-chain fragments including both light and heavy chain portions. Despite having only three CDRs, as opposed to the six CDRs of larger antibodies and antibody fragments, the target binding properties of can be satisfactory. However, for natural single-domain antibodies such as camelid VHH, stability under alkaline conditions is an issue, and poor alkaline stability will prevent the application of such VHH as affinity ligands for chromatography in many cases.

The portions of a heavy chain variable domain (VH) not forming the CDRs are referred to as framework. The VH framework is formed of four regions, herein referred to as framework region (FWR) 1 to 4, which are responsible for the general secondary and tertiary protein structure. The framework regions of a VH form a β-sandwich often comprising a cysteine bridge. The CDRs are typically presented as three surface loops at one end, one CDR per loop.

An advantage of using an sdAb as affinity binder is that the CDR sequences of an sdAb can be more diverse with regard to their length, than the CDRs of conventional antibodies. For example, the surface loops formed of the sdAb CDRs can spatially extend further from the main body of the sdAb and thus have a better ability to bind into pockets of a target epitope, or the surface loops may be shorter, forming flatter surfaces which may facilitate binding to other epitopes.

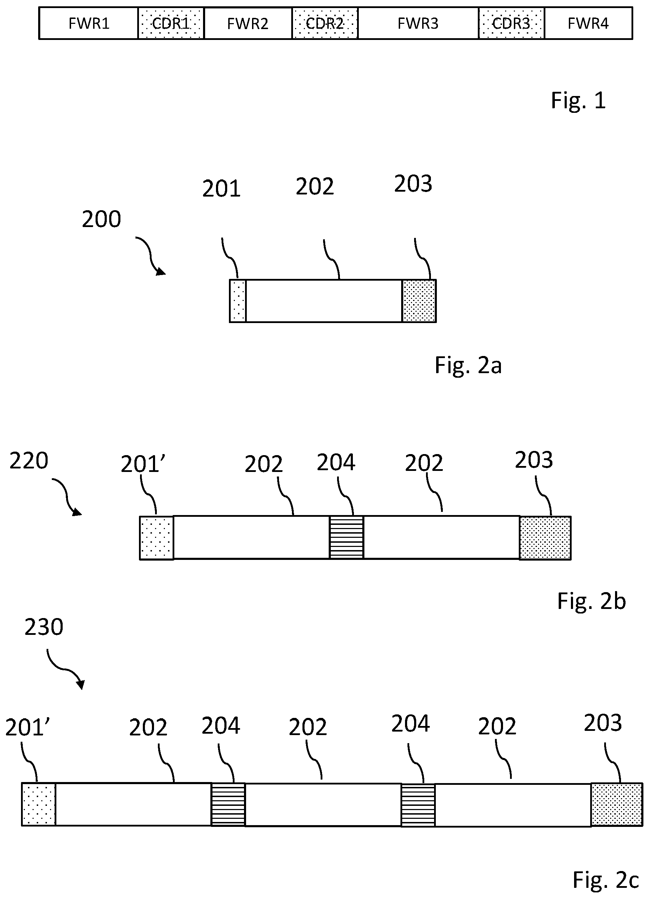

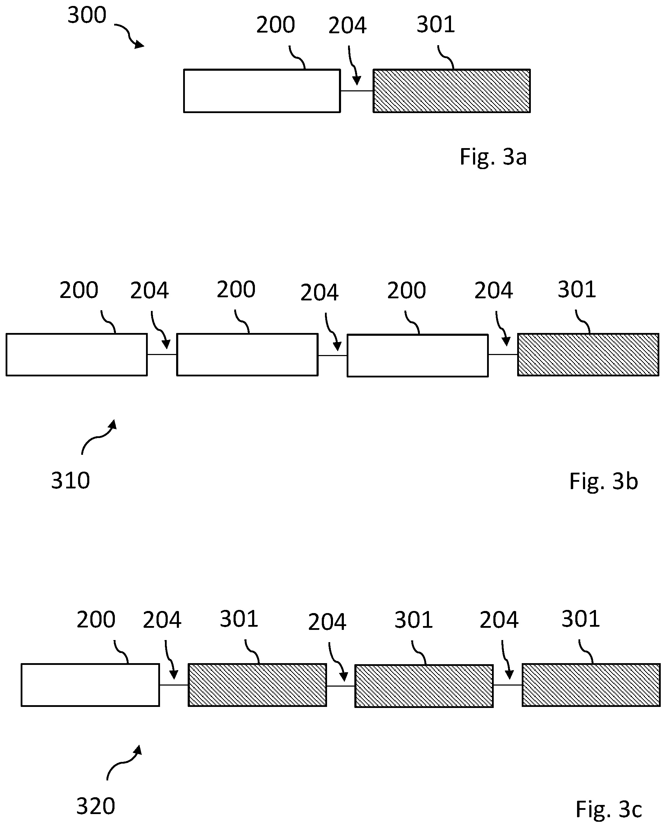

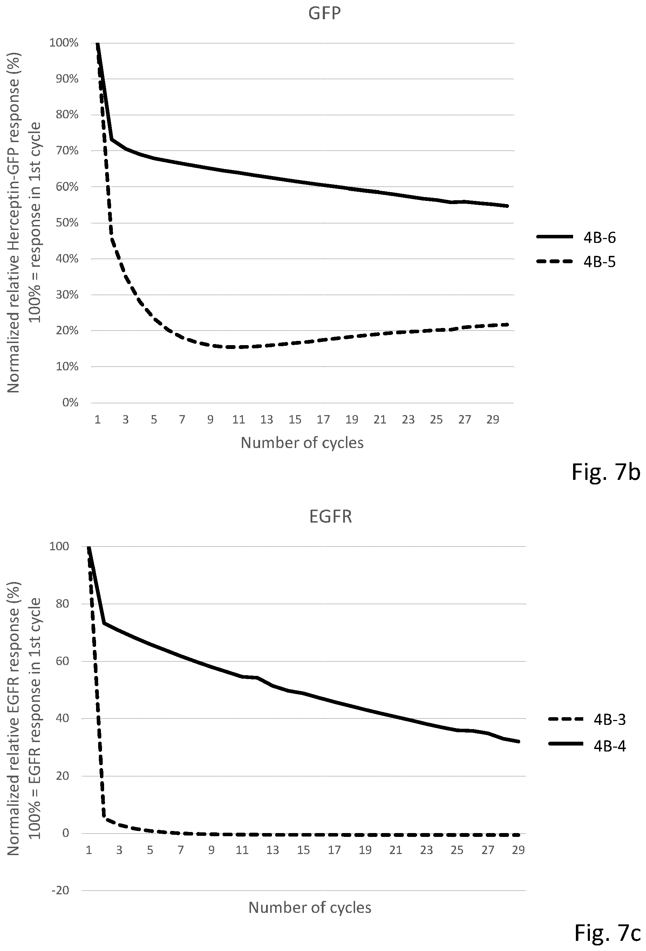

Thus, the polypeptides of the present disclosure comprise a variant of a heavy chain variable region (VH) of an antibody, and in particular a variant of a single-domain antibody (sdAb), also known as VHH or VH. Single-domain antibodies lack light chains and exist naturally in e.g. camelids. The polypeptides herein may thus be synthetic VH, originally derived from e.g. camelid sequences (or other species) into which various modifications have been introduced. Camelid VH sequences (also referred to as nanobodies when expressed in a bacterial host) exhibit a high degree of framework conservation - in fact even higher than VH domains of full-size antibodies (Mitchell & Colwell, Proteins. 2018; 86:697-706, see e.g. Figs. 2 and 3). Therefore, native camelid frameworks, such as framework regions of e.g. vh166/SEQ ID NO:157 (Llama glama) may be seen as an "original" starting sequence into which modifications (mutations) are introduced. Alternatively, vh98/SEQ ID NO: 73, which represents a synthetic sequence obtained by the present inventors by introducing certain modifications into a native camelid sequence, could be used as a starting point for introducing further mutations, as, even though the alkaline stability of vh98 is much improved over a native camelid sequence, it may be further improved for use in the context of affinity chromatography. Therefore, further modifications are proposed, which were found to increase alkali stability even further. Thus, the "starting" framework regions, into which modifications are to be introduced, may be based on the framework regions of native camelid sequences, such as an EGFR-binding VHH (vh166, SEQ ID NO: 157), an AAV9-binding VHH (vh342, SEQ ID NO: 152), or an GFP-binding VHH (vh60, SEQ ID NO: 155), or based on a synthetic framework such as the framework regions of vh97/vh98 (SEQ ID NO: 72/SEQ ID NO:73). The problem addressed by these modifications is the very poor alkaline stability of native camelid VHH domains, as is illustrated in Example 4B and Fig. 7c below, where an EGFR-binding VHH (vh166) having framework regions obtained from Llama glama is shown to lose virtually all binding activity after merely 2 cycles of alkali exposure.

Fig. 1 schematically illustrates the sequence of framework regions and complementarity determining regions (CDRs) of a VH, such as an sdAb, from the direction of the N terminus to the C terminus of the amino acid sequence. Further, Table 1 outlines the FWRs and CDRs as defined herein in relation to the Kabat amino acid numbering system (Kabat et al., 1991, J. Immunol. 147(5), 1709-1719), where example sequences for the FWRs are from vh97. For a VH variant of the present disclosure, Framework region 1 (FWR1) is formed of the amino acids in positions 1 to 26. Next, amino acids in Kabat positions 27 to 35d form CDR1, Kabat positions 35a-d being optional. Framework region 2 (FWR2) is formed of the amino acids in Kabat positions 36 to 49. CDR2 is formed of the amino acids in Kabat positions 50 to 58 (52a being optional). Framework region 3 (FWR3) is formed of the amino acids in Kabat positions 59 to 94. CDR3 is formed of the amino acids in Kabat positions 95 to 102 (100a-j being optional). Lastly, framework region 4 (FWR4) is formed of amino acids in Kabat positions 103 to 113. It may be noted that the definition of CDRs and framework regions within the present disclosure does not fully correspond to the definition of CDRs and framework regions of Kabat et al; however, the same amino acid numbering system is used.

**Table 1**

| Amino acid positions (Kabat) | 1-26 | 27-35d | 36-49 | 50-58 | 59-94 | 95-102 | 103-113 |
|---|---|---|---|---|---|---|---|
| Region | FWR1 | CDR1 | FWR2 | CDR2 | FWR3 | CDR3 | FWR4 |
| Example sequence | SEQ ID NO:4 | | SEQ ID NO:23 | | SEQ ID NO:45 | | SEQ ID NO:71 |

The present inventors have identified that in some amino acid positions of the framework regions, certain mutations of the amino acid residue in that position are not only tolerated without compromising functionality, but may even lead to improved performance, especially an improved alkaline stability, affinity and/or selectivity, of the VH variant. On the other hand, there also appears to be amino acid positions where mutations are less well tolerated. Hence, the framework regions, which do not include the hypervariable CDR regions, comprise regions or positions which allow some variability, as well as positions which are conserved to a higher degree.

Among the individual amino acid mutations (residue variations) described herein, positions 71 in FW3 and 24 in FW1 (Kabat numbering) have been identified as of great importance for several aspects, in particular binding to target molecule. These positions have side chains towards the interaction surface between target molecule and/or sdAb CDRs, and are therefore very important for the selective interaction as well as other properties such as stability etc. Accordingly, a VH variant of the present disclosure may have a framework region 3 and/or 1 as described herein, and in particular amino acid residues in Kabat positions 71 and/or 24 as described herein. Position 24 also has a significant impact on the alkaline stability.

Among the individual framework regions, FWR2 has been identified as having the most significant impact on alkaline stability. Accordingly, a VH variant of the present disclosure may have a framework region 2 as described herein, and in particular amino acids in Kabat positions 40, 43, 44, 45 and/or 47 as described herein.

Considering FWR1, FWR2 and FWR3, the following amino acid positions according to the Kabat numbering system have been identified as variable and potentially useful to improve alkaline stability of a VH variant:

**Table 2. Variable positions potentially enhancing alkaline stability**

| **Amino acid position (Kabat)** | **Framework region** |
|---|---|
| 6 | FWR1 |
| 14 | |
| 19 | |
| 23 | |
| 24 | |
| 40 | FWR2 |
| 43 | |
| 44 | |
| 45 | |
| 47 | |
| 60 | FWR3 |
| 76 | |
| 79 | |
| 82b | |
| 89 | |

In addition, it has also been found that variations in some other positions can be tolerated without necessarily improving alkaline stability in the same way as seen for the positions set out in Table 2. Such potentially variable, lower-impact positions include Kabat positions 1, 2, 5, 11, 61, 62, 64 and 78. In this context, "lower impact" refers to the stabilization effect.

However, it is contemplated that there may be even further amino acid positions in the framework regions that may tolerate more than one type of amino acid without compromising functionality.

Together, the framework regions FWR1-FWR3 as set out in the present disclosure may contain 79 amino acids. As mentioned above at least 24 positions in FWR1-3 may allow more than one amino acid. Considering this, the VH variant amino acid sequences contained within the present disclosure represent a sequence variability over the framework regions 1-3 (FWR1-3), of up to about 30 % in relation to FWR1-3 of any one of the individual VH sequences. Hence, for example, the present disclosure encompasses stabilized VH variant sequences whose framework regions have at least 70 % identical amino acids with SEQ ID NO:80, as well as sequences whose framework regions have more than 70 % sequence identity to SEQ ID NO:80, for example, at least 75 %, such as at least 80 % sequence identity, such as at least 85 % sequence identity.

The amino acid residues in the 16 positions that are presently identified as variable and having a potentially positive impact on stability and affinity of the polypeptide can be selected, for each position, from a limited group of amino acids that have been found to contribute to improved stability. The amino acids contemplated for each of these positions are presented in Tables 3-5 hereinbelow.

Among these 16 variable positions, the amino acid positions (Kabat numbering) set out in Table 3a have been identified as particularly important.

**Table 3a.**

| **Kabat position** | **Stabilizing amino acids** | **Preferred amino acid(s)** |
|---|---|---|
| 24 | I, V, S | |
| 40 | R, I, K, T | R, I |
| 45 | R, I, L | R |
| 76 | N, Q | N |
| 79 | Y, W, F | Y, W, F |
| 89 | V, I | V, I |

| | **Binding property-affecting amino acids** | **Preferred amino acid(s)** |
|---|---|---|
| 24 | A, R, N, D, Q, E, H, I, L, K, F, S, T, Y, V | |
| 71 | R, K, Q, Y, F, H | |

The VH variant of the present disclosure may have any one position independently selected according to Table 3a, or any combination or sub-group of these positions. For example, the amino acid residue in Kabat position 71 may be selected from R, K, Q, Y, F and H, and/or the amino acid in Kabat position 24 may be selected from A, R, N, D, Q, E, H, I, L, K, F, T, Y, V and S, and/or the amino acid residue in Kabat position 40 may be selected from R, I, K and T. The amino acid residue in Kabat position 76 may be selected from N and Q. Native amino acids in position 71 is mainly R, but other exist.

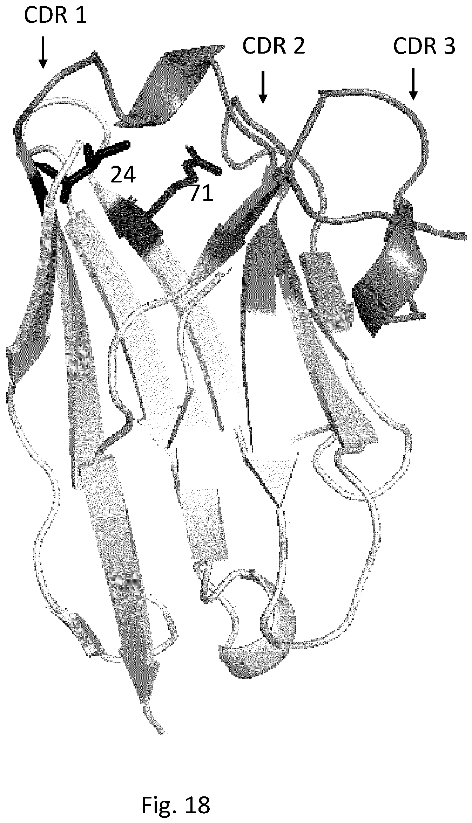

Regarding binding interaction, it has been shown that a mutation in position 71 may be essential for the binding, such that one amino acid in said position may greatly enhance the binding, while another obliterates the binding altogether. Thus, in some instances, the amino acid in this position may affect the binding more than any amino acid in the actual CDRs. Also position 24 was shown to greatly affect the binding affinity. It may be seen that residues in both position 24 and 71 are located such that they point towards the binding area of target molecule, interact with the CDRs or both of the sdAb in the tertiary structure, as seen in Fig. 18. In Fig. 18, the framework is light grey, the CDRs are dark grey and the residues 24 and 71 are black.

Although it was seen that some single-point mutations can significantly improve the alkaline stability, it is believed that the amino acid residues in multiple positions interact to provide a stabilized VH. For this reason, it is desirable to select more than one of the amino acid residues in accordance with Table 3. For example, at least four, or at least five out of the six positions may be selected accordingly. For example, position 24 or position 40 may have an amino acid that falls outside of the groups indicated in Table 3, yet result in a stabilized VH variant, such as an sdAb variant having an alanine (A) in Kabat position 24, or a proline (P) in Kabat position 40, as shown in Example 2A and 2B. However, if the residue in Kabat positions 24 is A, it may be preferred that the residue in Kabat position 40 is not A, and vice versa (such that the residues in Kabat positions 24 and 40 are not both A). In some embodiments, it may be preferred that the residue in Kabat position 40 is not A.

In some embodiments, the residue in Kabat position 76 is not T. Alternatively or additionally, in embodiments the residue in Kabat position 89 is not E.

Furthermore, it is hypothesized that amino acids in some position may have a stronger interaction or effect on stability together with one or more amino acids in some of the other positions, which could for example be due to the spatial position and orientation allowing interaction of the respective amino acids in the VH. For example, data suggests that Y, F or W in Kabat position 79 in combination with V or I, in Kabat position 89 may be particularly beneficial. It is contemplated that appropriately selecting the amino acid residues for positions 79 and 89 may provide a stabilizing effect such that a less preferred amino acid can be tolerated in another position, such as in position 24 (FWR1) and/or position 40 (FWR2), such as 24A and/or 40P.

In addition to the six positions indicated in Table 3a, also Kabat positions 19, 23, 43 and 44 have been found to contribute to stability of the VH variant when selected from the amino acids as outlined in Table 3b:

**Table 3b**

| **Kabat position** | **Stabilizing amino acids** | **Preferred amino acid(s)** |
|---|---|---|
| 19 | R, S, K, T | R, T |
| 23 | T, S, V | T |
| 43 | R, K, Q, E, G | R, K |
| 44 | E, Q, A, D, G | E |

It may be preferred that at least one of the residues in Kabat position 19 and position 23 is T. Having T in Kabat position 19 was found to promote alkaline stability, especially where the amino acid in Kabat position 23 was V (see Example 2). On the other hand, Kabat position 19 has been found to participate in VH3 interaction with protein A. Where it is desirable to preserve such capability of interaction, the amino acid residue in position 19 is advantageously R.

In total, Tables 3a-b above define ten positions. It is desirable that at least eight out of these ten positions have amino acids selected from the stabilizing amino acids according to Tables 3a-b. Preferably, at least nine, such as all, positions are selected as set out in Tables 3a-b.

When only eight of the positions have amino acids selected according to Tables 3a-b, it is possible that only four of the positions 24, 40, 45, 76, 79 and 89 are as defined in Table 3a. For example, such polypeptides may have 24A, 40P.

In embodiments, at least five of the positions 24, 40, 45, 76, 79 and 89 are as defined in Table 3a, and optionally any one of positions 19, 23, 43 and 44 are selected according to Table 3b.

In some embodiments, the residue in Kabat position 43 is R, K, Q or G.

For the remaining variable and potentially stabilizing positions of the VH framework regions, one or more of these positions may have amino acids independently selected as indicated in Table 4:

**Table 4.**

| **Kabat position** | **Stabilizing amino acids** | **Preferred amino acid(s)** |
|---|---|---|
| 6 | Q, E | |
| 14 | A, S, T | |
| 47 | F, L | F |
| 60 | A, T, N, Q, S | A, S |
| 82b | N,S, T, Q | |

Preferably, the residue in Kabat position 47 is selected from F and L, and more preferably is F. In embodiments where the residue in Kabat position 47 is not selected according to Table 4, it is preferably not G, S, T or Y.

Position 60 may tolerate some variability while maintaining acceptable alkaline stability. Preferably however, the amino residue in position 60 may be selected from A, T, N and S.

Kabat position 82b has been found to participate in VH3 interaction with Protein A. Where it is desirable to preserve such capability of interaction, the amino acid residue in 82b is advantageously selected from S and N.

Optionally, more than one of Kabat positions 6, 14, 47, 60 and 82b may have amino acids selected in accordance with Table 4. For example, at least two, at least three, at least four, or all five of these positions have stabilizing amino acids selected accordingly.

Considering all of the Kabat positions 6, 14, 19, 23, 24, 40, 43, 44, 45, 47, 60, 71, 76, 79, 82b and 89, it may be preferred that a VH variant according to the present disclosure has at least 13, such as at least 14, positions selected from the amino acid residues according to Tables 3a-b and 4.

As indicated above, 2 positions of the framework regions have been shown to affect the binding with the CDRs, and 15 (16) positions of the framework regions have been identified as susceptible of modification to improve stability, where positions 24 and 71 may affect both. As for the remaining part of the framework regions, the amino acids in some positions may be replaced by another amino acid, but in general, these parts of the framework regions are highly conserved and may preferably be kept constant to a large degree. These parts are herein defined by Kabat positions 1-5, 7-13, 25-26, 36-39, 41-42, 46, 48-49, 59, 61-70, 72-75, 77-78, 80-82a, 82c-94 and 103-113. Optionally, position 6 may be included in this group. The VH variants of the present invention may thus have a relatively high degree of sequence identity over these amino acid positions, which represent less variable parts of the framework regions.

Therefore, in a VH variant of the present disclosure each of FWR1, FWR2 and FWR3 may have at least 80 %, such as at least 85 %, at least 88 % or at least 90 %, identity to the amino acid sequence of a corresponding framework region of SEQ ID NO:80 wherein for the purpose of determining the sequence identity, the Kabat positions 14, 19, 23, 24, 40, 43, 44, 45,47, 60, 71, 76, 79, 82b and 89, and optionally Kabat position 6, are omitted from the comparison. As an example, FWR1 contains 26 aa, and after omitting positions 6, 14, 19, 23 and 24, the sequence identity is determined based on the remaining 21 amino acids, which represent the more conserved positions. Substituting 2 of these amino acids in relation to the FWR1 of SEQ ID NO: 80 may result in 90.5 % identity, whereas substituting 3 amino acids in relation to the FWR1 of SEQ ID NO: 80 may result in 85.7 % identity. FWR1 of SEQ ID NO: 80 is represented by SEQ ID NO: 8.

Taking the framework regions 1-3 as a whole, FWR1-3 may together have a sequence identity of at least 90 % to the corresponding amino acids of any other VH of the present disclosure. As an example, FWR1-3 may have a sequence identity of at least 90 %, such as at least 92 %, such as at least 94 %, such as at least 96 %, to the corresponding amino acids (FWR1-3) of SEQ ID NO:80, wherein for the purpose of determining sequence identity, the Kabat positions 14, 19, 23, 24, 40, 43, 44, 45,47, 60, 71, 76, 79, 82b and 89, and optionally position 6, are omitted.

Notwithstanding the above, it has been found that the amino acid in position 1 may advantageously be selected from Q, V, D and E, and may in particular be E. For example, any VH variant amino acid sequence disclosed herein in which Kabat position 1 is not E (e.g., is Q), may instead have E in Kabat position 1. In Kabat position 2, V or D may be used, although in some embodiments V may be preferred. Thus, the amino acids in positions 1 and 2 may optionally be QV or EV.

The VH variant of the present disclosure may have an amino acid sequence in which at least nine, such as eight, of the Kabat positions 19, 23, 24, 40, 43, 44, 45, 71, 76, 79 and 89 are independently selected according to Tables 3a-b and 4, or at least five of Kabat positions 24, 40, 45, 71, 76, 79 and 89 are selected according to Table 3a. Furthermore, the VH variant can have an amino acid sequence wherein Kabat positions 1-5, 7-13, 25-26, 36-39, 41-42, 46, 48-49, 59, 61-70, 72-75, 77-78, 80-82a and 82c-94 may include up to 15, such as up to 14, such as up to 13, such as 12, 11, or 10 amino acid residues that are substituted in relation to SEQ ID NO: 80 or SEQ ID NO: 195. For example, in FWR1, positions 1-5, 7-13 and 25-26 (21 residues) may together have up to 4, such as up to 3, amino acid residues that differ from the corresponding position of SEQ ID NO: 80 or SEQ ID NO:195. In FWR2, Kabat positions 36-39, 41-42, 46 and 48-49 (14 residues) may together have 1 or 2 amino acid residues that differ from the corresponding position of SEQ ID NO: 80. In FWR3, Kabat positions 59, 61-70, 72-75, 77-78, 80-82a, 82c-94 (33 residues) may have up to 6 amino acid residues that differ from the corresponding position of SEQ ID NO: 80.

Some amino acids of the less variable part of the framework regions as defined herein may be particularly beneficial to preserve. For example, the VH variant may have an amino acid sequence which has one or more of the following amino acid residues:
- 15G, 17S, 59Y, 64K, 65G, 66R, 68T, 70S, 81Q and/or 82aN, all of which are involved in VH interaction with protein A
- 22C, 92C
- 36W, 103W
- 38R

The framework regions of VH domains or sdAbs typically have a secondary structure comprising beta sheets, stabilized by intramolecular interactions between the side chains of amino acids positioned in different parts of the primary protein structure (amino acid sequence). Certain amino acid residues of have been identified as interacting with one or more other amino acid residues of the VH variant. For example, residues in position 76 and also position 44 have been found to be capable of interacting with several other residues. A high number of interactions is considered to provide a high degree of stabilization.

Amino acid position 24 in FW1, and amino acid position 71 in FW3 (corresponding to position 13 of the FW3 sequence) affect the interaction of the sdAb to its target as they have side chains towards the interaction surface between target molecule and sdAb CDRs. The effect of the amino acids, from each amino acid in said positions, depends on the CDRs of the sdAb, and an amino acid that promotes binding for certain CDRs, impairs binding for others. Thus, it is important to evaluate which amino acid would be optimal in said positions for certain CDRs. Thus, the amino acid in said position may be selected based on how they affect the CDR binding interaction. Alternatively, the amino acids in said positions may be optimized for other properties, such as stability or elution properties, and suitable CDRs may be selected for a subsequent screening process. In the present disclosure, several different mutations in said positions have been evaluated for different CDRs, as shown in the example section below.

Furthermore, due to its central position within the three-dimensional structure of the VH variant, FWR2 is considered to be of particular importance for the stability of the VH variant, as compared to FWR1, FWR3 and FWR4.

Accordingly, herein is disclosed a number of mutations that contribute to the stability of a polypeptide, where some positions or substitutions may be more important than others. The polypeptide may be a sdAb, but as shown in example 12 in the example section below, the stabilization is also present for an scFv. Thus, it is hypothesized that the mutations increase the stability of the polypeptide regardless of which polypeptide the framework is present in. Thus, starting from any polypeptide having at least three CDRs and four surrounding framework regions, the following mutations could be introduced to increase the stability of said polypeptide.

Thus, in some embodiments, the invention provides a polypeptide comprising a heavy chain variable domain (VH) variant comprising a plurality of antigen-binding regions and a framework comprising framework region 1 (FWR1), framework region 2 (FWR2) and framework region 3 (FWR3), wherein the VH variant has an amino acid sequence in which at least eight of the following criteria i) to xi) are fulfilled:
i) the residue in Kabat position 19 is R, S, K or T;
ii) the residue in Kabat position 23 is T, V, A or S, preferably T;
iii) the residue in Kabat position 24 is I, V, A, R, N, D, Q, E, H, L, K, F, T, Y or S;
iv) the residue in Kabat position 40 is selected from R, I, T or K, preferably R;
v) the residue in Kabat position 43 is R, K, Q, E or G, preferably R or K;
vi) the residue in Kabat position 44 is E, Q, A, D or G;
vii) the residue in Kabat position 45 is R, I or L, preferably R;
viii) the residue in Kabat position 71 is F, H, K, Q, Y or R;
ix) the residue in Kabat position 76 is N or Q;
x) the residue in Kabat position 79 is Y, W or F; and
xi) the residue in Kabat position 89 is V or I.

Optionally at least nine, such as ten, such as all, of the criteria i) to xi) are fulfilled.

In a further embodiment, there is provided a polypeptide comprising a heavy chain variable domain (VH) variant, comprising a plurality of antigen-binding regions and a framework comprising framework region 1 (FWR1), framework region 2 (FWR2) and framework region 3 (FWR3), wherein the VH variant has an amino acid sequence in which at least five of the following criteria i) to vii) are fulfilled:
i) the residue in Kabat position 24 is I, V, A, R, N, D, Q, E, H, L, K, F, T, Y or S;
ii) the residue in Kabat position 40 is selected from R, I, K, or T, preferably R;
iii) the residue in Kabat position 45 is R, I or L, preferably R;
iv) the residue in Kabat position 71 is F, H, K, Q, Y or R;
v) the residue in Kabat position 76 is N or Q;
vi) the residue in Kabat position 79 is Y, W or F; and
vii) the residue in Kabat position 89 is V or I.

Optionally, at least six, such as all seven criteria i)-vii) may be fulfilled.

A polypeptide of the present disclosure may satisfy at least eight of the criteria i)-xi) of the first embodiment and at the same time at least five of the criteria i)-vii) of the second embodiment.

In some embodiments, the criteria as defined in the embodiments above relate to mutations compared to i) a native camelid framework as defined in any one of SEQ ID NO: 157, SEQ ID NO: 155 or SEQ ID NO: 157, or ii) a synthetic framework as defined in any one of SEQ ID NO: 72 or SEQ ID NO:73. The mutations may also be in view of other native of synthetic frameworks that need to be stabilized, of sdAbs or other polypeptide comprising at least three CDRs with four surrounding framework regions, such as scFvs.

In some embodiments, the VH variant may have a framework region 1 (FWR1) comprising an amino acid sequence according to
X₁X₂QLX₅X₆SGGGX₁₁VQX₁₄GGSLX₁₉LSCX₂₃X₂₄SG (SEQ ID NO: 1)
wherein, independently,
X₁ is Q, V, D or E, preferably Q or E;
X₂ is V or D, preferably V;
X₅ is Q, V or E, preferably Q;
X₆ is Q or E, preferably Q;
X₁₁ is S or L, preferably S;
X₁₄ is A, S, or T, preferably A;
X₁₉ is R, S, K or T, preferably R or T;
X₂₃ is T, V, A or S, preferably T; and
X₂₄ is I, V, A, R, N, D, Q, E, H, L, K, F, T, Y or S, preferably I.

Alternatively or additionally, the VH variant may have a framework region 2 (FWR2) comprising an amino acid sequence according to
WFRQX₅PGX₈X₉X₁₀EX₁₂VA (SEQ ID NO: 2)
wherein, independently,
X₅ is R, I, T or K, preferably R;
X₈ is R, K, Q, E or G, preferably K or R;
X₉ is E, Q, A, D or G;
X₁₀ is R, I or L, preferably R; and
X₁₂ is F or L, preferably F.

Alternatively or additionally, the VH variant may have a framework region 3 (FWR3) comprising an amino acid sequence according to
YX₂X₃X₄VX₆GRFTISX₁₃DNAKX₁₈TX₂₀X₂₁LQMNX₂₆LKPEDTAX₃₄YYCAA (SEQ ID NO: 3)
wherein, independently,
X₂ is A, T, N, Q or S, preferably A;
X₃ is D or S, preferably D;
X₄ is S or A, preferably S;
X₅ is K or A, preferably K;
X₁₃ is F, H, K, Q, Y or R;
X₁₈ is N or Q, preferably N;
X₂₀ is V or A, preferably V;
X₂₁ is Y, W or F, preferably Y or W;
X₂₆ is N, S, T or Q, preferably S; and
X₃₄ is V or I.

In embodiments, in SEQ ID NO: 3 X₁₃ is not R.

In embodiments, in SEQ ID NO: 3 X₆ is K, X₂₁ is Y and X₂₆ is S.

Framework region 4 (FWR4) typically does not form part of the core structure of a VH, but forms a peripheral tailing structure at one end of the polypeptide, similar to a linker or spacer structure. The amino acid sequence of FWR4 may have a less significant influence on the stability and/or target binding affinity of a VH, and hence may have less potential for improving alkaline stability as compared to FWR1-3. A VH variant of the present disclosure may have a FRW4 amino acid sequence corresponding to a native FWR4 of e.g. a camelid VHH. Optionally, the VH variant may comprise a framework region 4 (FWR4) comprising or consisting of the amino acid sequence
WGQGTQVTVSS (SEQ ID NO: 71)
or an amino acid sequence having at least 70 %, such as at least 75 %, such as at least 80 % identity with SEQ ID NO: 4, with the proviso that the amino acid residue in position 1 is W.

However, it is contemplated that the VH may lack a traditional FWR4, or may have a partly or completely different structure instead of a VH FWR4, such as a peptide linker as disclosed elsewhere herein.

Tables 5a-c present exemplary amino acids for each complete framework region FWR1-3, with reference to the positions in the Kabat numbering system and to the relative positions in the respective SEQ ID NOs: 1-3.

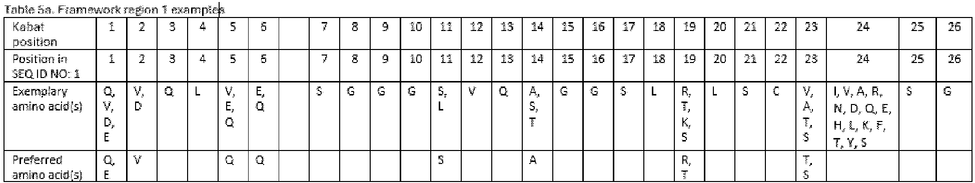

**Table 5b. Framework region 2 examples**

| | | | | | | | | | | | | | | |
|---|---|---|---|---|---|---|---|---|---|---|---|---|---|---|
| Kabat position | 36 | 37 | 38 | 39 | 40 | 41 | 42 | 43 | 44 | 45 | 46 | 47 | 48 | 49 |
| Position in SEQ ID NO: 2 | 1 | 2 | 3 | 4 | 5 | 6 | 7 | 8 | 9 | 10 | 11 | 12 | 13 | 14 |
| Exemplary amino acid(s) | W | F | R | Q | R, T, I, K | P | G | K, G, Q, R, E | E, A, Q G D, | R, L, I | E | F, L | V | A |
| Preferred | | | | | R, I | | | K, R | E | R | | F | | |

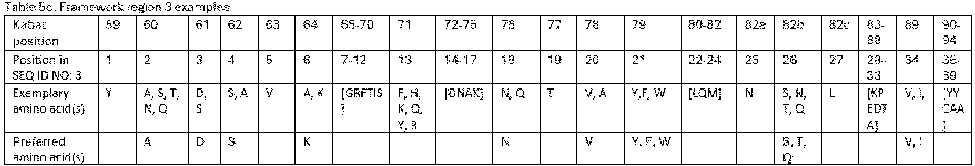

The present VH variant may have an FWR2 amino acid sequence having at least 85 %, such as at least 90 %, sequence identity to Kabat position amino acids 36-49 (FWR2) of SEQ ID NO: 80, wherein for the purpose of determining sequence identity, the Kabat positions 40, 43, 44, 45 and 47 are omitted. Alternatively, FWR2 may have an amino acid sequence having at least 70 %, such as at least 75%, such as at least 80 %, such as at least 85 % identity to a sequence selected from the group consisting of SEQ ID NOs:23-24 and 26-41, Kabat positions 40, 43, 44, 45 and 47 being included in the comparison. Also in this case, at least eight of the ten positions identified in Tables 3a-b may have stabilizing amino acids as indicated in Tables 3a-b, or at least five of the six positions of Table 3a may have stabilizing amino acids selected accordingly. Preferably the residues of Kabat positions 40, 43, 44, 45 and 47 are selected as outlined in Tables 3a-b and Table 4. FWR2 may have an amino acid sequence selected from the group consisting of SEQ ID NOs: 23-24 and 26-41, such as from the group consisting of SEQ ID NOs: 26, 32-39 and 41, or from the group consisting of SEQ ID NOs: 26, 38 and 41.

Alternatively or additionally, the present VH variant may have an FWR1 amino acid sequence having at least 85 %, such as at least 90 %, sequence identity to Kabat position amino acids 1-26 (FWR1) of SEQ ID NO: 80, wherein for the purpose of determining sequence identity, the Kabat positions 6, 14, 19, 23 and 24 are omitted. For example, said FWR1 may have an amino acid sequence having at least 70 %, such as at least 75%, such as at least 80 %, such as at least 85 % identity to an amino acid sequence selected from the group consisting of SEQ ID NOs:4-22, 190 and 194, Kabat positions 6, 14, 19, 23 and 24 being included in the comparison. Also in this case, at least eight of the ten positions identified in Tables 3a-b may have stabilizing amino acids selected accordingly, or at least five of the six positions of Table 3a may have stabilizing amino acids selected accordingly. Preferably the residues of Kabat positions 6, 14, 19, 23 and 24 are selected as outlined in Tables 3a-b and Table 4. FWR1 may optionally have an amino acid sequence selected from the group consisting of SEQ ID NOs:4-22, 190 and 194.

Alternatively or additionally, the present VH variant may have an FWR3 amino acid sequence having at least 85 %, such as at least 90 %, sequence identity to Kabat position amino acids 59-94 (FWR3) of SEQ ID NO: 80, wherein for the purpose of determining sequence identity, the Kabat positions 60, 71, 76, 79, 82b and 89 are omitted. For example, FWR3 may have an amino acid sequence having at least 70 %, such as at least 75%, such as at least 80 %, such as at least 85 % identity to a sequence selected from the group consisting of SEQ ID NOs: 45-49, 54-55, 58-63, 65, 67-70, 191 and 224-228, Kabat positions 60, 76, 79, 82b and 89 being included in the comparison. Also in this case, at least eight of the eleven positions identified in Tables 3a-b may have selective or stabilizing amino acids selected accordingly, or at least five of the Kabat positions of Table 3a may have selective or stabilizing acids selected accordingly. Preferably the residues of Kabat positions 60, 76, 79, 82b and 89 are selected as outlined in Tables 3a-b and Table 4. FWR3 may optionally have an amino acid sequence selected from the group consisting of SEQ ID NOs: 45-49, 54-55, 58-63, 65, 67-70, , 191 and 224-228.

As a whole, the framework of the present VH variant may have at least 90 % sequence identity to the framework of SEQ ID NO: 80 wherein for the purpose of determining sequence identity, the Kabat positions 6, 14, 19, 23, 24, 40, 43, 44, 45,47, 60, 71, 76, 79, 82b and 89 are omitted. For example, the framework regions of the present VH may have at least 92 %, such as at least 94 %, such as at least 97 %, sequence identity to the corresponding framework regions of SEQ ID NO:80, wherein for the purpose of establishing sequence identity, the Kabat positions 6, 14, 19, 23, 24, 40, 43, 44, 45,47, 60, 71, 76, 79, 82b and 89 are omitted.

In embodiments, the framework of the present VH variant may have a framework amino acid sequence corresponding to the framework of a VH variant selected from the group consisting of SEQ ID NOs:72-78, 80-114, 118-121, 126-127,130-135,137, 139-151, 156, 158, 192, 193 and 239-243. For example, the VH variant may have a framework corresponding to the framework of a VH selected from the group consisting of SEQ ID NOs: 76, 78, 80-114, 118-121, 126-127, 130-135, 137, 139-151, 156, 158 and 239-243.

The VH variants disclosed herein can form part of a polypeptide that contains further amino acids in addition to the amino acid sequence of the VH. Through the CDRs, such a polypeptide is capable of binding an antigen, and may be referred to as an antigen-binding polypeptide. A major part of the antigen-binding polypeptide may be constituted by a VH variant, or a plurality of VH variants, as disclosed herein. The binding of the CDRs may be affected by altering the amino acid residues in positions 24 and/or 71.Thus, the CDRs may be selected according to preferred amino acid residue(s) in said positions, wherein said residue is selected for optimizing properties of the polypeptide such as stability, expression, elution etc., or the residue(s) in said positions may be selected to optimize the selectivity and/or binding kinetics to a target of interest of the already present target binding CDRs.

Exemplary arrangement of polypeptides of the present disclosure are schematically illustrated in Fig. 2a-c. In Fig 2a, a polypeptide 200 comprises an N-terminal amino acid sequence 201, a VH variant 202 and a C-terminal amino acid sequence 203. Notably, both the N-terminal amino acid sequence 201 and the C-terminal sequence 203 are optional.

The polypeptide of the present disclosure may optionally be provided in the form of a multimeric polypeptide ("multimer") schematically illustrated in Fig. 2b-c. In Fig. 2b, a multimeric polypeptide 220 comprises multiple VH variants 202 as disclosed herein, which may optionally be joined by a linker sequence 204. N-terminal amino acid sequence 201' is optional and can be a leader peptide of e.g. 1-6, such as 4, amino acids. C-terminal sequence 203 is optional and may comprise a purification tag. The VH variant units 202 may be identical, or may differ slightly from one another with regard to their amino acid sequence. Optionally, the VH variants may have different CDRs, and may even have affinity for different targets. It is also possible that the VH variants of a multimer have different framework sequences, for example with regard to amino acid residues that are capable of interacting with IgG (VH3) binding proteins such that at least one VH variant of a multimer may be capable of such interaction while at least one other VH variant of the multimer is not capable of such interaction.

For example, the polypeptide may comprise two VH variants ("dimer"), or three VH variants ("trimer"). Fig. 2c shows an exemplary trimer 230, comprising three VH variants 202.

Optionally a multimeric polypeptide may contain at least one additional amino acid sequence which does not represent an VH variant, for example a stabilizing polypeptide as described in connection with the fusion protein elsewhere herein.

Multimeric variants of the present polypeptide may be advantageous in that they can provide an increased binding capacity, and/or an improved stability, in relation to a polypeptide comprising a single copy of a VH variant.

In addition to VH variant(s), the polypeptide may comprise an additional amino acid or at least one additional amino acid sequence, typically located at the C terminus and/or N terminus, or as linkers between individual VH variants of a multimer or a fusion protein. The polypeptide may comprise any suitable number of additional amino acid residues, for example one, two, three, four, five, six, seven, eight, nine, ten or more, such as up to 20, additional amino acid residues. Said additional amino acid may be a legacy from recombinant protein expression, a leader peptide, a signal peptide, a purification tag, an affinity tag, a peptide intended for coupling to a support, or the like. The additional amino acid may be a spacer or linker as defined elsewhere herein, or have the ability to serve as spacer or linker. Said additional amino acid residues may individually or collectively improve production, purification, stabilization in vitro or coupling of the polypeptide to substrates of interest, for example to a solid support, such as a solid support described herein. Such additional N-terminal or C-terminal amino acid residues do typically not affect the functional properties, such as binding affinity, of the antigen-binding polypeptide or the single domain antibody as such.

When the present polypeptide is provided in the form of a multimer, comprising a plurality of VH variants, or a fusion protein as described below, said additional amino acid residues may for example not be repeated for each occurrence of the VH variant. For example, where the antigen-binding polypeptide is provided in the form of a multimer, such additional amino acids may occur only at the N-terminus or the C-terminus of the multimer.

Said additional amino residues may be coupled to the VH or multimer by means of chemical conjugation (using known organic chemistry methods) or by any other means, such as expression as a fusion protein or joined in any other fashion, either directly or via a linker, for example a peptide linker as described above.

In some embodiments, the polypeptides and/or multimer, as disclosed above, further comprises one or more coupling elements, selected from the group consisting of at least one cysteine residue, a plurality of lysine residues and a plurality of histidine residues, e.g. a (His)₆ sequence, and combinations thereof. Such coupling elements may be provided anywhere in the polypeptide, but may optionally be arranged close to or at the C-terminal or N-terminal end of the polypeptide. The coupling element may e.g. be a single cysteine at the C-terminal end of the polypeptide. The coupling element(s) may be directly linked to the C- or N-terminal end, or may be linked via a linker comprising up to 15 amino acids, such as 1-5, 1-10 or 5-10 amino acids. This stretch should preferably also be sufficiently stable in alkaline environments not to impair the properties of the protein. For this purpose, it is advantageous if the stretch does not contain asparagine. It can additionally be advantageous if the stretch does not contain glutamine. An advantage of having a C-terminal cysteine is that endpoint coupling of the protein can be achieved through reaction of the cysteine thiol with an electrophilic group on a support. This provides excellent mobility of the coupled protein.

As the skilled person understands, the construction of a multimer, for example as a fusion protein, often involves use of linkers between the monomer moieties to be fused. A monomer moiety may refer to a single-domain polypeptide as described herein. The skilled person is aware of different kinds of linkers with different properties, such as flexible peptide linkers, rigid peptide linkers and cleavable peptide linkers. Linkers may be used in order to for example increase stability or improve folding of fusion proteins. A linker may also ensure a certain spatial distance between the VH variant and the fusion partner polypeptide, which may be beneficial for the access of the target to the binding surface of the VH variant. The presence of a linker within a fusion protein generally does not substantially affect the target binding capacity of a correctly folded antigen-binding polypeptide monomer, such as a single-domain antibody.

Thus, a multimer as defined herein may further comprise at least one linker. For example, a linker is present between each monomer within the multimer. Where there are multiple linkers present, the linkers may be the same or different. For each occurrence, the linker may for example be selected from the group consisting of flexible amino acid linkers, rigid amino acid linkers and cleavable peptide linkers. Peptide linkers may be structured or unstructured. Typically, unstructured linkers are more flexible and less rigid than structured linkers. Alternatively, the linker may be a non-peptidic linker. Thus, polypeptides disclosed herein, or sub-units thereof, may be linked to each directly by peptide bonds between the C-terminal and N-terminal ends of the polypeptides. Alternatively, two or more monomers, in other words monomer units or moieties, within the multimer can be linked by elements comprising oligomeric or polymeric species, such as elements comprising up to 15 or 30 amino acids, such as 1-5, 1-10 or 5-10 amino acids. In embodiments, said linker comprises up to 15 amino acid residues. The nature of such a link should preferably not destabilize the spatial conformation of the protein units, that is of the monomers within the multimer. This can e.g. be achieved by avoiding the presence of proline in the linkers. Furthermore, said linkers should preferably also be sufficiently stable in alkaline environments not to impair the properties of the protein units. For this purpose, it is advantageous if the linkers do not contain asparagine. It can additionally be advantageous if the linker does not contain glutamine. The multimer may further at the N-terminal end comprise additional amino acid residues as described above, e.g. originating from the cloning process or constituting a residue from a cleaved off signaling sequence. The number of additional amino acid residues may e.g. be 15 or less, such as 10 or less or 5 or less.

The antigen-binding polypeptide may be in the form of a fusion protein. As indicated above, it may contain a plurality of VH variants produced as a single fusion protein, optionally with additional amino acids, including linkers, as described above.

In some embodiments the antigen-binding polypeptide may be a fusion protein containing at least one VH variant as disclosed herein and at least one further polypeptide moiety, referred to as a fusion partner polypeptide. The fusion partner polypeptide may be a polypeptide that improves or adds at least one functionality or desirable property. For example, a fusion partner polypeptide may be another antigen-binding polypeptide directed against the same antigen as the present VH variant, or may be directed against a different antigen. It is contemplated that said other antigen-binding polypeptide need not be a VH variant as disclosed herein but can be an antigen-binding polypeptide based on a different scaffold, such as single-domain polypeptides as defined above. For example, the fusion partner polypeptide may be a stabilizing polypeptide, that is a polypeptide that further improves stability, such as alkaline stability, of the antigen-binding polypeptide. As another example, the fusion partner polypeptide may be a polypeptide that improves expression of the antigen-polypeptide in a recombinant cell, such as *E. coli.* As yet another example, a fusion partner polypeptide may provide an opportunity for purification of the antigen-binding polypeptide by affinity chromatography. As another example, the fusion partner polypeptide may improve coupling of the polypeptide to a solid support.

In some embodiments the polypeptide may be a fusion protein containing at least one VH variant as disclosed herein and a fusion partner polypeptide. Fig. 3a schematically illustrates a fusion protein 300 comprising, in the N- to C-terminal direction, a first polypeptide moiety 200 and a second polypeptide moiety 301, joined by an optional peptide linker 204. In this figure, the second polypeptide moiety 301 is located C-terminally of the first polypeptide moiety 200. It is also contemplated that the second polypeptide moiety may be located N-terminally of the first polypeptide moiety. The first polypeptide moiety 200 may comprise or consist of only a single-chain polypeptide 202 as illustrated in Fig. 2a, or may comprise or consist of a single-chain polypeptide 202 and additional amino acids, such as the additional amino acid sequence 201 and/or linker 204.

A fusion protein of the present disclosure may in particular comprise a VH variant, or a plurality of VH variants, fused to an α-helix-containing polypeptide, such as a protein domain. The α-helix containing polypeptide may contain at least one α-helix, such as at least 2 α-helices, such as 3, 4, 5 or 6 α-helices, such as up 10 α-helices. In embodiments, it may be an α-helix bundle domain, such as a three-helix bundle domain. In an α-helix bundle domain, the α-helices are spatially grouped together and form the major part of the protein domain, which may lack other prominent unstructured or beta-structured regions. Examples of α-helix bundle domains include protein domains of *Staphylococcus aureus* protein A (SpA) and albumin binding domain (ABD). For example, the fusion partner polypeptide of the fusion protein of the present disclosure may comprise an amino acid sequence derived from SpA domain A, domain B, domain C, domain D or domain E. For example, the fusion partner polypeptide may comprise a sequence having at least 80% identity such as at least 85 % or at least 90 % identity to SEQ ID NO: 159 or to SEQ ID NO: 160.

Also the fusion protein of the present disclosure may be a multimeric protein, meaning that it may comprise more than one VH, or more than one fusion partner. Multimeric variants containing multiple copies of a VH may be advantageous in that they can provide an increased binding capacity in relation to a fusion protein containing only one copy. A multimeric fusion protein containing multiple fusion partner polypeptides may provide an improved stability in relation to a fusion protein comprising a single stabilizing polypeptide.

Thus, the fusion protein may optionally be provided in the form of a multimeric fusion protein ("multimer") containing at least two polypeptides comprising VH variants, and/or at least two fusion partner polypeptides, as schematically illustrated in Fig. 3b-c. In Fig. 3b, a multimeric fusion protein 310 comprises multiple units of the polypeptide 200 arranged sequentially in the N-to C-terminal direction, and which may optionally be joined to one another by linker sequences 204, which may be the same or different for each occurrence. The VH based polypeptides 200 may be identical, or may be different from one another with regard to their amino acid sequence. Fig. 3b shows three polypeptides 200 ("trimer"), but it is envisaged that the fusion protein may comprise any suitable number of said polypeptide 200. For example, the fusion protein may contain at least two, or at least 3, 4, 5 or 6 units of the antigen-binding polypeptide. The fusion protein may contain e.g. up to 10 antigen-binding polypeptides 200.

Furthermore, the fusion protein may contain at least 2, such as 3, 4, 5 or 6 units, e.g. up to 10 units, of the fusion partner polypeptide. Fig. 3c illustrates a fusion protein 320 containing one first antigen-binding polypeptide 200 and three polypeptide moieties 301. Linkers 204 are optional, and may be the same or different for each occurrence. The polypeptide moieties 301 may be identical, or may be different with regard to their amino acid sequences. In other embodiments, the fusion protein may contain two fusion partner polypeptides and any suitable number of the antigen-binding polypeptide 200 as described above. For example, the fusion protein may contain one antigen-binding polypeptide flanked by two stabilizing polypeptides, or the fusion may contain an antigen-binding polypeptide comprising a VH variant flanked on one side by a different antigen-binding polypeptide, and on the other side by a stabilizing polypeptide. Where a fusion protein comprises multiple antigen-binding polypeptides and multiple stabilizing polypeptides, for example 2 or 3 of each, it is envisaged that the different polypeptide moieties 200, 301 may be arranged in an alternating fashion instead of polypeptides of the same kind being arranged sequentially as depicted in Fig. 3b (for the polypeptide 200) and Fig. 3c (for the stabilizing polypeptide 301). For example, a multimeric fusion protein containing two units of each polypeptide may have the following general structure: [antigen-binding polypeptide]-[fusion partner polypeptide]-[ antigen-binding polypeptide]-[fusion partner polypeptide].

More generally, a fusion protein according to the present disclosure may have a structure as follows:

([A-L1]ₘ-[Z-L2]ₙ)ₚ

wherein A represents a polypeptide as described herein, Z represents a further polypeptide moiety (fusion partner polypeptide) as described herein; L1 for each occurrence may be present or absent, and where present, represents a linker or spacer; L2 for each occurrence may be present or absent, and where present, represents a linker or spacer; m represents an integer of from 1 to 4; n represents an integer of from 1 to 4, or when m≥2, n represents 0 or an integer of from 1 to 4; and p represents an integer of from 1 to 4. Preferably, (m+n)*p, that is, the total number of polypeptide moieties A and Z in the fusion protein, may be up to 10, such as up to 8.

In some embodiments, n=1, m=1 and p=1. In other embodiments, m is 2 or 3, n is 1 and p is 1. In yet other embodiments, m is 1, n is 1 and p is 2 or 3.

For reference, in the exemplary fusion protein of Fig. 3a, m=1, n=1 and p=1. In the exemplary fusion protein of Fig. 3b, m=3, n=1 and p=1. In the exemplary fusion protein of Fig. 3c, m=1, n=3 and p=1.

In examples where the fusion protein comprises at least two fusion partner polypeptides, it may be preferred that at least one is located at the C-terminus of the fusion protein. Optionally, at least two fusion partner polypeptides may be arranged sequentially at the C-terminus of the fusion protein.

At the N-terminus of a fusion protein, such as a multimeric fusion protein, there may be a short amino acid sequence, such as a leader or signal peptide. At the C-terminus a fusion protein, such as a multimeric fusion protein, a tag or spacer, or another short amino acid sequence may optionally be provided as desired. For example, a plurality of histidine residues may be provided as described elsewhere herein.

In embodiments, the fusion partner polypeptide is not located at or near the N-terminus of the fusion protein.

For clarity, any reference herein to the polypeptide or fusion proteins of the present disclosure encompasses also multimeric variants thereof, unless stated otherwise.

In some embodiment, the fusion proteins are defined by SEQ ID NO: 229-243.

The polypeptides of the present invention, which have enhanced properties, such as improved interaction and alkaline stability as compared to polypeptides based on native VHH frameworks, can advantageously be used for generating affinity binders for a suitable target, by grafting CDR sequences directed to a desirable target into a VH variant framework as disclosed herein. Known CDR1-3 sequences for a particular antigen can identified or predicted from relevant literature, or new CDR sequences can be generated or identified by any method known to persons of skill in the art, including but not limited to immunization of animal (e.g., camelids), phage display, ribosome display, cell surface display, bacterial display, simulation *in silico,* and combinations thereof. When satisfactory binding polypeptides have been identified, the amino acid sequences of the CDRs can be selected and used for designing stabilized antigen-binding polypeptides having stabilized framework sequences as described herein. Exemplary methods for generating high affinity antigen-binding VHH having CDR sequences that may be used for grafting are described in, for instance, Schmitz et al, 2013, Structure 21, 1214-1224, and in Fleetwood et al., Cell. Mol. Life Sci. (2013) 70:1081-1093.

For example, the target entity can be an antibody or a part thereof, such as a monoclonal antibody, an antibody fragment or an antibody domain. In some embodiments, the target is an antibody light chain, such as a lambda (λ) or kappa (κ) light chain. As another example, the target entity may be a protein, such as a recombinant protein. Alternatively, the target entity can be a virus particle or a viral vector, such as an adenovirus, a retrovirus (y-retroviruses and lentiviruses), a poxvirus, an adeno-associated virus (AAV), a baculovirus, or a herpes simplex virus. For example, the target entity may be an adeno-associated virus (AAV), such as an AAV serotype 1, 2, 3, 4, 5, 6, 7, 8, 9, 10 (AAVrh10), 11 or 12, such as AAV8 or AAV9. Other examples of target entities include exosomes and lipid nanoparticles. In yet other examples, the target entity may be a nucleic acid molecule, such as DNA or RNA, e.g., mRNA. Other examples include protein modifications. CDRs providing affinity for such antigens can be provided as described above.

The polypeptides of the present disclosure, as well as multimers and fusion proteins as referred to herein, can be produced by conventional biotechnological methods, by recombinant protein production using genetically engineered host cells.

Thus, in further aspects, the present disclosure provides an isolated nucleic acid encoding a polypeptide, multimer or fusion protein as described herein; an expression vector comprising said nucleic acid; and a host cell comprising said expression vector.

Also encompassed by this disclosure is a method of producing the polypeptide as described herein, comprising culturing said host cell under conditions allowing expression of said polypeptide multimer or fusion protein from its expression vector, and isolating the polypeptide, multimer or fusion protein.

The host cells may be prokaryotic cells, such as bacteria, or eukaryotic cells. Exemplary prokaryotic host cells are *Escherichia coli.* Suitable eukaryotic cells may be yeast cells, such as *Saccharomyces cerevisiae* and *Pichia pastoris,* or animal cells, such as insect cells or mammalian cells commonly used in the field of biotechnology to produce proteins, e.g., Chinese Hamster Ovary (CHO) cells or human embryonic kidney (HEK) cells.

Following isolation of the polypeptide from the host cells, which can be done by conventional means of harvesting, clarification and/or filtration etc., the polypeptide can be purified, e.g. by conventional means known in the art. For example, where the polypeptide comprises a histidine tag, a step of purifying the polypeptide may comprise immobilized metal affinity chromatography (IMAC). Other methods of purification that may be used include affinity chromatography. For example, an affinity ligand that binds to a framework region of a VH variant may be used, or the affinity chromatography may use an affinity ligand based on a domain of SpA, wherein the affinity ligand binds to an VH region, such as VH3, of the polypeptide. Alternatively, where the polypeptide forms part of a fusion protein, the affinity ligand may bind to a fusion partner, such as to an affinity tag included in the amino acid sequence of a fusion partner polypeptide.

The polypeptide as disclosed herein may alternatively be produced by *in vitro* translation, or by non-biological peptide synthesis using amino acids and/or amino acid derivatives having protected reactive side-chains. Non-biological peptide synthesis may comprise:
- step-wise coupling of the amino acids and/or the amino acid derivatives to form a polypeptide having protected reactive side-chains,
- removal of the protecting groups from the reactive side-chains of the, and
- folding of the polypeptide in aqueous solution.

In general, the present polypeptide may be used for capture of the target entity for which it has an affinity. However, preferably the polypeptide as such is not intended for use as a therapeutic compound, and is preferably not intended for use *in vivo.* Hence, the polypeptide may be used for *in vitro* capture of the target entity.

The capture may be for the detection of the target entity within a sample, or for separation of the target entity from other components in a sample. Thus, an antigen-binding polypeptide may be used for analytical separation of the target entity, or it may be used for preparative purification of the target entity. The term "preparative" refers to the process of preparing a purified target entity, in particular during the manufacture of a product, such as a pharmaceutical product or a therapeutic composition, which comprises the target entity. Separation, detection and/or quantification using the polypeptide of the present disclosure may alternatively be performed in the context of a sensor application.

In particular, the polypeptide of the present invention may be used in applications involving interaction between VH variant and its target entity, where either the polypeptide or the target entity is coupled to a support. When the polypeptide is coupled or immobilized to a support, the VH variant is still capable of binding its target entity.

Where the polypeptide is coupled to a support, this may be referred to as an adsorbent material or an affinity capture material. Adsorbent materials may be used in various industrial or laboratory applications, including separation, purification, detection and/or quantification of the target entity. A separation matrix as described herein is an adsorbent material intended for use in separation of a target entity from other components. The coupling of the present polypeptide to the support may optionally be provided via a C-terminal amino acid sequence suitable for coupling to a support. For example, a tag or spacer ending with a cysteine may be provided for coupling via a thioether bond, as described in more detail below. Where the polypeptide forms part of a fusion protein, a C-terminal fusion partner polypeptide may have such a C-terminal amino acid sequence suitable for coupling to a support as described below.

Where the polypeptide is not coupled to a support, it may be used e.g. in a detection assay where the target or analyte is present on a surface and is contacted with the polypeptide (capable of binding said target) present in a solution. In such a case, it is envisaged that a reporter entity may be used for generating a detectable signal, wherein the reporter entity is capable of binding to the polypeptide, or capable of binding to the fusion partner in the case of a fusion protein. The reporter entity may be e.g. a fluorescent labelled or radiolabelled entity as known in the art. If the polypeptide or where present, fusion partner polypeptide, is capable of binding to IgG, the reporter entity may be based on IgG.

The polypeptide may be used in applications where it may be exposed to alkaline conditions, such as treatment with NaOH. Such treatments are frequently used in the field of chromatography, to strip a chromatography matrix of bound impurities before the next purification cycle (cleaning-in-place, as described above). However, alkali exposure or cleaning of a binding surface may be used also in other contexts.

The support may be a solid support and may optionally be a porous material.

The support may be or comprise a surface onto which the polypeptide is coupled. Examples of such support materials include conventional protein-binding support and surfaces such as a chip, a plate, a well, and a sheet.

The support may optionally be provided in other forms such as a fiber, a membrane, a fibrous matrix, a filter, a porous monolith, a particle or a bead, such as a gel bead as used in chromatography resins. Particles or beads can be porous or non-porous. Particles or beads may include magnetic beads. Supports in the form of beads or particles can be used as a packed bed or in a suspended form. Suspended forms include those known as expanded beds and pure suspensions, in which the particles or beads are free to move. In case of monoliths, packed bed and expanded beds, a separation procedure commonly follows conventional chromatography with a concentration gradient. In case of pure suspension, batch-wise mode will be used.

The support may be made of any suitable material, as outlined in more detail below. As a non-limiting example, a conventional affinity separation matrix is often of organic nature and based on polymers that expose a hydrophilic surface to the aqueous media used, i.e. expose hydroxy (-OH), carboxy (-COOH), carboxamido (-CONH₂, possibly in N- substituted forms), amino (-NH₂, possibly in substituted form), oligo- or polyethylenoxy groups on their external and, if present, also on internal surfaces.

The polypeptide may be attached to the support via known coupling techniques utilizing e.g. thiol, amino and/or carboxy groups present in the antigen-binding polypeptide. Bisepoxides, epichlorohydrin, CNBr, N-hydroxysuccinimide (NHS) etc. are well-known coupling reagents. Between the support and the polypeptide, a spacer molecule can be introduced, which improves the availability of a binding region and/or facilitates the chemical coupling of the polypeptide to the support. Depending on the nature of the polypeptide and the coupling conditions, the coupling may be a random or multipoint coupling (e.g. via a plurality of lysines or histidines) or a single point coupling (e.g. via a single cysteine). In embodiments, to increase coupling control, it may be preferable that the VH variant such does not comprise any histidine residues. However also in such cases, the polypeptide as a whole may comprise a histidine tag, such as a (His)₆ tag. Alternatively, the polypeptide may be attached to the support by non-covalent bonding, such as physical adsorption or biospecific adsorption.

The polypeptide may be coupled to the support via thioether bonds. Methods for performing such coupling are well-known in this field and easily performed by the skilled person in this field using standard techniques and equipment. Thioether bonds are flexible and stable and generally suited for use in affinity chromatography. In particular when the thioether bond is via a terminal or near-terminal cysteine residue on the polypeptide, the mobility of the coupled antigen-binding polypeptide is enhanced which provides improved binding capacity and binding kinetics. In some embodiments the polypeptide is coupled via a C-terminal cysteine provided on the protein as described above. This allows for efficient coupling of the cysteine thiol to electrophilic groups, e.g. epoxide groups, halohydrin groups etc. on a support, resulting in a thioether bridge coupling.

With regard to specific materials, the support may comprise a polymeric material. Polymeric materials include materials of natural or synthetic polymers, and combinations thereof. For example, the support may comprise a polyhydroxy polymer, such as a polysaccharide. Examples of polysaccharides include e.g. dextran, starch, cellulose, pullulan, agar, agarose etc, including derivatives thereof. Polysaccharides are inherently hydrophilic with low degrees of nonspecific interactions, they provide a high content of reactive (activatable) hydroxyl groups, and they are generally stable towards alkaline cleaning solutions used in bioprocessing. The support may comprise agar or agarose, such as crosslinked agarose. Such supports used in the present invention can easily be prepared according to standard methods, such as inverse suspension gelation (S Hjerten: Biochim Biophys Acta 79(2), 393-398 (1964)). Alternatively, the base matrices are commercially available products, such as crosslinked agarose beads sold under the name of SEPHAROSE^{™} FF (Cytiva^{™}). In an embodiment, which is especially advantageous for large-scale separations, the support has been adapted to increase its rigidity using the methods described in US6602990 or US7396467, which are hereby incorporated by reference in their entirety, and hence renders the matrix more suitable for high flow rates.

Synthetic polymers useful as material for the support include polyvinyl alcohol, polyhydroxyalkyl acrylates, polyhydroxyalkyl methacrylates, polyacrylamides, polymethacrylamides etc. In case of hydrophobic polymers, such as matrices based on divinyl and monovinyl-substituted benzenes, the surface of the matrix can be hydrophilized to expose hydrophilic groups as defined above to a surrounding aqueous liquid. Such polymers are easily produced according to standard methods. As an alternative, a commercially available product, such as SOURCE^{™} (Cytiva^{™}) may be used.

Alternatively, the solid support according to the invention comprises a support of inorganic nature, e.g. silica, zirconium oxide etc.

In embodiments, the polypeptide may be coupled to a support which is a convection-based chromatography matrix. Such convection-based chromatography matrix may comprise a porous polymer membrane, a filter, a fibrous matrix, or a porous monolith. Examples of a porous polymer membrane include Mustang^{™} membranes (Cytiva) and Sartobind^{™} membranes (Sartorius). A fibrous support may be based on electrospun polymeric fibers or cellulose fibers, optionally non-woven fibers. A fibrous matrix may thus be a non-woven fibrous matrix. The fibers may have a cross-sectional diameter of 10-1000 nm, such as 200-800 nm, 200-400 nm or 300-400 nm. Such a fibrous support can be found in a HiTrap Fibro^{™} unit (Cytiva^{™}). Alternative fibrous supports are disclosed in e.g. WO2019/137869 and WO2018/011600.

An adsorbent material or separation matrix as described herein may be used for the same purposes as mentioned above for the polypeptide.

Thus, in one aspect, the invention provides a chromatography material comprising a separation matrix as disclosed herein, as well as chromatography columns or devices incorporating such separation matrix.

In another aspect, the adsorbent material or separation matrix may be a sensor surface intended for use in a sensor or other detection or quantification devices.

The present invention provides a method of separating or isolating a target entity, wherein a separation matrix or an adsorbent material as disclosed herein is used. In some embodiments, the method comprises contacting a liquid sample comprising the target entity with an adsorbent material as disclosed herein. The contacting is performed under conditions under which the target entity can bind to the VH variant of the polypeptide. The method may furthermore comprise washing said adsorbent material with a washing liquid, eluting the target entity from the adsorbent material with an elution liquid, and optionally cleaning the adsorbent material with a cleaning liquid. The cleaning liquid can alternatively be called a cleaning-in-place (CIP) liquid. The cleaning liquid typically is an alkaline solution, such as comprising NaOH or KOH of at least 0.05 M, such as 0.05-1 M, such as 0.05-0.5 M, such as at least 0.1 M, such as 0.1-0.5 M, e.g. 0.3 M or 0.5 M. The contact (incubation) time may be at least 10 min. The binding, eluting and cleaning steps may advantageously be repeated at least 5 times, such as at least 10 times, such as at least 20 times.

As shown herein, the polypeptides of the present disclosure have improved alkaline stability and are thus more resistant to alkaline conditions such as employed during a CIP step, and can retain a high degree of binding capacity for the target entity also after repeated cycles of conventional CIP treatment (using e.g. 0.1-0.5 M NaOH). For example, after at least 12 cycles, such as after at least 15 cycles, or even after 20 cycles, of contact with alkaline liquid, the polypeptide may still retain at least 50 % such as at least 60 %, such as at least 80 %, such as at least 90 %, of the initial target binding capacity. Alternatively, after 12 or 15 cycles, or after 20 cycles, of contact with alkaline liquid, the polypeptide may retain at least 50 % such as at least 60 %, such as at least 80 %, such as at least 90 %, such as at least 95 %, such as at least 98 %, of the target entity binding capacity of the 2^{nd} cycle.

A skilled person will understand that the liquid sample to be purified may be any sample comprising the target entity in an environment from which it is to be purified or separated. The sample may be obtained from a cell culture, such as a clarified cell culture harvest, and may have been subjected to one or more conventional steps of filtration, concentration, dilution and/or buffer exchange, and optionally one of more initial chromatography steps, in particular a step which is not based on affinity chromatography, such as ion exchange chromatography, hydrophobic interaction chromatography, multimodal chromatography, or size exclusion chromatography. For example, the sample may be a clarified and filtered cell culture harvest. In some cases the sample may have been subjected to one or more steps of filtration by tangential flow filtration (TFF).

Prior to contacting with the present adsorbent material, the liquid sample contains the target entity and at least one impurity, such as host cell protein (HCP) or host cell nucleic acids. The adsorbent material is useful for separating the target entity from such impurities, and after performing the above process, the eluate containing the target entity has a reduced level of at least one such impurity.

Optionally, after performing a separation based on affinity capture as described herein of the present disclosure, the eluate containing purified target entity may be subjected to one or more steps of filtration, concentration, dilution or buffer exchange, or chromatography step(s) not based on affinity chromatography, such as ion exchange chromatography, hydrophobic interaction chromatography, multimodal chromatography, or size exclusion chromatography. A further chromatography step performed after the affinity separation of the present disclosure may be referred to as a polishing step.

In some embodiments, the selective binding and binding kinetics for CDRs interaction to a certain target molecule may be optimized by varying the amino acid residue in position 24 and/or 71. As these positions affect the CDR binding by interaction with one or more CDR(s), or interaction directly with the target molecule, different CDRs will require different amino acid residues in these positions. Thus, based on the CDRs present, one or both of these two positions may be varied, and different variants tested to optimize binding. Thus, in some embodiments are provided a method of selecting a variant of a polypeptide, multimer or fusion protein as described herein, wherein the variant selected is optimized for target binding, the method comprising: providing a polypeptide, multimer or fusion protein having a framework according to the present disclosure and CDRs binding a target of interest (CDRs previously selected for binding a target, for example using the methods described herein or any other suitable method known to the skilled person); providing variants of the polypeptide, multimer or fusion protein, wherein the residue in Kabat position 71 for each variant has been altered to be one of F, H, K, Q, Y or R; evaluating, for each variant, the binding affinity or binding selectivity for the target; and selecting the variant that provides the optimal binding or binding selectivity for the target as the variant most optimized for target binding. In some embodiments, the residue in position 24 is also varied, such that for each variant having a distinct residue 71, sub variants are created, in which the residue in position 24 is varied. The method thus comprises providing sub-variants of the variants, wherein the residue in Kabat position 24 for each sub-variant has been altered to be one of I, V, A, R, N, D, Q, E, H, L, K, F, T, Y or S; evaluating, for each sub-variant, the binding affinity or binding selectivity for the target; and selecting the sub-variant that provides the optimal binding or binding selectivity for the target as the sub-variant most optimized for target binding.

In alternative embodiments, the framework of the target binding molecule (polypeptide, multimer or fusion protein having a framework according to the present disclosure) is first selected, where the amino acid residues in positions 24 and/or 71 are selected for enhancing a desired property of the molecule, such as elution properties, alkaline stability, expression etc., followed by a screening procedure for identifying good binding CDRs for a target of interest, which CDRs provide good binding affinity and selectivity together with the selected residues in positions 24 and/or 71 (CDRs providing affinity for such targets may be provided as described above), or for further maturing CDRs of an already target binding sdAb, i.e. for optimizing CDRs to the selected residues in positions 24 and/or 71. Thus, in some embodiments are disclosed a method for providing an enhanced variant of a target binding polypeptide, multimer or the fusion protein of the present disclosure, the method comprising: providing a polypeptide, multimer or fusion protein having a framework FW1, FW2 and FW3 according to the present disclosure, wherein the residue in Kabat position 71 is one of F, H, K, Q, Y or R and/or the residue in Kabat position 24 is one of I, V, A, R, N, D, Q, E, H, L, K, F, T, Y or S; screening a library of CDRs that potentially bind the target to identify target binding CDRs; selecting the CDRs that in combination with the framework provides the good binding affinity or binding selectivity for the target. The method can be used both for identifying a target binder, as well as for maturing already binding CDRs.

In some embodiments are provided a polypeptide, multimer or fusion protein having a framework FW1, FW2 and FW3 according to the present disclosure and CDRs CDR1 CDR2 and CDR3 binding a target, wherein the residue in Kabat position 71 has been selected to enhance target binding, such as target selectivity and target affinity. The residue in Kabat position 71 is selected as F, H, K, Q, Y or R. The selection may depend on the CDRs and/or the target, where the residue is selected to enhance the target binding of the current target binding CDRs and/or current target.

While the invention is described herein with respect to exemplary embodiments, the skilled person will appreciate that the invention is not limited to these. In the claims, any reference signs placed between parentheses shall not be construed as limiting the claim.

Use of the verb "to comprise" and its conjugations does not exclude the presence of elements or steps other than those stated. For example, a polypeptide "comprising" framework regions FWR1, FWR2 and FWR3 can also have further framework regions, such as an FWR4, and may have other regions, such as CDRs, and optionally other structures or sequences. The article "a" or "an" preceding an element does not exclude the presence of a plurality of such elements.

The mere fact that certain measures are recited in mutually different dependent claims does not indicate that a combination of these measures cannot be used to advantage.

### EXAMPLES

### Example 1A: Preparation of glycerol stock solutions from IDT oligo

This example describes the generation of glycerol stock solution for the production of candidate polypeptides used in Examples 2A-B and 4B.

Materials and equipment used were as follows: vector plasmid with kanamycin resistance; G-block polypeptide candidates (IDT^{™}); restriction enzymes Kpnl-HF and Hindlll-HF (NEB^{™}); Antarcic phosphatase (NEB^{™}); GFX^{™} PCR DNA and Gel Band Purification Kit (Cytiva^{™}); T4 DNA ligase (New England Biolabs); Top10 E. coli cells (ThermoFisher^{™}); chemically competent BL21(DE3) E. coli cells (prepared in-house); chemically competent K12-017 E. coli cells (prepared in-house); SOC-media (Invitrogen^{™}); stock of kanamycin 50 mg/ml (PanReac AppliChem^{™}); KCM buffer (5X) 0.5M KCl, 0.15M CaCl₂, 0.25M MgCl₂; Luria-Bertani (LB) culture medium (Invitrogen^{™}); agar plate supplemented with kanamycin (50 µg/ml); 14 ml Falcon^{™} Round-Bottom tube (Corning^{™}); PlasmidPrep Mini Spin Kit (Cytiva ^{™}); Infors HT Shaking incubator.

The vector plasmid and G-blocks containing the DNA sequences for all candidates were digested with restriction enzymes Kpnl-HF and Hindlll-HF. The plasmid was dephosphorylated using Antarctic phosphatase and gel purified according to manufacturer's protocols.

Restriction enzyme digested G-blocks were ligated into the gel purified plasmid using T4 DNA ligase for 2h at room temperature and transformed into Top10 E. coli cells. Briefly, cells and ligation mix were incubated for 20 min on ice and 10 min at room temperature in KCM-buffer. Cells were then transferred to SOC media and incubated at 37°C for 1h and then seeded on agar plates containing kanamycin 50 µl/ml and incubated at 37°C over night. A single colony was used to inoculate 4 mL LB media supplemented with 50 µg/ml kanamycin in a 14 ml round-bottom tube and grown over night at 37°C, 200 rpm. The plasmid was prepared according to manufacturer's protocols and the purified plasmid was transformed into either KCM competent K12-017 E. coli or KCM competent BL21(DE3) E. coli. Cells and plasmid were incubated for 20 min on ice and 10 min at room temperature in KCM-buffer. Cells were then transferred to SOC media and incubated at 37°C for 1h and then seeded on agar plates containing kanamycin 50 µl/ml and incubated at 37°C over night. A single colony was used to inoculate 4 mL LB media supplemented with 50 µg/ml kanamycin in a 14 ml round-bottom tube and grown over night at 37°C, 200 rpm. A glycerol stock of each variant was prepared with 900 µl overnight culture and 500 µl 50% glycerol and stored at -80°C.

### Example 1B: Preparation of glycerol stock solutions from IDT plasmids

This example describes the generation of glycerol stock solution for the production of candidate polypeptides used in Examples 3, 4A and 5-7.

Materials and equipment used were as follows: plasmid DNA for the candidate polypeptides to be produced; chemically competent BL21(DE3) *E. coli* (prepared in-house), KCM buffer (5X) 0.5M KCl, 0.15M CaCl₂, 0.25M MgCl₂, LB culture medium (Invitrogen^{™}), carbenicillin (100 mg/ml), agar plates (BD BACTO^{™} Agar) supplemented with carbenicillin (100 µg/ml) (PanReac AppliChem^{™}), 14 ml Falcon^{™} Round-Bottom (Corning^{™}), Infors HT Shaking incubator.

Desired amino acid sequences of candidate polypeptides were back-translated to DNA and optimized to remove rare codons. Constructs were ordered as plasmid DNA based on an expression vector with T5 promotor, OmpA signal peptide, ampicillin resistance and pUC origin of replication. Plasmid DNA was used for transformation into chemically competent BL21(DE3) *E. coli,* plated on agar plate supplemented with 100 µg/ml carbenicillin and grown overnight at 37°C. For each candidate, a single colony was used to inoculate 4 mL LB media supplemented with 100 µg/ml carbenicillin in a 14 ml round-bottom tube and grown over night at 37°C, 200 rpm agitation. A glycerol stock of each variant was prepared with 900 µl overnight culture and 500 µl glycerol 50% and stored at -80°C until further use.

### Example 2A: Alkaline stability of sdAb variants having framework variations

This example investigated the alkaline stability of an AAV9-binding VHH having a synthetic FWR3 and further variations in framework regions 1 (FWR1) and 2 (FWR2). The impact of mutations in the VHH framework was investigated to identify amino acid positions that enabled high alkaline stability and preserved AAV9 binding.

Candidate VHH sequences are set out in Table 6. Amino acid substitutions (Kabat numbering) are set out in relation to SEQ ID NO:73, a synthetic VHH having a framework modified compared to native VHH framework sequences and which had been found to tolerate repeated cleaning with 0.1 M NaOH (data not shown). CDR sequences were the same for all candidates.

The VHH sequences were expressed with SEQ ID NO:159 as a C-terminal fusion via a peptide linker (AA), and all constructs had a C-terminal tag (HHHHHHC). Total molecular weight was 21 kDa.

**Table 6**

| | FWR1 | | FWR2 | | FWR3 | Full sdAb |
|---|---|---|---|---|---|---|
| | variant | SEQ ID NO: | variant | SEQ ID NO: | SEQ ID NO: | SEQ ID NO: |
| vh98 | 19R, 23V, 24A | 4 | 40P, 47F | 23 | 45 | 73 |
| vh99 | 19T, 23V, 24A | 5 | 40P, 47F | | | 74 |
| vh100 | 19R, 23T, 24A | 6 | 40P, 47F | | | 75 |
| vh103 | 19T, 23T, 24A | 7 | 40P, 47F | | | 77 |
| vh105 | 19T, 23T, 24A | | 40R, 47F | 24 | | 78 |

The following aspects were evaluated: (1) Affinity assessment of AAV9 interaction, tested through high concentration injection of AAV9. (2) Alkaline stability, tested by reduction of AAV9 binding after increasing number of treatments with 0.3 M NaOH.

### Materials and methods

### Generation of IgG Sepharose purified candidate polypeptides

5 µl K12-017 glycerol stock of each variant produced as described in Example 1A was inoculated in 4 ml LB medium supplemented with 50 µg/ml kanamycin in 14 ml round-bottom tube and incubated at 37°C overnight at 160 rpm agitation.

Protein expression culture medium prepared from Terrific Broth (TB) medium supplemented with 50 µg/ml kanamycin and 2 mM MgCl₂ (200 mM) was added to filled 100 ml baffled glass shake flasks (20 ml per flask). Each flask was inoculated with approximately 100 µl from the previous overnight culture to obtain a starting OD600 of 0.05. The flasks were incubated in Infors HT shaking incubator for approximately 3 hours at 37°C and 140 rpm shaking until OD600 reached 1.0. Thereafter 20 µl IPTG (1 M) was added to a final concentration of 1 mM and the flasks were incubated in Infors HT shaking incubator at 27°C and 140 rpm shaking for 18 hours, whereafter the cultures were transferred to Falcon tubes (50 mL). To generate crude variant lysates the samples were sonicated (40% amplitude, 1.5 s total on time, 1 s on/off pulses) on ice. Cell debris was pelleted by centrifugation at 12000 g for 10 min and filtration of supernatant with a 0.22 µm filter.

The samples were purified using gravity flow IgG Sepharose 6FF chromatography resin (Cytiva^{™}) packed in-house in emptied PD-10 columns (Cytiva^{™}). Clarified lysate was loaded on the chromatography column, equilibrated in 50 mM Tris, 150 mM NaCl, 0.05% Tween20 (TST buffer). The column was washed with 10 column volumes (CV) of TST buffer followed by 2 CV of mM NH₄Ac pH 5. Protein was eluted using 2.5 ml of 0.5 M HAc. Eluted protein was buffer-exchanged into phosphate buffered saline (Medicago) pH 7.4 using gravity flow PD-10 (columns Cytiva) prior to further analysis.

Concentration of IgG Sepharose purified samples was measured using NanoDrop (Thermo Scientific) according to manufacturer's instructions. Following purification, samples were kept in refrigerator in Eppendorf tubes until all measurements had been performed and were then kept in freezer until Biacore analysis.

### Biacore analysis of binding affinity for AAV9

To assess binding to AAV9, the candidate polypeptides were immobilized on a Biacore^{™} Series S CM5 chip. AAV9 (2E12 vp/ml) were used as analyte.

Materials and equipment used were as follows: Biacore^{™} Series S CM5 sensor chips (Cytiva^{™}); Biacore^{™} Amine coupling kit (Cytiva^{™}); Biacore^{™} Acetate buffer pH 5.0 (Cytiva^{™}); Biacore^{™} 8K+ instrument (Cytiva^{™}); AAV9 (prepared in-house); IgG Sepharose purified candidate polypeptides as described above.

Immobilization was performed using a standard method in Biacore software with coupling of candidate polypeptides in Flow Cell 2 (FC2) and activation/inactivation in Flow cell 1 (FC1). Candidate polypeptides were diluted in acetate buffer at a concentration of 35 µg/ml. The immobilization levels varied somewhat between different polypeptide variants (average 5200±SD 436 RU).

In each run the polypeptide was immobilized in FC2. Multiple sensor chips were used until all candidates were tested.

Biacore^{™} method for binding analysis: Running buffer: PBS-P+; Flow rate: 5 µl/min; Sample injection: 2400 s/40 min over both Flow Cells (FC1 and FC2); Dissociation time: 2400 s/40 min; Regeneration: 10 mM Glycine-HCl pH 1.5, 30 µl/min, 2x30 s.

Injections of analyte (AAV9) for each channel (cycles) were as follows: running buffer, AAV9 6E10 vp/ml, 1.25E11 vp/ml, 2.5E11 vp/ml, 5E11 vp/ml, 1E12 vp/ml, 2E12 vp/ml.

All sensorgrams were generated as reference subtracted and the responses as the difference between baseline before injection and signal just before end of injection.

### Biacore analysis of alkaline stability

After evaluation of AAV9 binding (see above) the same chips were subjected to repeated cycles of binding to AAV9 (5E11 vp/ml) followed by injection of NaOH (0.3 M) for the assessment of alkaline stability.

Biacore method for alkaline stability (per cycle): Running buffer: PBS-P+; Flow rate:
10 µl/min; Sample injection 1 (AAV9 5E11 vp/ml): 300 s over both Flow Cells; Dissociation time 1: 30 s; Sample injection 2 (0.3 M NaOH): 600 s over both flow cells; Dissociation time 2: 0 s; Regeneration: 10 mM Glycine-HCl pH 1.5, 30 µl/min, 2x30 s. This cycle was repeated 25 times to follow stability of AAV9 response values.

All sensorgrams were generated as reference subtracted and the responses as the difference between baseline before injection and signal just before end of injection.

### Results

The binding affinity analysis showed that the candidates vh98, vh100, vh103, vh105 had similar relative binding responses to AAV9, and never reached saturation even at the highest analyte concentration.

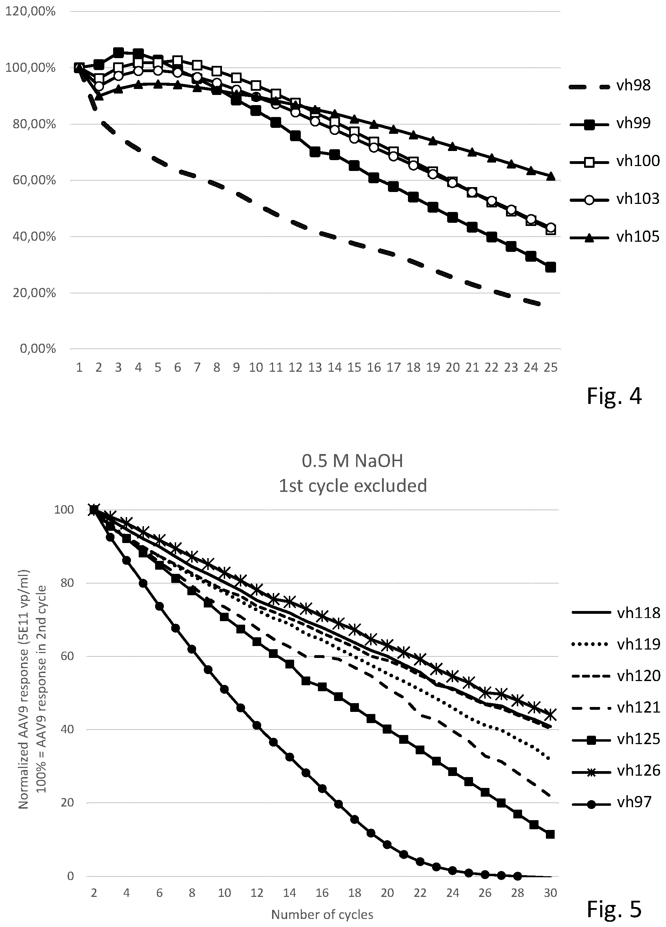

Fig. 4 shows the result of the alkaline stability study, all responses normalized to the response of the first cycle (i.e., before the first NaOH exposure). vh98 was moderately stable under exposure to 0.3 M NaOH, whereas vh100 and vh103 exhibited a slight improvement. vh105 demonstrated further improved stability, deteriorating at a slower rate than vh103. The results are summarized in Table 7. It can be concluded that both 19T and 23T are beneficial for stability. Also P40R resulted in improved stability.

**Table 7**

| **Candidate** | **Amino acids in Kabat positions 19, 23, 24, 40, 43, 44, 45, 76, 79, 89:** | **Alkaline stability (0.3 M NaOH)** |
|---|---|---|
| vh98 | 19R, 23V, 24A, 40P, 43K, 44A, 45R, 47F, 76N, 79Y, 89V | Moderate |
| vh99 | 19T, 23V, 24A, 40P, 43K, 44A, 45R, 47F, 76N, 79Y, 89V | Good |
| vh100 | 19R, 23T, 24A, 40P, 43K, 44A, 45R, 47F, 76N, 79Y, 89V | Good |
| vh103 | 19T, 23T, 24A, 40P, 43K, 44A, 45R, 47F, 76N, 79Y, 89V | Good |
| vh105 | 19T, 23T, 24A, 40R, 43K, 44A, 45R, 47F, 76N, 79Y, 89V | Good |

### Example 2B: Alkaline stability of sdAb variants having further framework variations

This example investigated the alkaline stability of AAV9-binding VHH variants having identical FWR3 (SEQ ID NO:45) and further variations in framework regions 1 (FWR1) and 2 (FWR2). The impact of mutations in the VHH framework was investigated to identify amino acid positions that enabled retaining high alkaline stability and AAV9 binding.

Candidate VHH sequences are identified in Table 8. CDRs were the same in all candidates.

**Table 8**

| | **FWR1** | | **FWR2** | | **Full sdAb** |
|---|---|---|---|---|---|
| | **variant** | **SEQ ID NO:** | **variant** | **SEQ ID NO:** | **SEQ ID NO:** |
| vh97 | 6E, 14A, 19R, 23V, 24A | 4 | 40P, 43K, 44A, 45R, 47F | 23 | 72 |
| vh118 | 6Q, 14A, 19R, 23T, 24I | 8 | 40R, 43K, 44E, 45R, 47F | 26 | 80 |
| vh119 | 6E, 14A, 19R, 23T, 24I | 9 | 40R, 43K, 44E, 45R, 47F | | 81 |
| vh120 | 6Q, 14A, 19R, 23T, 24V | 10 | 40R, 43K, 44E, 45R, 47F | | 82 |
| vh121 | 6Q, 14A, 19R, 23T, 24I | 8 | 40P, 43K, 44E, 45R, 47F | 27 | 83 |
| vh122 | 6Q, 14A, 19R, 23T, 24I | | 40R, 43K, 44A, 45R, 47F | 24 | 84 |
| vh123 | 6E, 14A, 19R, 23T, 24I | 9 | 40R, 43K, 44A, 45R, 47F | | 85 |
| vh124 | 6Q, 14A, 19R, 23T, 24V | 10 | 40R, 43K, 44A, 45R, 47F | | 86 |
| vh125 | 6Q, 14A, 19R, 23T, 24I | 8 | 40P, 43K, 44A, 45R, 47F | 23 | 87 |
| vh126 | 6Q, 14A, 19T, 23T, 24I | 11 | 40R, 43K, 44E, 45R, 47F | 26 | 88 |

The sdAb sequences were expressed with SEQ ID NO:159 as a C-terminal fusion via a peptide linker, and all constructs had a C-terminal tag (HHHHHHC). Total molecular weight was 21 kDa.

The following aspects were evaluated: (1) Affinity assessment of AAV9 interaction, tested through high concentration injection of AAV9. (2) Alkaline stability, tested by reduction of AAV9 binding after increasing number of treatments with 0.5 M NaOH.

### Materials and methods

### Generation of IgG Sepharose purified candidate polypeptides

Candidate polypeptides were produced and purified as described in Example 2A, except that to generate the crude lysates, the Falcon tubes were incubated in 48°C water bath for 2 h followed by pelleting of cell debris by centrifugation at 8000 g for 10 min instead of sonication on ice.

### Biacore analysis of binding affinity

To assess binding to AAV9, the candidate polypeptides were immobilized on a Biacore^{™} Series S CM5 chip. AAV9 (2E12 vp/ml) were used as analyte.

Materials and equipment used were as follows: Biacore^{™} Series S CM5 sensor chips (Cytiva^{™}); Biacore^{™} Amine coupling kit (Cytiva^{™}); Biacore^{™} Acetate buffer pH 5.0 (Cytiva^{™}); Biacore^{™} 8K+ instrument (Cytiva^{™}); AAV9 (prepared in-house); IgG Sepharose purified candidate polypeptides as described above.

Immobilization was performed using a standard method in Biacore software with coupling of candidate polypeptides in Flow Cell 2 (FC2) and activation/inactivation in Flow cell 1 (FC1). Candidate polypeptides were diluted in acetate buffer at a concentration of 25 µg/ml. The immobilization levels did vary somewhat between different polypeptide variants (average 4319 RU +SD 640 RU). The polypeptide was immobilized in FC2.

Biacore^{™} method for binding analysis: Running buffer: PBS-P+; Flow rate: 5 µl/min; Sample injection: 2400 s/40 min over both Flow Cells (FC1 and FC2); Dissociation time: 2400 s/40 min; Regeneration: 10 mM Glycine-HCl pH 1.5, 30 µl/min, 2x30 s.

Injections of analyte (AAV9) for each channel (cycles) were as follows: running buffer, AAV9 1.25E11 vp/ml, 5E11 vp/ml, 2E12 vp/ml.

All sensorgrams were generated as reference subtracted and the responses as the difference between baseline before sample injection and signal just before end of injection.

### Biacore analysis of alkaline stability

After evaluation of AAV9 binding (see above) the same chips were subjected to repeated cycles of binding to AAV9 (3E11 vp/ml) followed by injection of NaOH (0.5 M) for the assessment of alkaline stability.

Biacore method for alkaline stability (per cycle): Running buffer: PBS-P+; Flow rate:
10 µl/min; Sample injection 1 (AAV9 3E11 vp/ml): 300 s over both Flow Cells; Dissociation time 1: 30 s; Sample injection 2 (0.5 M NaOH): 600 s over both flow cells; Dissociation time 2: 0 s; Regeneration: 10 mM Glycine-HCl pH 1.5, 30 µl/min, 2x30 s. This cycle was repeated 30 times to follow stability of AAV9 response values.

All sensorgrams were generated as reference subtracted and the responses as the difference between baseline before injection and signal just before end of injection.

### Results

All candidates were found to have similar and high AAV9 binding capacity (Biacore response values in the range of approximately 12000-15000 RU; immobilization levels varying somewhat between different candidates), with vh97 exhibiting the highest capacity. It was concluded that the tested framework variations had no significant impact on target binding capacity.

The alkaline stability assessment study demonstrated that the candidates having one or more of the FWR1 or FWR2 variations set forth in Table 8 were more alkali tolerant than vh97. Fig. 5 shows the AAV9 response for increasing number of cycles involving of alkali exposure (0.5 M NaOH), normalized to the response of the second cycle (i.e. excluding the first cycle).

It can be seen in Fig. 5 that vh118 and vh120 have very similar NaOH resistance, and vh122 and vh124 (both omitted from graph) performed very similar, on the same level as vh119. This implies that A24I and A24V have comparable impact on NaOH resistance. Candidates having R in position 40 performed particularly well.

Candidate vh118 has better alkaline stability compared to vh119, and vh122 is more stable compared to vh123 (also omitted from graph; stability similar to vh121), indicating that the substitution E6Q can improve NaOH tolerance.

The candidates with E in position 44 performed better regarding NaOH resistance compared to the corresponding mutant with A in position 44 (vh118 vs vh122; vh119 vs vh123; vh120 vs vh124; vh121 vs vh125). This suggests that E in position 44 is more favorable to alkaline stability than A.

This example showed that even though the previously tested combination of FW mutations, such as vh98, show greatly enhanced alkaline stability as compared to native frameworks, additional substitutions, such as vh118, may render the resulting polypeptides even more stable. It is however concluded that also the variants lacking all the best mutations still are considered alkaline stable, and fulfilling the objective of the present disclosure to obtain alkaline stable antigen binding polypeptides.

### Example 3: Binding and stability of candidate polypeptides with FW point modifications

The impact of point mutations in the framework of an AAV9-binding VHH was investigated to identify amino acid positions that enabled a high alkaline stability and AAV9 binding. Based on SEQ ID NO:80, a class of AAV9 binding polypeptides having different point mutations were produced as set out in Tables 9a-c below.

The following aspects were evaluated: (1) Affinity assessment of AAV9 interaction (tested through high concentration injection of AAV9). (2) Alkaline stability (tested by reduction of AAV9 binding after increasing number of treatments with 0.5 M NaOH).

### Materials and methods

### Generation of Immobilized Metal Affinity Chromatography (IMAC) purified candidate polypeptides

5 µl BL21(DE3) glycerol stock of each variant, produced as described in Example 1B, was inoculated in 4 ml LB supplemented with 100 µg/ml carbenicillin in 14 ml round-bottom tube and incubated at 37°C overnight at 160 rpm.

Protein expression culture medium (TB medium supplemented with 100 µg/ml carbenicillin and 2 mM MgCl₂) was prepared and added to filled 500 ml baffled glass shake flasks (50 ml per flask). Each flask was inoculated with approximately 500 µl from the previous overnight culture to obtain a starting OD600 of 0.05. The flasks were incubated in Infors HT shaking incubator for approximately 3.5 hours at 37°C and 140 rpm shaking until OD600 reached 1.0. Thereafter 50 µl IPTG (1 M) was added to a final concentration of 1 mM and the flasks were incubated in Infors HT shaking incubator at 27°C and 140 rpm shaking for 18 hours, whereafter the cultures were transferred to Falcon tubes (50 mL). To generate crude variant lysates the Falcon tubes were incubated in 48°C water bath for 2 hours followed by pelleting of cell debris by centrifugation at 8000 g for 10 min and filtration of supernatant with a 0.22 µm filter.

Clarified lysate was loaded on HisTrap^{™} FF 1 mL chromatography columns (Cytiva^{™}), equilibrated in 50 mM sodium phosphate pH 7.5, 500 mM NaCl, allowing a residence time of about 2 min. The column was then washed with 15 column volumes (CV) of 50 mM sodium phosphate pH 7.5, 500 mM NaCl. Protein was eluted using 0.5 M imidazole pH 7.5-8, linear gradient (0-100 %) over 10 CV. Eluted protein was buffer-exchanged into phosphate buffered saline (Medicago) pH 7.4 using gravity flow columns prepacked with Sephadex^{™} G-25 resin (Cytiva) prior to further analysis.

Concentration of HisTrap^{™} FF purified samples was measured using NanoDrop (Thermo Scientific) according to manufacturer's instructions. Following purification, samples were kept in refrigerator in Eppendorf tubes until all measurements were performed and were then kept in freezer until Biacore analysis.

### Biacore analysis of binding affinity for AAV9

To assess binding to AAV9, the candidate polypeptides were immobilized on a Biacore^{™} Series S CM5 chip.

Materials and equipment used were as follows: Biacore^{™} Series S CM5 sensor chips, Biacore^{™} Amine coupling kit, Biacore^{™} Acetate buffer pH 5.0, Biacore^{™} 8K+ instrument (all from Cytiva^{™}); AAV9 (prepared in-house); HisTrap^{™} FF purified candidate polypeptides.

Immobilization was performed using a standard method in Biacore software with coupling of AAV9 binding polypeptide variants in Flow Cell 2 (FC2) and activation/inactivation in Flow cell 1 (FC1). AAV9 binding polypeptide variants were diluted in acetate buffer pH 5.0 at a concentration of 25 µg/ml. The immobilization levels varied somewhat between different polypeptide variants (average 3763 RU ±SD 500 RU).

In each run the polypeptide was immobilized in FC2. Multiple sensor chips were used until all candidates were tested.

Biacore^{™} method for binding analysis: Running buffer: PBS-P+ ; Flow rate: 5 µl/min ; Sample injection: 2400 s/40 min over both Flow Cells (FC1 and FC2) ; Dissociation time: 2400 s/40 min ; Regeneration: 10 mM Glycine-HCl pH 1.5, 30 µl/min, 2x30 s. Injections of analyte (AAV9) for each channel (cycles) were as follows: running buffer, AAV9 2E11 vp/ml, 5E11 vp/ml, 2E12 vp/ml.

All sensorgrams were generated as reference subtracted and the responses as the difference between baseline before injection and signal just before end of injection.

### Biacore analysis of alkaline stability

After evaluation of AAV9 binding (see above) the same chips were subjected to 40 repeating cycles of binding to AAV9 (5E11 vp/ml) followed by injection of NaOH (0.5 M).

Biacore method for alkaline stability (per cycle): Running buffer: PBS-P+ ; Flow rate: 10 µl/min ; Sample injection 1 (AAV9 5E11 vp/ml): 300 s over both flow cells; Dissociation time 1: 30 s ; Sample injection 2 (0.5 M NaOH): 600 s over both flow cells ; Dissociation time 2: 0 s ; Regeneration: 10 mM Glycine-HCl pH 1.5, 30 µl/min, 2x30 s. This cycle was repeated 40 times to follow stability of AAV9 response values.

All sensorgrams were generated as reference subtracted and the responses as the difference between baseline before injection and signal just before end of injection.

### Results

The response levels of AAV9 interactions of the candidate polypeptides were normalized to the response of SEQ ID NO: 80 and summarized in Tables 9a-c. It should be noted in this regard that vh118 (SEQ ID NO: 80) comprises a preferred framework having almost an optimal combination of mutations to the currently tested CDRs as compared to the native or synthetic starting frameworks. Thus, most other variants will show less alkaline stability as compared to SEQ ID NO: 80. The variants below all show stability as compared to the practically non-existent stability of the native frameworks, and the moderate stability of the synthetic starting frameworks. Thus, the mutations introduced in these variants are still stabilizing as compared to the original framework, but not as good as the most preferred mutations. A comparison between SEQ ID NO: 80 and the least preferred mutations is included in Example 8 below.

Alkaline stability was recorded as the number of cycles with at least 50 % binding capacity retained. The first cycle was excluded and binding capacity values were normalized against the binding capacity of the second cycle, i.e. binding after the first exposure to NaOH. In each cycle, 0.5 M NaOH was injected with a contact time of 10 min at 10 µl/min. The result for each candidate is presented in Tables 9a-c.

It was seen that a satisfactory alkaline stability was achieved for many of the investigated point mutations, especially in FWR1 and FWR2. An initial AAV9 binding capacity of at least 70 % of that of SEQ ID NO: 80 is considered satisfactory. With regard to alkaline stability, maintaining a binding capacity of at least 50 % after 12 cycles is preferable; a retained binding capacity of at least 50 % after 15 cycles being more preferred. Particularly preferred are those variants maintained at least 50 % binding capacity for 20 cycles or more such as at least 24 cycles of alkali exposure.

For example, it was found that in FWR2, position 40, all tested amino acids (R, T, I, K, A) resulted in very stable polypeptides. Also, in position 45, L and I gave good results both in terms of target binding and alkaline stability. In Kabat position 47, amino acids F and L resulted in very stable candidates. It was also seen that point mutations in FWR3, Kabat positions 76, 79 and 89 had a significant impact on alkaline stability. In position 76, N and Q were identified as beneficial. Amino acids Y, F and W in position 79 provided excellent alkaline stability, and A was acceptable. In Kabat position 89, V, I and L resulted in very stable polypeptides.

In FWR 1, 24I/S/V provided excellent alkaline stability.

**Table 9a. FWR1 variants.**

| sdAb | Kabat position mutation | FWR1 SEQ ID NO: | Full sdAb SEQ ID NO: | Normalized AAV9 binding capacity (%) | NaOH stability (no. of exposure cycles with ≥50% binding maintained) |
|---|---|---|---|---|---|
| vh118 | - | 8 | 80 | 100 | 23 |
| vh247 | Q5V | 12 | 89 | 106 | 23 |
| vh248 | Q5E | 13 | 90 | 105 | 21 |
| vh249 | Q6E | 9 | 91 | 86 | 19 |
| vh250 | A14S | 14 | 92 | 96 | 21 |
| vh251 | A14T | 15 | 93 | 99 | 22 |
| vh252 | R19K | 16 | 94 | 92 | 23 |
| vh253 | R19S | 17 | 95 | 86 | 17 |
| vh332 | T23V | 18 | 96 | 98 | 22 |
| vh254 | T23S | 19 | 97 | 73 | 22 |
| vh344 | T23A, V89I | 20 | 98 | 100 | 20 |
| vh255 | I24S | 21 | 99 | 103 | 25 |
| vh256 | I24V | 10 | 100 | 102 | 25 |

**Table 9b. FWR2 variants**

| | Kabat position mutation | FWR2 SEQ ID NO: | Full sdAb SEQ ID NO: | Normalized AAV9 binding capacity (%) | NaOH stability (no. of exposure cycles with ≥50% binding maintained) |
|---|---|---|---|---|---|
| vh118 | - | 26 | 80 | 100 | 23 |
| vh257 | R40T | 28 | 101 | 69 | 18 |
| vh258 | R40I | 29 | 102 | 87 | 19 |
| vh341 | R40K | 30 | 103 | 83 | 19 |
| vh345 | R40A, V89I | 31 | 104 | 101 | 18 |
| vh259 | K43G | 32 | 105 | 72 | 21 |
| vh260 | K43Q | 33 | 106 | 77 | 21 |
| vh261 | K43R | 34 | 107 | 105 | 21 |
| vh262 | E44Q | 35 | 108 | 104 | 20 |
| vh263 | E44A | 24 | 109 | 108 | 21 |
| vh264 | E44D | 36 | 110 | 92 | 21 |
| vh265 | E44G | 37 | 111 | 101 | 20 |
| vh266 | R45L | 38 | 112 | 115 | 24 |
| vh325 | R45I | 39 | 113 | 97 | 21 |
| vh346 | F47L, V89I | 40 | 114 | 100 | 18 |

**Table 9c. FWR3 variants**

| | Kabat position mutation | FWR3 SEQ ID NO: | Full sdAb SEQ ID NO: | Normalized AAV9 binding capacity (%) | NaOH stability (no. of exposure cycles with ≥50% binding maintained) |
|---|---|---|---|---|---|
| vh118 | - | 45 | 80 | 100.0 | 23 |
| vh327 | A60N | 46 | 118 | 109 | 20 |
| vh328 | A60Q | 47 | 119 | 110 | 14 |
| vh269 | A60T | 48 | 120 | 85 | 18 |
| vh270 | A60S | 49 | 121 | 103 | 22 |
| vh271 | A60V | 50 | 122 | 105 | 13 |
| Nb273 | N76S | 52 | 124 | 82 | 13 |
| Nb274 | N76E | 53 | 125 | 91 | 13 |
| vh275 | N76Q | 54 | 126 | 94 | 20 |
| Nb329 | N76D | 55 | 127 | 111 | 14 |
| Nb276 | Y79L | 56 | 128 | 92 | 13 |
| Nb277 | Y79V | 57 | 129 | 80 | 12 |
| vh278 | Y79A | 58 | 130 | 99 | 16 |
| vh279 | Y79F | 59 | 131 | 98 | 23 |
| vh330 | Y79W | 60 | 132 | 112 | 35 |
| vh280 | S82bN | 61 | 133 | 100 | 22 |
| vh281 | S82bT | 62 | 134 | 103 | 19 |
| vh282 | S82bQ | 63 | 135 | 88 | 19 |
| vh284 | V89A | 65 | 137 | 109 | 15 |
| vh286 | V89I | 67 | 139 | 112 | 22 |
| vh331 | V89L | 68 | 140 | 109 | 18 |
| vh344 | T23A, V89I | 67 | 141 | 100 | 20 |
| vh345 | R40A, V89I | 67 | 142 | 101 | 18 |
| vh346 | F47L, V89I | 67 | 143 | 100 | 18 |

### Example 4A: Alkaline stability of native vs modified sdAb frameworks

This example compares single domain antibodies having a native camelid sdAb framework to sdAb having a framework that is modified to enhance alkaline stability. The following aspects were evaluated: (1) Affinity assessment of target molecule interaction (tested through various concentration injection of target molecule). (2) Alkaline stability (tested by reduction of target binding after increasing number of treatments with 0.1 M or 0.3 M NaOH).

The single domain antibodies were VHH variants directed against AAV9, Green fluorescent protein (GFP) or epidermal growth factor receptor (EGFR) respectively, and were based on native camelid VHH frameworks or had a modified framework to enhance alkaline stability of the VHH variant as such. The native camelid frameworks corresponded to the framework sequences of the VHH "EgA1" or "9G8", respectively, described in Schmitz et al, 2013, Structure 21, 1214-1224, or to the framework sequence of a GFP-binding VHH "S-Nb3" described in Fleetwood et al., Cell. Mol. Life Sci. (2013) 70:1081-1093, except that in relation to the latter the first two amino acids were replaced by VD. CDR regions were the same for candidates binding the same target.

Each sdAb was expressed as a fusion protein, fused C-terminally to an SpA domain Z variant (SEQ ID NO:160) in which binding to the Fc and VH3 portions of IgG had been abolished. All constructs had a C-terminal linker (GS) followed by a His₆ tag.

The tested polypeptide candidates (fusion proteins) are outlined in Table 10.

**Table 10**

| Candidate no. | VHH variant | Target | Type of VHH framework | VHH sequence | Full protein sequence |
|---|---|---|---|---|---|
| 4A-1 | vh342 | AAV9 | native camelid (EgA1) | SEQ ID NO:152 | SEQ ID NO:163 |
| 4A-2 | vh343 | AAV9 | native camelid (9G8) | SEQ ID NO:153 | SEQ ID NO:164 |
| 4A-3 | vh118 | AAV9 | stabilized | SEQ ID NO:80 | SEQ ID NO:162 |
| 4A-4 | vh166 | EGFR | native (9G8) | SEQ ID NO:157 | SEQ ID NO:168 |
| 4A-5 | vh 167 | EGFR | stabilized | SEQ ID NO:158 | SEQ ID NO:169 |
| 4A-6 | vh25 | GFP | native camelid (S-Nb3) modified in position 1-2 only | SEQ ID NO:154 | SEQ ID NO:165 |
| 4A-7 | vh60 | GFP | native (EgA1) | SEQ ID NO:155 | SEQ ID NO:166 |
| 4A-8 | vh113 | GFP | stabilized | SEQ ID NO:156 | SEQ ID NO:167 |

### Materials and methods

### Generation of Immobilized Metal Affinity Chromatography (IMAC) purified candidate polypeptides

For each candidate 5 µl of BL21(DE3) glycerol stock produced as described in Example 1B was inoculated in 4 ml LB culture medium supplemented with 100 µg/ml carbenicillin in 14 ml round-bottom tube and incubated at 37°C overnight at 160 rpm.

Protein expression culture medium (TB medium supplemented with 100 µg/ml carbenicillin and 2 mM MgCl₂) was prepared and added to filled 500 ml baffled glass shake flasks (50 ml per flask). Each flask was inoculated with approximately 500 µl from the previous overnight culture to obtain a starting OD600 of 0.05. The flasks were incubated in Infors HT shaking incubator for approximately 3.5 hours at 37°C and 140 rpm shaking until OD600 reached 1.0. Thereafter 50 µl IPTG (1 M) was added to a final concentration of 1 mM and the flasks were incubated in Infors HT shaking incubator at 27°C and 140 rpm shaking for 18 hours, whereafter the cultures were transferred to Falcon tubes (50 mL). To generate crude variant lysates the Falcon tubes were incubated in 48°C water bath for 2 hours followed by pelleting of cell debris by centrifugation at 8000 g for 10 min and filtration of supernatant with a 0.22 µm filter.

The samples were purified using HisTrap^{™} FF 1mL chromatography column (Cytiva). Clarified lysate was loaded on the chromatography column, equilibrated in 50 mM sodium phosphate pH 7.5, 500 mM NaCl, allowing a residence time of about 2 min. The column was then washed with 15 column volumes (CV) of 50 mM sodium phosphate pH 7.5, 500 mM NaCl. Protein was eluted using 0.5 M imidazole pH 7.5 - 8, linear gradient (0-100 %) over 10 CV. Eluted protein was buffer-exchanged into phosphate buffered saline (Medicago) pH 7.4 using gravity flow columns prepacked with Sephadex^{™} G-25 resin (Cytiva) prior to further analysis.

Concentration of purified and buffer exchanged samples was measured using NanoDrop (Thermo Scientific) according to manufacturer's instructions. Following purification, samples were kept in refrigerator in Eppendorf tubes until all measurements were performed and were then kept in freezer until Biacore analysis.

### Biacore analysis of binding affinity for target molecules

To assess binding to target molecule (AAV9, GFP or EGFR), candidate polypeptides were immobilized on a Biacore^{™} Series S CM5 chip (Cytiva) using Amine coupling kit (Cytiva) and analyzed with a Biacore^{™} 8K+ instrument (Cytiva). The respective analytes used were AAV9 (2E13 vp/mL), GFP (1 g/L) and EGFR (0.2 g/L).

Immobilization was performed using a standard method in Biacore software with coupling of polypeptide variants in Flow Cell 2 (FC2) and activation/inactivation in Flow cell 1 (FC1). Polypeptide variants were diluted in Biacore^{™} Acetate buffer pH 5.0 (Cytiva^{™}) at a concentration of 25 µg/ml. The immobilization levels on CM5 chip ranged between about 1000 and 3300 RU but were generally around 3000 RU.

In each run the polypeptide was immobilized in FC2. Multiple sensor chips were used until all candidates were tested.

Biacore^{™} method for binding analysis to AAV9: Running buffer: PBS-P+; Flow rate: 5 µl/min ; Sample injection: 2400 s/40 min over both Flow Cells (FC1 and FC2) ; Dissociation time: 2400 s/40 min; Regeneration: 10 mM Glycine-HCl pH 1.5, 30 µl/min, 2x30 s. Injections of analyte (AAV9) for each channel (cycles) were as follows: running buffer, 2E11 vp/ml, 5E11 vp/ml, 2E12 vp/ml.

Biacore^{™} method for binding analysis to GFP or EGFR: Running buffer: PBS-P+ ; Flow rate: 10 µl/min ; Sample injection: 600 s/10 min over both Flow Cells (FC1 and FC2) ; Dissociation time: 600 s/10 min; Regeneration: 10 mM Glycine-HCl pH 1.5, 30 µl/min, 2x30 s. Injections of analyte (GFP) for each channel immobilized with GFP binding polypeptides (cycles) were as follows: running buffer, 10 nM, 25 nM and 100 nM. Injections of analyte (EGFR) for each channel immobilized with EGFR binding polypeptides (cycles) were as follows: running buffer, 6.25 nM, 12.5 nM, 25 nM, 50 nM, 100 nM and 200 nM.

All sensorgrams were generated as reference subtracted and the responses as the difference between baseline before injection and signal just before end of injection.

### Biacore analysis of alkaline stability

After the binding analysis (see above) the same CM5 chips having immobilized polypeptides were subjected to repeated cycles of binding to the respective analyte followed by injection of NaOH for the assessment of alkaline stability (0.3 M for AAV9 binding polypeptides and 0.1 M for GFP and EGFR binding polypeptides, respectively).

Biacore method for alkaline stability (per cycle): Running buffer: PBS-P+ ; Flow rate: 10 µl/min ; Sample injection 1 (AAV9 5E11 vp/ml or GFP 50 nM or EGFR 75 nM): 300 s over both flow cells ; Dissociation time 1: 30 s; Sample injection 2 (0.1 M or 0.3 M NaOH): 600 s over both flow cells; Dissociation time 2: 0 s ; Regeneration: 10 mM Glycine-HCl pH 1.5, 30 µl/min, 2x30 s. This cycle was repeated 40 times to follow stability of target response values.

All sensorgrams were generated as reference subtracted and the responses as the difference between baseline before injection and signal just before end of injection.

### Results

All fusion proteins showed satisfactory binding to the respective target, and tables 11a-c summarize the results of the binding capacity and alkaline stability measurements.

**Table 11a. AAV9 binding candidates**

| Sample | Binding capacity AAV9 (2E12 vp/ml) (RU) | Alkaline stability (0.3 M) | |
|---|---|---|---|
| | | No. of cycles with at least 50 % binding maintained (100% = response of 1^{st} cycle) | No. of cycles with at least 50 % binding maintained (100% = response of 2^{nd} cycle) |
| 4A-1 | 9924 | 1 | 2 |
| 4A-2 | 12361 | 1 | 2 |
| 4A-3 | 12443 | 26 | 34 |

**Table 11b. EGFR binding candidates**

| Sample | Binding capacity EGFR (200 nM) (RU) | Alkaline stability (0.1 M) | |
|---|---|---|---|
| | | No. of cycles with at least 50 % binding maintained (100% = response of 1^{st} cycle) | No. of cycles with at least 50 % binding maintained (100% = response of 2^{nd} cycle) |
| 4A-4 | 2813 | 1 | 2 |
| 4A-5 | 2352 | 6 | 28 |

**Table 11c. GFP binding candidates**

| Sample | Binding capacity GFP (100 nM) (RU) | Alkaline stability (0.1 M) | |
|---|---|---|---|
| | | No. of cycles with at least 50 % binding maintained (100% = response of 1^{st} cycle)) | No. of cycles with at least 50 % binding maintained (100% = response of 2^{nd} cycle) |
| 4A-6 | 1124 | 1 | 2 |
| 4A-7 | 1124 | 1 | 8 |
| 4A-8 | 868 | 26 | >40 |

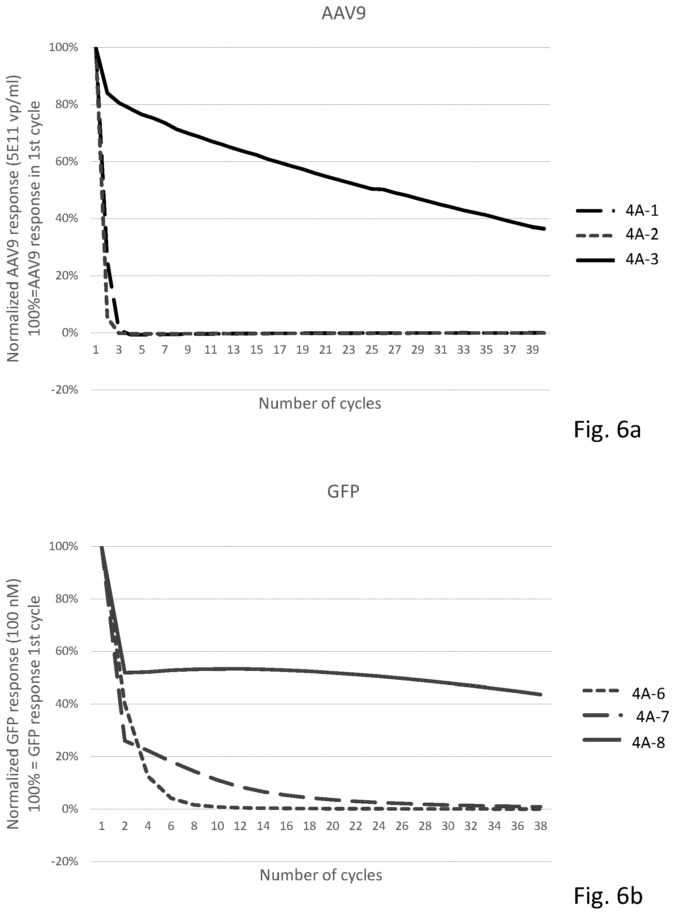

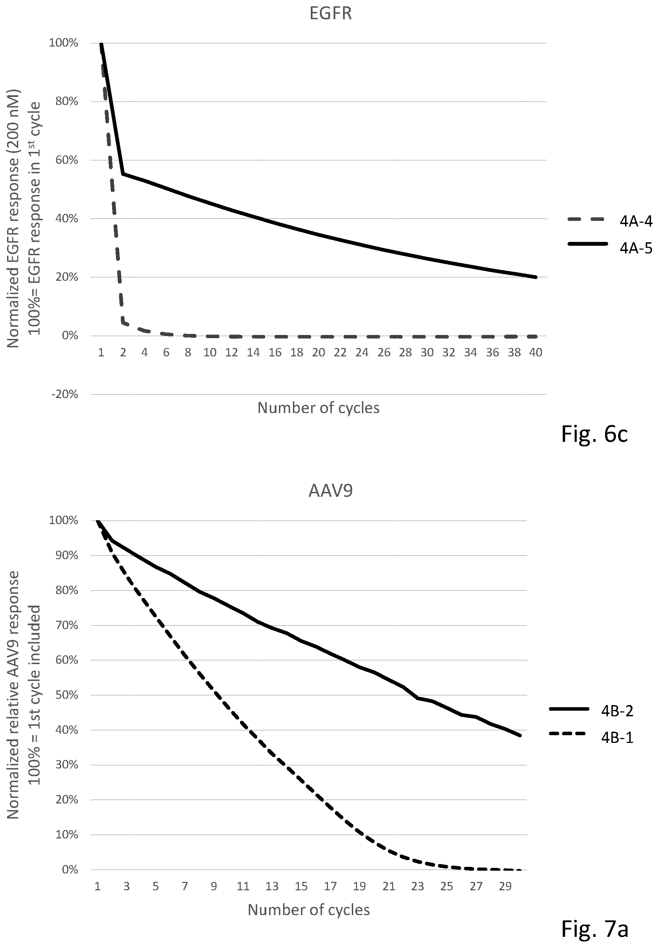

The target binding capacities, measured for the highest tested analyte concentration (report point late binding) showed small or no difference in target binding capacities between the candidates have different framework sequences. However, the differences in alkaline stability were striking. Figs. 6a-c show the binding capacities obtained for increasing number of cycles involving NaOH exposure (sample injection 2 above) as normalized to the response of the first cycle for the AAV9 binding polypeptides (Fig. 6a), GFP-binding polypeptides (Fig. 6b) and EGFR-binding polypeptides (Fig. 6c). As can be seen in these figures, VHH affinity ligands based on stabilized VHH framework (4A-3, 4A-5, 4A-8) showed improved alkaline stability in relation to the reference VHH having a native camelid VHH framework. In fact, non-stabilized candidates 4A-1 and 4A-2 lost virtually all target binding response after the first cycle.

### Example 4B: Alkaline stability of various sdAb frameworks

This example compares single domain antibodies having a native camelid or synthetic sdAb framework to sdAbs having a framework that was modified to enhance alkaline stability. The following aspects were evaluated:
(1) Affinity assessment of target molecule interaction (tested through high concentration injection of target molecule).
(2) Alkaline stability (tested by reduction of target binding after increasing number of treatments with 0.1, 0.3 or 0.5 M NaOH).

The single domain antibodies were VHH variants directed against AAV9, Green fluorescent protein (GFP) or epidermal growth factor receptor (EGFR), and either were based on a native camelid VHH framework, a synthetic framework, or had a synthetic stabilized framework modified to enhance alkaline stability of the VHH variant as such. The native camelid frameworks corresponded to the framework sequences of the VHH "EgA1" or "9G8", respectively, described in Schmitz et al. The CDRs were the same for candidates binding the same target.

Each sdAb was expressed as a fusion protein, fused C-terminally to an SpA domain Z variant (SEQ ID NO:159).

The tested polypeptide candidates (fusion proteins) are outlined in Table 12.

**Table 12**

| Candidate no. | VHH variant | Target | Type of VHH framework | VHH sequence | Full protein sequence |
|---|---|---|---|---|---|
| 4B-1 | vh97 | AAV9 | synthetic | SEQ ID NO:72 | SEQ ID NO:170 |
| 4B-2 | vh118 | AAV9 | Synthetic, stabilized | SEQ ID NO:80 | SEQ ID NO:171 |
| 4B-3 | vh166 | EGFR | Native (9G8) | SEQ ID NO:157 | SEQ ID NO:174 |
| 4B-4 | vh167 | EGFR | Synthetic, stabilized | SEQ ID NO:158 | SEQ ID NO:175 |
| 4B-5 | vh60 | GFP | Native (EgA1) | SEQ ID NO:155 | SEQ ID NO:172 |
| 4B-6 | vh113 | GFP | Synthetic, stabilized | SEQ ID NO:156 | SEQ ID NO:173 |

### Materials and methods

For each candidate 5 µl of BL21(DE3) glycerol stock produced as described in Example 1A was inoculated in 4 ml LB culture medium supplemented with 50 µg/ml kanamycin in 14 ml round-bottom tube and incubated at 37°C overnight at 160 rpm.

Protein expression culture medium (TB medium supplemented with 50 µg/ml kanamycin and 2 mM MgCl₂) was prepared and added to filled 100 ml baffled glass shake flasks (20 ml per flask). Each flask was inoculated with approximately 100 µl from the previous overnight culture to obtain a starting OD600 of 0.05. The flasks were incubated in Infors HT shaking incubator for approximately 3 hours at 37°C and 140 rpm shaking until OD600 reached 1.0. Thereafter 20 µl IPTG (1 M) was added to a final concentration of 1 mM and the flasks were incubated in Infors HT shaking incubator at 27°C and 140 rpm shaking for 18 hours, whereafter the cultures were transferred to Falcon tubes (50 mL). To generate crude variant lysates the Falcon tubes were incubated in 48°C water bath for 2 hours followed by pelleting of cell debris by centrifugation at 8000 g for 10 min and filtration of supernatant with a 0.22 µm filter.

The samples were purified using gravity flow in PD-10 columns (Cytiva^{™}) packed in-house with IgG Sepharose 6FF chromatography resin (Cytiva^{™}). Clarified lysate was loaded on the column, equilibrated in 50 mM Tris, 150 mM NaCl, 0.05% Tween20 (TST buffer). The column was washed with 10 column volumes (CV) of TST buffer followed by 2 CV of 2 CV 5 mM NH₄Ac pH 5. Protein was eluted using 2.5 ml 0.5 M Hac. Eluted protein was buffer-exchanged into phosphate buffered saline (Medicago) pH 7.4 using gravity flow columns PD-10 (Cytiva) prior to further analysis.

Concentration of IgG Sepharose purified samples was measured using NanoDrop (Thermo Scientific) according to manufacturer's instructions. Following purification, samples were kept in refrigerator in Eppendorf tubes until all measurements were performed and were then kept in freezer until Biacore analysis.

### Biacore analysis of binding affinity for target molecules

To assess binding to target molecule (AAV9, GFP or EGFR), candidate polypeptides were immobilized on a Biacore^{™} Series S CM5 chip (Cytiva) using Amine coupling kit (Cytiva) and analyzed with a Biacore^{™} 8K+ instrument (Cytiva). The respective analytes used were AAV9 (prepared in-house), trastuzumab-GFP (prepared in-house) and EGFR (SinoBiological, 10001-H08H), Fc from mAb (prepared inhouse).

Immobilization was performed using a standard method in Biacore software with coupling of polypeptide variants in Flow Cell 2 (FC2) and activation/inactivation in Flow cell 1 (FC1). AAV9 binding candidates were diluted in Biacore^{™} Acetate buffer pH 5.0 (Cytiva^{™}) at a concentration of 25 µg/ml, and the average immobilization levels was 4319 RU ±SD 640 RU. GFP binding polypeptide variants were diluted in Biacore^{™} Acetate buffer pH 4.5 (Cytiva^{™}) at a concentration of 25 µg/ml, and average immobilization level was 3803 RU ±SD 302 RU). EGFR binding polypeptide variants were diluted in Biacore^{™} Acetate buffer pH 4.5 (Cytiva^{™}) at a concentration of 25 µg/ml, and average immobilization level was 3719 RU ±SD 463 RU). The polypeptides were immobilized in FC2.

Biacore^{™} method for AAV9 binding analysis: Running buffer: PBS-P+ ; Flow rate: 5 µl/min ; Sample injection: 2400 s/40 min over both FC1 and FC2; Dissociation time: 2400 s/40 min ; Regeneration: 10 mM Glycine-HCl pH 1.5, 30 µl/min, 2x30 s. Injections of analyte (AAV9) for each channel (cycles) were as follows: running buffer, 1.25E11 vp/ml, 5E11 vp/ml, 2E12 vp/ml.

Biacore^{™} method for GFP binding analysis: Running buffer: PBS-P+ ; Flow rate: 10 µl/min ; Block injection: 90 s over both FC1 and FC2; Sample injection: 600 s over both FC1 and FC2; Dissociation time: 1800 s; Regeneration: 10 mM Glycine-HCl pH 1.5, 30 µl/min, 2x30 s. Injections of block (Fc) for each channel (cycles) were 2 µM. Injections of analyte (trastuzumab-GFP) for each channel (cycles) were as follows: running buffer, 1, 10, 100, 1000 nM.

Biacore^{™} method for EGFR binding analysis: Running buffer: PBS-P+ ; Flow rate: 10 µl/min ; Sample injection: 600 s over both FC1 and FC2; Dissociation time: 1800 s; Regeneration: 10 mM Glycine-HCl pH 1.5, 30 µl/min, 2x30 s. Injections of analyte (EGFR) for each channel (cycles) were as follows: running buffer, 0.1-200 nM in 1:1 dilution steps.

All sensorgrams were generated as reference subtracted and the responses as the difference between baseline before sample injection and signal just before end of injection.

### Biacore analysis of alkaline stability

After the binding analysis (see above) the same CM5 chips having immobilized polypeptides were subjected to repeated cycles of binding to the respective analyte followed by injection of NaOH for the assessment of alkaline stability (0.1/0.3/0.5 M).

Biacore method for AAV9 alkaline stability (per cycle): Running buffer: PBS-P+ ; Flow rate: 10 µl/min ; Sample injection 1 (AAV9 3E11 vp/ml): 300 s over both flow cells; Dissociation time 1: 30 s; Sample injection 2 (0.5 M NaOH): 600 s over both flow cells; Dissociation time 2: 0 s; Regeneration: 10 mM Glycine-HCl pH 1.5, 30 µl/min, 2x30 s.

Biacore method for GFP alkaline stability (per cycle): Running buffer: PBS-P+ ; Flow rate: 10 µl/min; Block injection: (Fc from mAb 2 µM) 90 s over both flow cells; Dissociation time block: 0 s; Sample injection 1 (trastuzumab-GFP 2µM ): 300 s over both flow cells ; Dissociation time 1: 30 s; Sample injection 2 (0.3 M NaOH): 600 s over both flow cells ; Dissociation time 2: 0 s; Regeneration: 10 mM Glycine-HCl pH 1.5, 30 µl/min, 2x30 s.

Biacore method for EGFR alkaline stability (per cycle): Running buffer: PBS-P+ ; Flow rate: 10 µl/min; Sample injection 1 (EGFR 0.1 M): 300 s over both flow cells; Dissociation time 1: 30 s; Sample injection 2 (0.1 M NaOH): 600 s over both flow cells; Dissociation time 2: 0 s; Regeneration: 10 mM Glycine-HCl pH 1.5, 30 µl/min, 2x30 s.

These cycles were repeated 30 times to follow stability of target response values.

All sensorgrams were generated as reference subtracted and the responses as the difference between baseline before injection and signal just before end of injection.

### Results

All fusion proteins showed satisfactory binding to the respective target.

Tables 13a-c summarize the results of the binding capacity and alkaline stability measurements.

**Table 13a. AAV9 binding candidates**

| Sample | Binding capacity | Alkaline stability (0.5 M NaOH) | |
|---|---|---|---|
| | AAV9 response in analyte binding late (2E12 vp/ml, RU) | No. of cycles with at least 50 % binding maintained (100% = response of 1^{st} cycle) | No. of cycles with at least 50 % binding maintained (100%=response of 2^{nd} cycle) |
| 4B-1 | 14983 | 9 | 10 |
| 4B-2 | 12193 | 22 | 24 |

**Table 13b. EGFR binding candidates**

| Sample | Binding capacity | Alkaline stability (0.1 M NaOH) | |
|---|---|---|---|
| | EGFR response in analyte binding late (200 nM, RU) | No. of cycles with at least 50 % binding maintained (100% = response of 1^{st} cycle) | No. of cycles with at least 50 % binding maintained (100%=response of 2^{nd} cycle) |
| 4B-3 | 2234 | 1 | 2 |
| 4B-4 | 1896 | 13 | 24 |

**Table 13c. GFP binding candidates**

| Sample | Binding capacity | Alkaline stability (0.3 M NaOH) | |
|---|---|---|---|
| | trastuzumab-eGFP response in analyte binding late (1 µM, RU) | No. of cycles with at least 50 % binding maintained (100% = response of 1^{st} cycle) | No. of cycles with at least 50 % binding maintained (100%=response of 2^{nd} cycle) |
| 4B-5 | 4304 | 1 | 5 |
| 4B-6 | 3906 | >30 | >30 |

Figs. 7a-c show the binding capacities obtained for increasing number of cycles involving NaOH exposure (sample injection 2) as normalized to the response of the first cycle (i.e., including the first cycle) for the AAV9 binding fusion proteins (Fig. 7a), GFP-binding fusion proteins (Fig. 7b) and EGFR-binding fusion proteins (Fig. 7c). As can be seen in these figures, fusion proteins in which the VHH affinity ligand was based on stabilized VHH framework (4B-2, 4B-4, 4B-6) showed improved alkaline stability in relation to the corresponding fusion protein incorporating a VHH having a native camelid VHH framework (4B-3, 4B-5) or a synthetic but not optimally stabilized framework (4B-1).

### Example 5: AAV9 binding polypeptides having different framework and CDR variants.

This example further investigated two different sets of CDR sequences (both directed to AAV9) in different, stabilized sdAb frameworks. The candidate polypeptides tested were expressed as fusion protein in which the respective sdAb had SEQ ID NO: 160 fused to its C-terminal via a linker, and had a C-terminal (His)₆-tag. The sdAb sequences variants are presented in Table 14. FWR4 was the same for all candidates (SEQ ID NO:71).

The following aspects were evaluated: (1) Affinity assessment of AAV9 interaction (tested through high concentration injection of AAV9). (2) Alkaline stability (tested by reduction of AAV9 binding after increasing number of treatments with 0.5 M NaOH).

**Table 14**

| | FWR1 | FWR2 | FWR3 | CDR1/CDR2/CDR3 | Full sdAb |
|---|---|---|---|---|---|
| vh118 | QVQLQQSGGG SVQAGGSLRL SCTISG (SEQ ID NO: 8) | WFRQRPGKEREFVA (SEQ ID NO:26) | YADSVKGRFT ISRDNAKNTV YLQMNSLKPE DTAVYYCA (SEQ ID NO:45) | SEQ ID NO:176/SEQ ID NO:180/SEQ ID NO:184 | SEQ ID NO:80 |
| vh374 | QVQLQQSGGG SVQAGGSLRL SCTSSG (SEQ ID NO:21) | WFRQRPGRAREFVA (SEQ ID NO:41) | YTDSVKGRFT ISRDNAKNTV FLQMQSLKPE DTAIYYCAA (SEQ ID NO:69) | SEQ ID NO:177/SEQ ID NO:181/SEQ ID NO:185 | SEQ ID NO:144 |
| vh359 | QVQLVESGGG SVQAGGSLSL SCTISG (SEQ ID NO:22) | WFRQRPGKEREFVA (SEQ ID NO:26) | YTDSVKGRFT ISRDNAKNTV YLQMQSLKPE DTAVYYCAA (SEQ ID NO:70) | | SEQ ID NO:145 |
| vh428 | QVQLQQSGGG SVQAGGSLRL SCTISG (SEQ ID NO: 8) | WFRQRPGKEREFVA (SEQ ID NO:26) | YADSVKGRFT ISRDNAKNTV YLQMNSLKPE DTAVYYCA (SEQ ID NO:45) | | SEQ ID NO:146 |

### Materials and methods

### Generation of Immobilized Metal Affinity Chromatography (IMAC) purified candidate polypeptides

For each variant, 5 µl glycerol stock produced as described in Example 1B was inoculated in 4 ml LB culture medium supplemented with 100 µg/ml carbenicillin in 14 ml round-bottom tubes and grown over night at 37°C, 200 rpm agitation. Protein expression culture medium (TB medium supplemented with 100 µg/ml carbenicillin and 2 mM MgCl₂) was prepared and added to filled 500 ml baffled glass shake flasks (50 ml per flask, 7 flasks in total). Each flask was inoculated with approximately 500 µl from the previous overnight culture to obtain a starting OD600 of 0.05. The flasks were incubated in Infors HT shaking incubator for approximately 3.5 hours at 37°C and 140 rpm shaking until OD600 reached 1.0. Thereafter 50 µl IPTG (1 M) was added to a final concentration of 1 mM and the flasks were incubated in Infors HT shaking incubator at 27°C and 140 rpm shaking for 18 hours, whereafter the cultures were transferred to Falcon tubes (50 mL). To generate crude variant lysates the Falcon tubes were incubated in 48°C water bath for 2 hours followed by pelleting of cell debris by centrifugation at 8000 g for 10 min and filtration of supernatant with a 0.45 µm filter.

Clarified lysate was loaded on HisTrap^{™} FF 1 mL chromatography columns (Cytiva^{™}), equilibrated in 50 mM sodium phosphate pH 7.5, 500 mM NaCl, allowing a residence time of about 2 min. The column was then washed with 15 column volumes (CV) of 50 mM sodium phosphate pH 7.5, 500 mM NaCl. Protein was eluted using 0.5 M imidazole pH 7.5-8, linear gradient (0-100 %) over 10 CV. Eluted protein was buffer-exchanged into phosphate buffered saline (Medicago) pH 7.4 using gravity flow columns prepacked with Sephadex^{™} G-25 resin (Cytiva) prior to further analysis.

Concentration of HisTrap^{™} FF purified samples was measured using NanoDrop (Thermo Scientific) according to manufacturer's instructions. Following purification, samples were kept in refrigerator in Eppendorf tubes until all measurements were performed and were then kept in freezer until Biacore analysis. Protein mass was determined by liquid chromatography-mass spectrometry on a ACQUITY Rda Detector (Waters^{™}) using a BioRessolve column (Waters^{™}).

### Biacore analysis of binding affinity for AAV9

To assess binding to AAV9, the AAV9 binding polypeptides were immobilized on a Biacore^{™} Series S CM5 chip. AAV9 (2E12 viral particles (vp)/ml) was used as analyte.

Materials and equipment used were as follows: Biacore^{™} Series S CM5 sensor chips, Biacore^{™} Amine coupling kit, Biacore^{™} Acetate buffer pH 5.0, Biacore^{™} 8K+ instrument (all from Cytiva, Sweden); AAV9 (prepared in-house); candidate polypeptides generated as described above.

Immobilization was performed using a standard method in Biacore^{™} software with coupling of AAV9 binding polypeptide variants in Flow Cell 2 (FC2) and activation/inactivation in Flow cell 1 (FC1). AAV9 binding polypeptide variants were diluted in acetate buffer pH 5.0 at a concentration of 25 µg/ml. The immobilization levels obtained were 3001 +/- 159 RU. In each run the polypeptide was immobilized in FC2.

Running buffer: PBS-P+; Flow rate: 5 µl/min; Sample injection: 2400 s/40 min over both Flow Cells (FC1 and FC2); Dissociation time: 2400 s/40 min; Regeneration: 10 mM Glycine-HCl pH 1.5, 30 µl/min, 2x30 s. Injections of analyte (AAV9) for each channel (cycles) were as follows: running buffer, AAV9 2E11 vp/ml, 5E11 vp/ml, 2E12 vp/ml.

All sensorgrams were generated as reference subtracted and the responses as the difference between baseline before injection and signal just before end of injection.

### Biacore analysis of alkaline stability

Assessment of alkaline stability was done on the Biacore^{™} chips prepared above. After evaluation of AAV9 binding (see above), the samples were subjected to followed by repeated cycles of binding to AAV9 (5E11 vp/ml) followed by injection of NaOH (0.5 M).

Biacore method for alkaline stability (per cycle): Running buffer: PBS-P+; Flow rate: 10 µl/min; Sample injection 1 (AAV9 5E11 vp/ml): 300 s over both Flow Cells; Dissociation time 1: 30 s; Sample injection 2 (0.5 M NaOH): 600 s over both flow cells; Dissociation time 2: 0 s; Regeneration: 10 mM Glycine-HCl pH 1.5, 30 µl/min, 2x30 s. This cycle was repeated 40 times to follow stability of AAV9 response values.

All sensorgrams were generated as reference subtracted and the responses as the difference between baseline before injection and signal just before end of injection.

### Results

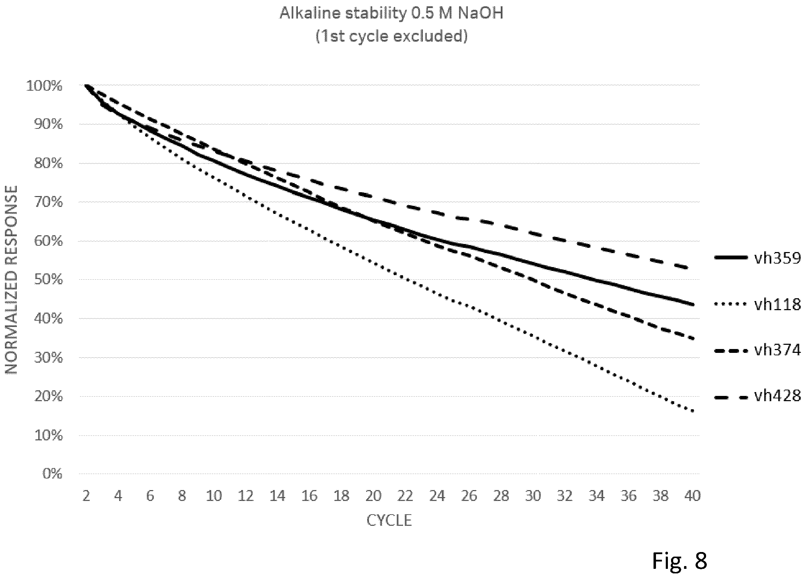

The response levels of AAV9 interactions of the candidate polypeptides using the maximum AAV9 concentration (2E12 vp/ml) was normalized to the response of vh118 and summarized in Table 15, recording alkaline stability as the number of cycles with at least 50 % binding capacity retained. Fig. 8 presents the result of the alkaline stability assessment, plotting the binding capacity against the no. of cycles. The binding response values were normalized against the binding capacity recorded for the second cycle (i.e., excluding the first cycle). In each cycle, 0.5 M NaOH was injected with a contact time of 10 min at 10 µl/min.

It was seen that candidates vh374, vh359 and vh428, which had the same set of CDRs but differed in the framework regions, had even higher alkaline stability than vh118.

**Table 15**

| | Normalized AAV9 binding capacity (%) | NaOH stability (no. of exposure cycles with at least 50% binding maintained) |
|---|---|---|
| vh118 | 100.0 | 22 |
| vh374 | 102.2 | 30 |
| vh359 | 86.4 | 34 |
| vh428 | 107.6 | 40 |

### Example 6: Comparative study of AAV9 binding and stability

In this example, 7 candidate polypeptides were tested and compared to a commercially available affinity ligand (CaptureSelect^{™} Biotin Anti-AAV9 Conjugate, ThermoFischer Scientific). The candidate polypeptides were expressed as fusion proteins in which the respective sdAb had SEQ ID NO: 160 fused to its C-terminal via a peptide linker (AA), and all constructs had a C-terminal tag (HHHHHHC). The sdAb sequences variants are presented in Table 16. FWR sequences were identical for all candidates.

**Table 16**

| | CDR1/CDR2/CDR3 SEQ ID NO | Framework variations (Kabat positions) | FWR1/FWR2/FWR3 SEQ ID NO | sdAb SEQ ID NO: |
|---|---|---|---|---|
| vh118 | SEQ ID NO:176/ SEQ ID NO:180/ SEQ ID NO:184 | 6Q, 19R, 23T, 24I, 40R, 44E, 45R, 79Y, 89V | SEQ ID NO:8 / SEQ ID NO:26 / SEQ ID NO:45 | 80 |
| vh97 | SEQ ID NO:176/ SEQ ID NO:180/ SEQ ID NO:184 | 6E, 19R, 23V, 24A, 40P, 44A, 45R, 79Y, 89V | SEQ ID NO:4/SEQ ID NO:23 / SEQ ID NO:45 | 72 |
| vh376 | SEQ ID NO:178/ SEQ ID NO:182/ SEQ ID NO:186 | 6Q, 19R, 23T, 24I, 40R, 44E, 45R, 79Y, 89V | SEQ ID NO:8 / SEQ ID NO:26 / SEQ ID NO:45 | 147 |
| vh324 | SEQ ID NO:179/ SEQ ID NO:183/ SEQ ID NO:187 | 6Q, 19R, 23T, 24I, 40R, 44E, 45R, 79Y, 89V | SEQ ID NO:8 / SEQ ID NO:26 / SEQ ID NO:45 | 148 |
| vh377 | SEQ ID NO:179/ SEQ ID NO:183/ SEQ ID NO:188 | 6Q, 19T, 23T, 24V, 40R, 44E, 45L, 79W, 89V | SEQ ID NO:190 / SEQ ID NO:38 / SEQ ID NO:60 | 149 |
| vh378 | SEQ ID NO:179/ SEQ ID NO:183/ SEQ ID NO:189 | 6Q, 19R, 23T, 24S, 40R, 44E, 45R, 79W, 89V | SEQ ID NO:21 / SEQ ID NO:26 / SEQ ID NO:60 | 150 |
| vh379 | SEQ ID NO:179/ SEQ ID NO:183/ SEQ ID NO:189 | 6Q, 19R, 23T, 24V, 40R, 44E, 45R, 79F, 89L | SEQ ID NO:10 / SEQ ID NO:26 / SEQ ID NO:191 | 151 |

### Materials and methods

IMAC purified candidate polypeptides were generated as described in Example 5.

### Biotinylation of purified candidate polypeptides

Purified candidate polypeptides were biotinylated at the C-terminal cysteine using EZ-Link^{™} Maleimide-PEG2-Biotin, No-Weigh^{™} Format (ThermoFisher Scientific) with a 2X molar excess of biotin.

### SEC purification of biotinylated candidate polypeptides

Proteins were additionally purified by size exclusion chromatography (SEC) for separation from excess biotin, non-biotinylated protein dimers and other impurities. SEC was performed in phosphate buffered saline (Medicago) pH 7.4 at a flow rate of 0.75 mL/min using Superdex^{™} 75 Increase 10/300 GL chromatography column (Cytiva). Proteins of interest eluted as sharp symmetric peak. Purest fractions (as analyzed by SDS-PAGE) were pooled and used for further analyses.

### Biacore analysis of binding affinity for AAV9

To assess binding to AAV9, the AAV9 binding polypeptide candidates and the reference CaptureSelect^{™} Biotin Anti-AAV9 Conjugate (Thermo Scientific^{™}) were immobilized on a Biacore^{™} Series S SA chip. AAV9 (2E12 viral particles (vp)/ml) was used as analyte.

Materials and equipment used were as follows: Biacore^{™} Series S SA sensor chips, Biacore^{™} 8K+ instrument (all from Cytiva, Sweden); AAV9 (prepared in-house); candidate polypeptides generated as described above; CaptureSelect^{™} Biotin Anti-AAV9 Conjugate (Thermo Scientific^{™}).

Immobilization was performed using a standard method in Biacore^{™} software with coupling of biotinylated AAV9 binding polypeptide variants and CaptureSelect^{™} Biotin Anti-AAV9 Conjugate (Thermo Scientific^{™}) in Flow Cell 2 (FC2) and activation/inactivation in Flow cell 1 (FC1). Biotinylated AAV9 binding polypeptide variants were diluted in PBS at a concentration of 100 µg/ml and CaptureSelect^{™} Biotin Anti-AAV9 Conjugate (Thermo Scientific^{™}) was diluted in PBS at a concentration of 10 µg/ml.

Biacore^{™} method for binding analysis: Running buffer: PBS-P+; Flow rate: 5 µl/min; Sample injection: 2400 s/40 min over both Flow Cells (FC1 and FC2); Dissociation time: 2400 s/40 min; Regeneration: 10 mM Glycine-HCl pH 1.5, 30 µl/min, 2x30 s. Injections of analyte (AAV9) for each channel (cycles) were as follows: running buffer, 2E11 vp/ml, 5E11 vp/ml, 2E12 vp/ml.

All sensorgrams were generated as reference subtracted and the responses as the difference between baseline before injection and signal just before end of injection.

### Biacore analysis of alkaline stability

Assessment of alkaline stability was done on the same Biacore^{™} chips as used for binding analysis as described above. After evaluation of AAV9 binding (see above), the samples were subjected to 88 repeating cycles of exposure to NaOH (0.3 M). Every second cycle included with binding to AAV9 (5E11 vp/ml) otherwise running buffer PBS-P+ was used as sample injection to reduce analyte consumption.

Biacore method for alkaline stability (per cycle): Running buffer: PBS-P+; Flow rate: 10 µl/min; Sample injection 1 (AAV9 5E11 vp/ml or running buffer PBS-P+ every second cycle to reduce analyte consumption): 300 s over both flow cells; Dissociation time 1: 30 s; Sample injection 2 (0.3 M NaOH): 600 s over both flow cells; Dissociation time 2: 0 s; Regeneration: 10 mM Glycine-HCl pH 1.5, 30 µl/min, 2x30 s. This cycle was repeated 88 times to follow stability of AAV9 response values.

All sensorgrams were generated as reference subtracted and the responses as the difference between baseline before injection and signal just before end of injection.

### Results

Table 17 shows the immobilization levels obtained for the tested polypeptides, as well as the AAV9 binding capacity using the maximum AAV9 concentration, and the NaOH stability. The candidate polypeptides were expressed as fusion with SEQ ID NO:160, and hence had a molecular weight that was higher than for an sdAb as such. The relative immobilization level is the immobilization level divided by the molecular weight.

**Table 17**

| | MW (Da) | Immobilization level (RU) | Relative immobilization level (RU/Da) | AAV9 capacity, 2E12 vp/ml (RU) | NaOH stability (no. of exposure cycles with at least 50% binding retained) |
|---|---|---|---|---|---|
| vh118 | 21385 | 3479 | 0.16 | 12487 | 34 |
| vh97 | 21215 | 6056 | 0.29 | 13109 | 26 |
| vh376 | 21284 | 2032 | 0.10 | 10236 | 28 |
| vh324 | 21386 | 2127 | 0.10 | 10722 | 36 |
| vh377 | 21373 | 4598 | 0.22 | 12694 | >88 |
| vh378 | 21478 | 3568 | 0.17 | 12669 | 66 |
| vh379 | 21161 | 5237 | 0.25 | 13808 | 72 |
| CaptureSelect^{™} Biotin Anti-AAV9 Conjugate* | 13000 | 4128 | 0.32 | 8855 | 4 |

| | | | | | |
|---|---|---|---|---|---|
| *prior art | | | | | |

It was seen that despite being immobilized at lower molar levels than the prior art reference, CaptureSelect^{™} Biotin Anti-AAV9 Conjugate, the tested AAV9 binding polypeptides of the present invention had a markedly higher AAV9 binding capacity. Furthermore, the alkaline stability of the inventive candidates was exceedingly high. In fact, for one candidate, vh377, the AAV9 response never dropped below 50 % during the 88 cycles.

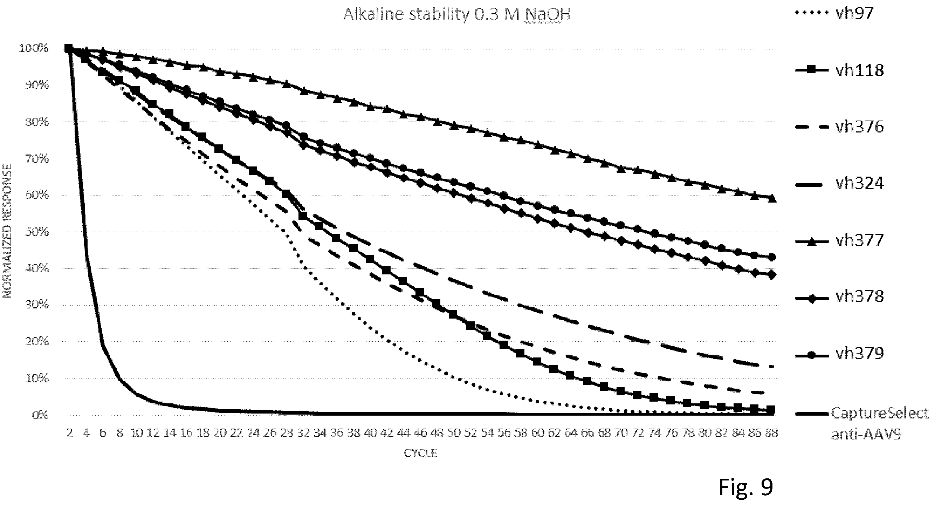

The result of the alkaline stability assessment is also shown in Fig. 9. During the alkaline stability analysis, the Biacore run stopped between cycle 28 and 30, hence the data point at cycle 30 was not measured. The run was continued, and next binding data was measured at cycle 32.

### Example 7: Functional evaluation - affinity chromatography

An AAV9 binding sdAb variant (SEQ ID NO:80) produced in the form of a fusion protein (SEQ ID NO:161) was immobilized on a support to form an affinity separation matrix and tested in a chromatography experiment.

### Materials and methods

5 µl glycerol stock produced as described in Example 1B was inoculated in 4 ml LB culture medium supplemented with 100 µg/ml carbenicillin in 14 ml round-bottom tube and incubated at 37°C overnight at 160 rpm.

Protein expression culture medium (TB culture medium supplemented with 100 µg/ml carbenicillin and 2 mM MgCl₂) was prepared and added to filled 500 ml baffled glass shake flasks (50 ml per flask). Each flask was inoculated with approximately 500 µl from the previous overnight culture to obtain a starting OD600 of 0.05. The flasks were incubated in an Infors HT shaking incubator for approximately 3.5 hours at 37°C and 140 rpm shaking until OD600 reached 1.0. Thereafter 50 µl of IPTG (1 M) was added to a final concentration of 1 mM and the flasks were incubated in Infors HT shaking incubator at 27°C and 140 rpm shaking for 18 hours, whereafter the cultures were transferred to Falcon tubes (50 ml). To generate crude variant lysates the Falcon tubes were incubated in 48°C water bath for 2 hours followed by pelleting of cell debris by centrifugation at 8000 g for 10 min and filtration of supernatant with a 0.22 µm filter.

Clarified lysate for each sample was loaded onto a 1 ml HiTrap MabSelect^{™} PrismA chromatography column (Cytiva^{™}), equilibrated in phosphate buffered saline (PBS) pH 7.4, allowing a residence time of about 2.4 min. The column was then washed with 5 column volumes (CV) of PBS pH 7.4, followed by 5 CV of 50 mM sodium acetate pH 6. Protein was eluted using 50 mM sodium acetate pH 3.5 in a step gradient over 5 CV.

Concentration of the purified samples was measured using NanoDrop (ThermoFisher Scientific) according to manufacturer's instructions. Following purification, samples were kept in refrigerator in Eppendorf tubes until all measurements were performed and were then kept in freezer until Biacore analysis. Protein mass was determined by liquid chromatography-mass spectrometry on a ACQUITY Rda Detector (Waters^{™}) using a BioRessolve column (Waters^{™}).

The fusion protein was immobilized on epoxy activated chromatography resin (highly crosslinked agarose beads) using cysteine coupling. 0.2 ml of the resin was packed in Tricorn 5/20 columns (Cytiva). Using an ÄKTA pure 25 system (Cytiva), 50 ml of tangential flow filtrated AAV9 material (prepared in-house) was loaded on the column with four minutes residence time and eluted with 100 mM citrate buffer pH 2.5.

Collected eluate fraction was analysed on a Coomassie SDS-PAGE gel. The low pH elution sample was neutralized with 1:1 volume of Tris buffer (400 mM). 15 µl of sample and 5 µl sample buffer + DTT was heated to 70 °C for 10 min. 5 µl sample was loaded and the gel was run for 35 minutes at 200V and then soaked in Coomassie solution overnight. The gel was then de-stained with water until satisfactory staining was achieved. The gel was photographed using ImageQuant 800.

To assess the alkaline stability of the immobilized fusion protein, the chromatography resin was subjected to cleaning-in-place (CIP) with 10 mM Glycine-HCl pH 1.5, followed by an alkaline stability study involving exposure to 0.5 M NaOH. The full stability study consisted of 40 cycles where each cycle involved contacting the chromatography material with 0.5M NaOH for 30 min. A solution containing pure AAV9 particles was added every 10^{th} cycle with 15 seconds residence time. PBS buffer was used for all other cycles.

### Results

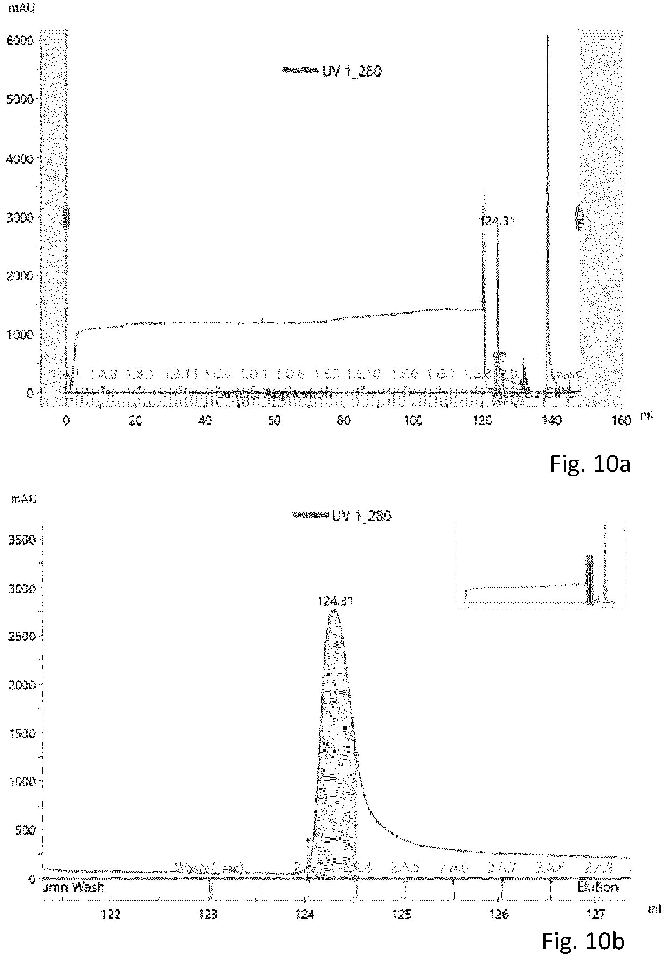

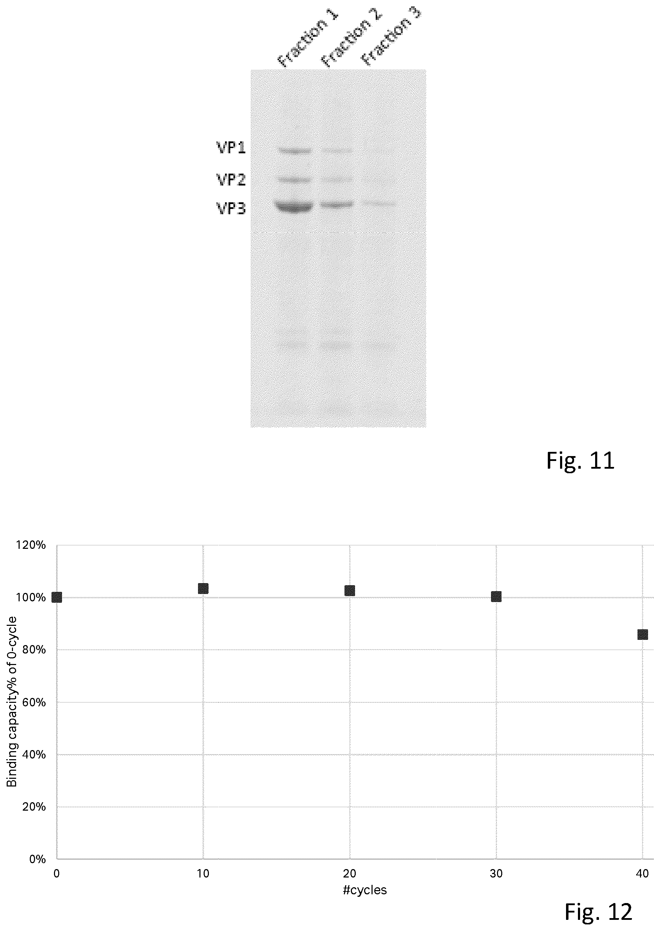

Fig. 10a shows the chromatogram obtained for purification of AAV9 TFF material using the candidate vh118 as an affinity ligand. The elution peak is marked with a rectangle. Fig. 10b shows only the region of the elution peak. Fig. 11 shows the photograph of the gel. VP1, VP2 and VP3 denotes the AAV9 virion proteins 1, 2 and 3, respectively.

The results show that the present candidate was able to successfully purify AAV9 viral particles, with an estimated dynamic binding capacity (QB10%) of about 3E+14 viral particles/ml resin and estimated recovery of loaded virus particles of 70-80%.

The result of the alkaline stability assessment is shown in Fig. 12 which plots the binding capacity normalized to the binding capacity before the first NaOH exposure. Over the 40 cycles of exposure to 0.5 M NaOH, which corresponded to an accumulated exposure time of 20 hours, the target binding capacity was reduced by less than 20 %.

### Example 8: Alkaline stability of native vs differently modified sdAb frameworks

### A: sdAbs directed to AAV

This example investigated the alkaline stability of an AAV9-binding VH having multiple variations in the framework regions, where the mutations were selected from less preferred modifications, to show that also a combination of multiple less preferred modifications give rise to increased alkaline stability as compared to non-modified (native) frameworks. In the example, the alkaline stability of a native framework, one with the preferred modifications and one with suggested but not preferred modifications are compared.

For the modified frameworks amino acid substitutions in positions 19, 23, 24, 40, 43, 44, 45, 76, 79 and 89 (Kabat numbering) were made, one selected from a combination of preferred substitutions, and one a combination of suggested but not preferred substitutions. Thus, three candidate VH sequences were tested, one having a non-modified framework (vh342), one having a framework with preferred amino acid substitutions compared to the non-modified framework (vh118), and one having a combination of possible, but less preferred mutations (vh540). Amino acid substitutions (Kabat numbering) for each candidate VHH and their corresponding full sdAb sequences are set out in Table 18. CDR sequences were the same for all candidates (as in vh118).

The VH sequences were expressed with SEQ ID NO:160 as a C-terminal fusion via a peptide linker. Total molecular weight was 21 kDa.

**Table 18**

| **Candidate** | **Amino acids in Kabat positions 19, 23, 24, 40, 43, 44, 45, 76, 79, 89:** | **Alkaline stability (0.3 M NaOH)** |
|---|---|---|
| vh342 (SEQ ID NO: 73) | 19R, 23A, 24A, 40A, 43K, 44Q, 45R, 76T, 79Y, 89V | No stability |
| vh118 (SEQ ID NO: 80) | 19R, 23T, 24I, 40R, 43K, 44E, 45R, 76N, 79Y, 89V | Good |
| vh540 (SEQ ID NO: 216) | 19S, 23S, 24I, 40I, 43G, 44D, 45I, 76Q, 79F, 89I | Good |

The following aspects were evaluated: (1) Affinity assessment of AAV9 interaction, tested through high concentration injection of AAV9. (2) Alkaline stability, tested by reduction of AAV9 binding after increasing number of treatments with 0.3 M NaOH.

### B: sdAbs directed to GFP

As in example A above, this example investigated the alkaline stability of the frameworks using a different target than AAV9, where GFP-binding VHs were generated, one having multiple variations in the framework regions, where the mutations were selected from less preferred mutations, one having the native framework and one having a preferred modified framework.

Three candidate VH sequences were tested, one having a non-modified framework (vh 60), one having a framework with preferred amino acid substitutions compared to the non-modified framework (vh113), and one having a combination of less preferred mutations (vh540). Amino acid substitutions (Kabat numbering) for each candidate VHH and their corresponding full sdAb sequences are set out in Table 19. CDR sequences were the same for all candidates.

The VH sequences were expressed with SEQ ID NO:160 as a C-terminal fusion via a peptide linker. Total molecular weight was 21 kDa.

**Table 19**

| **Candidate** | **Amino acids in Kabat positions 19, 23, 24, 40, 43, 44, 45, 76, 79, 89:** | **Alkaline stability (0.1 M NaOH)** |
|---|---|---|
| vh60 (SEQ ID NO: 155) | 19R, 23A, 24A, 40A, 43K, 44Q, 45R, 76T, 79Y, 89V | Low |
| vh113 (SEQ ID NO: 156) | 19R, 23T, 24I, 40R, 43K, 44E, 45R, 76N, 79Y, 89V | Good |
| vh539 (SEQ ID NO: 217) | 19S, 23S, 24I, 40I, 43G, 44D, 45I, 76Q, 79F, 89I | Good |

The following aspects were evaluated: (1) Affinity assessment of AAV9 interaction, tested through high concentration injection of AAV9. (2) Alkaline stability, tested by reduction of AAV9 binding after increasing number of treatments with 0.1 M NaOH.

### C: sdAbs directed to EGFR

As in examples B above, this example investigated the alkaline stability of the frameworks using a different target than AAV9, where EGFR-binding VHs were generated, one having multiple variations in the framework regions, where the mutations were selected from less preferred mutations, one having the native framework and one having a preferred modified framework.

Three candidate VH sequences were tested, one having a non-modified framework (vh166), one having a framework with preferred amino acid substitutions compared to the non-modified framework (vh167), and one having a combination of less preferred mutations (vh541). Amino acid substitutions (Kabat numbering) for each candidate VHH and their corresponding full sdAb sequences are set out in Table 20. CDR sequences were the same for all candidates.

The VH sequences were expressed with SEQ ID NO:160 as a C-terminal fusion via a peptide linker. Total molecular weight was 21 kDa.

**Table 20**

| **Candidate** | **Amino acids in Kabat positions 19, 23, 24, 40, 43, 44, 45, 76, 79, 89:** | **Alkaline stability (0.1 M NaOH)** |
|---|---|---|
| vh166 (SEQ ID NO: 157) | 19R, 23A, 24A, 40A, 43K, 44Q, 45R, 76T, 79Y, 89V | No stability |
| vh167 (SEQ ID NO: 156) | 19R, 23T, 24I, 40R, 43K, 44E, 45R, 76N, 79Y, 89V | Moderate |
| vh541 (SEQ ID NO: 218) | 19S, 23S, 24I, 40I, 43G, 44D, 45I, 76Q, 79F, 89I | Moderate |

The following aspects were evaluated: (1) Affinity assessment of AAV9 interaction, tested through high concentration injection of AAV9. (2) Alkaline stability, tested by reduction of AAV9 binding after increasing number of treatments with 0.1 M NaOH.

### Materials and methods

The candidate polypeptides were generated and purified either on IMAC, as in example 5 above, or using PrismA, as in example 7 above. Biacore analysis of binding affinity for AAV9 and analysis of alkaline stability were also performed as in the examples above.

### Results

For the AAV-binding VHH candidates, it was seen that both vh540 and vh118 showed good binding of AAV, where vh342 showed a slightly lower binding (data not shown). When assessing the alkaline stability, stability until 10% of cycle 1, vh342 having the native framework showed zero stability at 0.3M NaOH, i.e. it was not alkali stable. The candidate including several point mutations being less preferred showed 12 cycles of alkaline stability at 0.3M NaOH. Thus, even though not as stable as vh118, vh540 showed good alkaline stability, and especially compared to the native framework used in vh342, which showed no stability at all. This shows that also a combination of less preferred mutations will render a stable framework, as compared to native frameworks.

For the GFP binding VHH candidates, all three showed a slightly lower binding towards the target. The vh60 having the native framework showed 2 cycles stability at 0.1M NaOH, while vh539, having the framework with the non-preferred substitutions, showed an alkaline stability of 29 cycles at 0.1M NaOH (as compared to over 30 cycles for vh113), which is a huge increase. Thus, it may be concluded that the frameworks also greatly stabilize the GFP binding CDRs.

For EGFR, the modified frameworks showed good target binding, while the native framework showed a slightly lower binding. The vh166 having the native framework showed no stability at 0.1M NaOH, while vh541, having the framework with the non-preferred substitutions, showed an alkaline stability of 3 cycles at 0.1M NaOH, as compared to 12 cycles of vh167 having the preferred mutations. Even though this is a lower stability as compared to the other targets, it is still a huge increase compared to the native frameworks. However, this also shows that if the CDRs will affect the stability, and that more stable CDRs will also give rise to a more stable sdAb when the framework is modified.

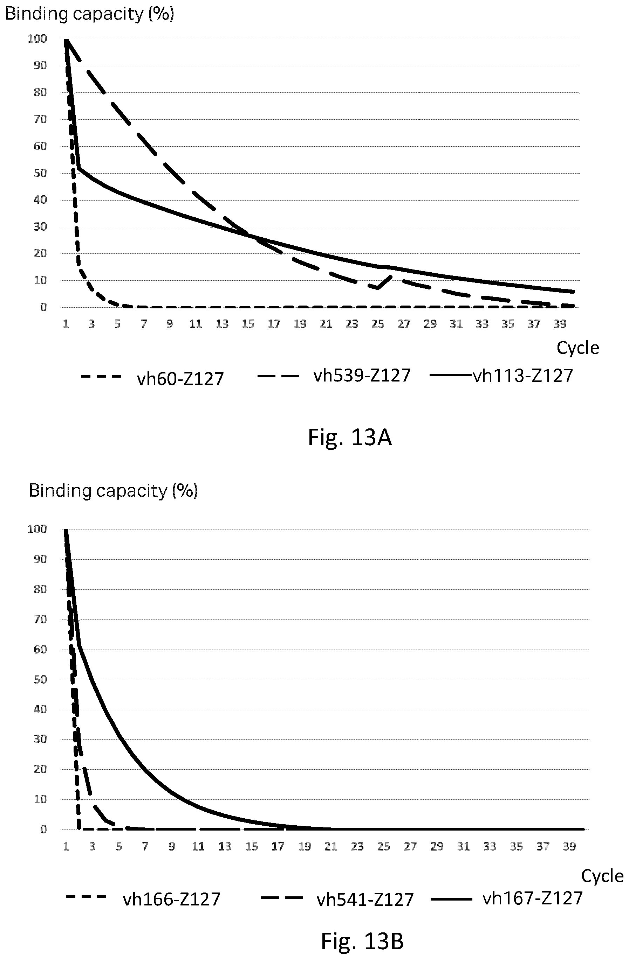

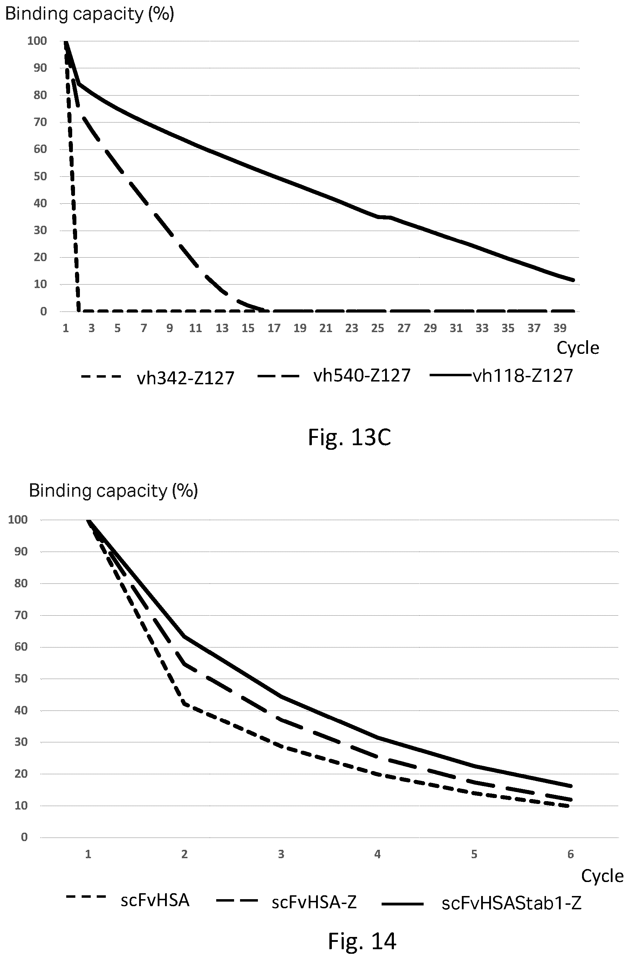

The results are visualized in Fig. 13, where Fig. 13A shows the results for GFP, Fig. 13B for EGFR and Fig. 13C for AAV9. Looking at a remaining binding capacity of under 8%, for GFP as target it may be seen that it is reached already after one cycle for vh60, after 25 cycles for vh539 and after 35 cycles for vh113, for EGFR as target, the same is seen for vh166 after merely one cycle, after 3 cycles for vh541 and after 12 cycles for vh16, and lastly, for AAV9 as target, after 1 cycle for vh342, after 13 cycles for vh540 and after more than 40 cycles for vh118.

### Example 9: AAV9 binding polypeptides in different frameworks and two sets of CDRs

This example further investigated five additional distinct sdAb frameworks with the same sdAb CDR sequences. One framework was also tested with two different sets of CDR sequences. All sdAbs have the same CDR1 and CDR2 sequences, while vh516 had a distinct CDR3 from vh511-vh515. Vh511-515 have the same CDRs as vh324 but have different frameworks. Vh324 has framework variant 1, while vh511-515 has five new framework permutations, as shown in the tables below.

The candidate polypeptides tested were expressed as fusion protein in which the respective sdAb had SEQ ID NO: 160 fused to its C-terminal via a linker, and had a C-terminal (His)₆-tag. The sdAb sequences variants are presented in Tables 21 and 22.

The following aspects were evaluated: (1) Affinity assessment of AAV9 interaction (tested through high concentration injection of AAV9). (2) Alkaline stability (tested by reduction of AAV9 binding after increasing number of treatments with 0.3 M NaOH).

**Table 21**

| sdAb | FW variant | CDR1 | CDR2 | CDR3 | sdAb SEQ ID NO: |
|---|---|---|---|---|---|
| vh511 | 4 | RTLSDFTMG (SEQ ID NO: 179) | AISWSGAYTK (SEQ ID NO: 183) | GPTGLLSKKTTPADYDY (SEQ ID NO: 187) | 200 |
| vh512 | 5 | | | | 201 |
| vh513 | 6 | | | | 202 |
| vh514 | 7 | | | | 203 |
| vh515 | 8 | | | | 204 |
| vh516 | 8 | RTLSDFTMG (SEQ ID NO: 179) | AISWSGAYTK (SEQ ID NO: 183) | GPTGLLSQKTTPADYDY (SEQ ID NO: 196) | 205 |

**Table 22**

| FW variant | FWR1 | FWR2 | FWR3 | FWR4 |
|---|---|---|---|---|
| 1 | QVQLQQSGGGSVQ AGGSLRLSCTISG (SEQ ID NO:8) | WFRQRPGKEREFVA (SEQ ID NO:26) | YADSVKGRFTISRDNAKNTV WLQMNSLKPEDTAVYYCAA (SEQ ID NO: 45) | WGQGTQVTVSS (SEQ ID NO: 71) |
| 4 | QVQLQQSGGGSVQ AGGSLSLSCTSSG (SEQ ID NO: 206) | WFRQRPGKELEFVA (SEQ ID NO: 210) | YADSVKGRFTISRDNAKNTV WLQMNSLKPEDTAVYYCAA (SEQ ID NO:213) | WGQGTQVTVSS (SEQ ID NO: 71) |
| 5 | QVQLQQSGGGSVQ AGGSLRLSCTSSG (SEQ ID NO:207) | WFRQIPGKEREFVA (SEQ ID NO:211) | YADSVKGRFTISRDNAKQTV WLQMNSLKPEDTAVYYCAA (SEQ ID NO:214) | WGQGTQVTVSS (SEQ ID NO: 71) |
| 6 | QVQLQQSGGGSVQ AGGSLRLSCTSSG (SEQ ID NO:207) | WFRQRPGKDLEFVA (SEQ ID NO:212) | YADSVKGRFTISRDNAKNTV WLQMNSLKPEDTAIYYCAA (SEQ ID NO: 215) | WGQGTQVTVSS (SEQ ID NO: 71) |
| 7 | QVQLQQSGGGSVQ AGGSLRLSCTVSG (SEQ ID NO: 208) | WFRQRPGKELEFVA (SEQ ID NO:210) | YADSVKGRFTISRDNAKNTV WLQMNSLKPEDTAVYYCAA (SEQ ID NO:213) | WGQGTQVTVSS (SEQ ID NO: 71) |
| 8 | QVQLQQSGGGSVQ AGGSLTLSCTVSG (SEQ ID NO:209) | WFRQRPGKELEFVA (SEQ ID NO:210) | YADSVKGRFTISRDNAKNTV WLQMNSLKPEDTAVYYCAA (SEQ ID NO:213) | WGQGTQVTVSS (SEQ ID NO: 71) |
| 8 | QVQLQQSGGGSVQ AGGSLTLSCTVSG (SEQ ID NO:209) | WFRQRPGKELEFVA (SEQ ID NO:210) | YADSVKGRFTISRDNAKNTV WLQMNSLKPEDTAVYYCAA (SEQ ID NO:213) | WGQGTQVTVSS (SEQ ID NO: 71) |

### Materials and methods

### Generation of Immobilized Metal Affinity Chromatography (IMAC) purified candidate polypeptides

For each variant, 5 µl glycerol stock produced as described in Example 1 was inoculated in 4 ml LB culture medium supplemented with 100 µg/ml carbenicillin in 14 ml round-bottom tubes and grown over night at 37°C, 200 rpm agitation. Protein expression culture medium (TB medium supplemented with 100 µg/ml carbenicillin and 2 mM MgCl₂) was prepared and added to filled 500 ml baffled glass shake flasks (50 ml per flask, 7 flasks in total). Each flask was inoculated with approximately 500 µl from the previous overnight culture to obtain a starting OD600 of 0.05. The flasks were incubated in Infors HT shaking incubator for approximately 3.5 hours at 37°C and 140 rpm shaking until OD600 reached 1.0. Thereafter 50 µl IPTG (1 M) was added to a final concentration of 1 mM and the flasks were incubated in Infors HT shaking incubator at 27°C and 140 rpm shaking for 18 hours, whereafter the cultures were transferred to Falcon tubes (50 mL). To generate crude variant lysates the Falcon tubes were incubated in 48°C water bath for 2 hours followed by pelleting of cell debris by centrifugation at 8000 g for 10 min and filtration of supernatant with a 0.45 µm filter.

Clarified lysate was loaded on HisTrap^{™} FF 1 mL chromatography columns (Cytiva^{™}), equilibrated in 50 mM sodium phosphate pH 7.5, 500 mM NaCl, allowing a residence time of about 2 min. The column was then washed with 15 column volumes (CV) of 50 mM sodium phosphate pH 7.5, 500 mM NaCl. Protein was eluted using 0.5 M imidazole pH 7.5-8, linear gradient (0-100 %) over 10 CV. Eluted protein was buffer-exchanged into phosphate buffered saline (Medicago) pH 7.4 using gravity flow columns prepacked with Sephadex^{™} G-25 resin (Cytiva) prior to further analysis.

Concentration of HisTrap^{™} FF purified samples was measured using NanoDrop (Thermo Scientific) according to manufacturer's instructions. Following purification, samples were kept in refrigerator in Eppendorf tubes until all measurements were performed and were then kept in freezer until Biacore analysis. Protein mass was determined by liquid chromatography-mass spectrometry on a ACQUITY Rda Detector (Waters^{™}) using a BioRessolve column (Waters^{™}).

### Biotinylation of purified candidate polypeptides

Purified candidate polypeptides were biotinylated at the C-terminal cysteine using EZ-Link^{™} Maleimide-PEG2-Biotin, No-Weigh^{™} Format (ThermoFisher Scientific) with a 2X molar excess of biotin.

### SEC purification of biotinylated candidate polypeptides

Proteins were additionally purified by size exclusion chromatography (SEC) for separation from excess biotin, non-biotinylated protein dimers and other impurities. SEC was performed in phosphate buffered saline (Medicago) pH 7.4 at a flow rate of 0.75 mL/min using Superdex^{™} 75 Increase 10/300 GL chromatography column (Cytiva). Proteins of interest eluted as sharp symmetric peak. Purest fractions (as analyzed by SDS-PAGE) were pooled and used for further analyses.

### Biacore analysis of binding affinity for AAV9

To assess binding to AAV9, the AAV9 binding polypeptide candidates were immobilized on a Biacore^{™} Series S SA chip. AAV9 (2E12 viral particles (vp)/ml) was used as analyte.

Materials and equipment used were as follows: Biacore^{™} Series S SA sensor chips, Biacore^{™} 8K+ instrument (all from Cytiva, Sweden); AAV9 (prepared in-house); candidate polypeptides generated as described above; CaptureSelect^{™} Biotin Anti-AAV9 Conjugate (Thermo Scientific^{™}).

Immobilization was performed using a standard method in Biacore^{™} software with coupling of biotinylated AAV9 binding polypeptide variants in Flow Cell 2 (FC2) and activation/inactivation in Flow cell 1 (FC1). Biotinylated AAV9 binding polypeptide variants were diluted in PBS at a concentration of 100 µg/ml.

Biacore^{™} method for binding analysis: Running buffer: PBS-P+; Flow rate: 5 µl/min; Sample injection: 2400 s/40 min over both Flow Cells (FC1 and FC2); Dissociation time: 2400 s/40 min; Regeneration: 10 mM Glycine-HCl pH 1.5, 30 µl/min, 2x30 s. Injections of analyte (AAV9) for each channel (cycles) were as follows: running buffer, 2E11 vp/ml, 5E11 vp/ml, 2E12 vp/ml.

All sensorgrams were generated as reference subtracted and the responses as the difference between baseline before injection and signal just before end of injection.

### Biacore analysis of alkaline stability

Assessment of alkaline stability was done on the same Biacore^{™} chips as used for binding analysis as described above. After evaluation of AAV9 binding (see above), the samples were subjected to 88 repeating cycles of exposure to NaOH (0.3 M). Every second cycle included with binding to AAV9 (5E11 vp/ml) otherwise running buffer PBS-P+ was used as sample injection to reduce analyte consumption.

Biacore method for alkaline stability (per cycle): Running buffer: PBS-P+; Flow rate: 10 µl/min; Sample injection 1 (AAV9 5E11 vp/ml or running buffer PBS-P+ every second cycle to reduce analyte consumption): 300 s over both flow cells; Dissociation time 1: 30 s; Sample injection 2 (0.3 M NaOH): 600 s over both flow cells; Dissociation time 2: 0 s; Regeneration: 10 mM Glycine-HCI pH 1.5, 30 µl/min, 2x30 s. This cycle was repeated 88 times to follow stability of AAV9 response values.

All sensorgrams were generated as reference subtracted and the responses as the difference between baseline before injection and signal just before end of injection.

### Results

The response levels of AAV9 interactions of the candidate polypeptides using the maximum AAV9 concentration (2E12 vp/ml) was normalized to the response of vh377 and summarized in Table 23, recording alkaline stability as the number of cycles with at least 50 % binding capacity retained. The binding response values were normalized against the binding capacity recorded for the second cycle (i.e., excluding the first cycle). In each cycle, 0.3 M NaOH was injected with a contact time of 10 min at 10 µl/min.

It was seen that candidates vh511-515, which had the same set of CDRs but differed in the framework regions, had even higher alkaline stability than vh377. Comparing vh516 with vh515, which had identical framework regions, it was concluded that the different CDRs had an equal positive contribution to alkaline stability.

**Table 23**

| | Normalized AAV9 binding capacity (%) | NaOH stability (no. of exposure cycles with at least 50% binding maintained) |
|---|---|---|
| vh377 | 100.0 | 76 |
| vh511 | 119 | 46 |
| vh512 | 111 | 56 |
| vh513 | 118 | 60 |
| vh514 | 113 | 69 |
| vh515 | 122 | 46 |
| vh516 | 119 | 46 |

### Example 10: Substituting first position of the framework from Q to E

In this experiment it was investigated whether using an E instead of a Q in the first position of the framework (FW1) would affect the stability of the polypeptide.

### Materials and methods

Four polypeptides where tested, vh118 (SEQ ID NO:80) and vh498 (SEQ ID NO: 195), where vh498 differs from vh118 in that the fist position is substituted from a Q to an E. Similarly, vh324 (SEQ ID NO: 187) and vh459 (SEQ ID NO: 219) were tested, where vh458 has the first position substituted from Q to E. The constructs were produced and tested, purification on PrismA and coupling to CM5 chip.

### Generation of MabSelect PrismA^{™} purified candidate polypeptides

For each candidate, 5 µl of the glycerol stock produced as described in Example 1 above was inoculated in 4 ml LB culture medium supplemented with 100 µg/ml carbenicillin in 14 ml round-bottom tube and grown over night at 37°C, 200 rpm agitation. Protein expression culture medium (TB medium supplemented with 100 µg/ml carbenicillin and 2 mM MgCl₂) was prepared and added to filled 500 ml baffled glass shake flasks (50 ml per flask, 43 flasks in total). Each flask was inoculated with approximately 500 µl from the previous overnight culture to obtain a starting OD600 of 0.05. The flasks were incubated in Infors HT shaking incubator for approximately 3.5 hours at 37°C and 140 rpm shaking until OD600 reached 1.0. Thereafter 50 µl IPTG (1 M) was added to a final concentration of 1 mM and the flasks were incubated in Infors HT shaking incubator at 27°C and 140 rpm shaking for 18 hours, whereafter the cultures were transferred to Falcon tubes (50 ml). To generate crude variant lysates the Falcon tubes were incubated in 48°C water bath for 2 hours followed by pelleting of cell debris by centrifugation at 8000 g for 10 min and filtration of supernatant with a 0.45 µm filter.

The samples were purified using HiTrap MabSelect PrismA^{™} chromatography columns (Cytiva). Clarified lysate for each sample was loaded onto a 1 mL column, equilibrated in phosphate buffered saline (PBS) pH 7.4, allowing a residence time of about 2.4 min. The column was then washed with 5 column volumes (CV) of PBS pH 7.4, followed by 5 CV of 50 mM sodium acetate pH 6. Protein was eluted using 50 mM sodium acetate pH 3.5 in a step gradient over 5 CV.

Concentration of the purified samples was measured using NanoDrop (Thermo Scientific) according to manufacturer's instructions. Following purification, samples were kept in refrigerator in 80ntarctic tubes until all measurements had been performed and were then kept in freezer until Biacore analysis. Protein mass was determined by liquid chromatography-mass spectrometry on a ACQUITY Rda Detector (Waters^{™}) using a BioRessolve column (Waters^{™}).

### Biacore analysis of binding affinity for AAV9

To assess binding to AAV9, the candidate polypeptides were immobilized on a Biacore^{™} Series S CM5 chip. AAV9 (2E12 viral particles (vp)/ml) was used as analyte.

Materials and equipment used were as follows: Biacore^{™} Series S CM5 sensor chips, Biacore^{™} Amine coupling kit, Biacore^{™} Acetate buffer pH 5.0, Biacore^{™} 8K+ instrument (all from Cytiva, Sweden); AAV9 (prepared in-house); candidate polypeptides generated as described above.

Immobilization was performed using a standard method in Biacore^{™} software with coupling of AAV9 binding polypeptide variants in Flow Cell 2 (FC2) and activation/inactivation in Flow cell 1 (FC1). AAV9 binding polypeptide variants were diluted in Biacore^{™} Acetate buffer pH 5.0 at a concentration of 25 µg/ml. The immobilization levels obtained were 3484 +/- 197 RU.

In each run the polypeptide was immobilized in FC2. Multiple sensor chips were used until all candidates were tested.

Running buffer: PBS-P+; Flow rate: 5 µl/min; Sample injection: 2400 s/40 min over both Flow Cells (FC1 and FC2); Dissociation time: 2400 s/40 min; Regeneration: 10 mM Glycine-HCl pH 1.5, 30 µl/min, 2x30 s. Injections of analyte (AAV9) for each channel (cycles) were as follows: running buffer, 2E11 vp/ml, 5E11 vp/ml, 2E12 vp/ml.

All sensorgrams were generated as reference subtracted and the responses as the difference between baseline before injection and signal just before end of injection.

### Biacore analysis of alkaline stability

Assessment of alkaline stability was done by immobilizing the candidate polypeptides on the Biacore^{™} chips. After evaluation of AAV9 binding (see above) the same chips were subjected to repeated cycles of binding to AAV9 (5E11 vp/ml) followed by injection of NaOH (0.5 M).

Biacore method for alkaline stability (per cycle):
Running buffer: PBS-P+; Flow rate: 10 µl/min; Sample injection 1 (AAV9 5E11 vp/ml): 300 s over both Flow Cells; Dissociation time 1: 30 s; Sample injection 2 (0.5 M NaOH): 600 s over both flow cells; Dissociation time 2: 0 s; Regeneration: 10 mM Glycine-HCl pH 1.5, 30 µl/min, 2x30 s. This cycle was repeated 40 times to follow stability of AAV9 response values.

All sensorgrams were generated as reference subtracted and the responses as the difference between baseline before injection and signal just before end of injection.

### Results

Even though the stability of the polypeptide was a little bit lower for the polypeptides having E as the first amino acid in the framework, it was demonstrated that also using E in the first position of FW1 give rise to highly stable polypeptides, as summarized in table 24 below. Vh118 substituted with E in position 1 is denoted vh118 (Q1E). vh324 is denoted correspondingly.

**Table 24**

| | SEQ ID NO: | NaOH stability (no. of exposure cycles with at least80% binding maintained) |
|---|---|---|
| vh118 | 80 | 8 |
| vh118 (Q1E) | 195 | 7 |
| Vh324 | 187 | 29 |
| Vh324 (Q1E) | 219 | 15 |

### Example 11: Measuring alkaline stability for AAV9, EGFR and GFP, vs ZVH3

The alkaline stability was measured by assessing polypeptides with target binding to AAV9 (5e11 vp/ml) or binding to ZVH3 hexamer (MabSelect^{™} VH3 ligand) (50nM) that interacts with the ligand's backbone. Alternating AAV9 or ZVH3 binding, the polypeptides were exposed to NaOH (0.5M) for 10 minutes for a total of 40 cycles. This was to show that both the target binding CDRs, and the framework, add to the alkaline stability of the polypeptide. The data attained from the present example was compared to the results for AAV9-binding sdAbs of the previous examples.

### Materials and methods

The ZVH3 hexamer was provided as described in WO2023174900A1, the hexamer having the sequence according to SEQ ID NO: 177 of WO2023174900A1, which is a hexamer of the monomer as defined by SEQ ID NO: 15 of WO2023174900A1, which is incorporated by reference herein.

### Biacore analysis of alkali stability

Materials and equipment used were as follows: Biacore^{™} Series S CM5 sensor chips, Biacore^{™} Amine coupling kit, Biacore^{™} Acetate buffer pH 5.0, Biacore^{™} 8K+ instrument (all from Cytiva, Sweden); 0.5 M NaOH; ZVH3-ligand; candidate polypeptides generated as described above. Assessment of alkali stability was done on the Biacore^{™} chips prepared above. The polypeptides were subjected to 40 repeating cycles of binding to AAV9 (5e11vp/ml) or ZVH3 hexamer (50nM), followed by 10-minute exposure to NaOH (0.5 M). The NaOH stability of the polypeptides was assessed by comparing the cycle number when the polypeptides had lost 50% of their initial binding to AAV9 or ZVH3 hexamer.

All sensorgrams were generated as reference subtracted and the responses as the difference between baseline before injection and signal just before end of injection.

### Results

The results of the alkaline stability assessment for the polypeptides are summarized in Table 25 below. The polypeptide CDR AAV9 binding is compared to the AAV9 ZVH3 backbone binding for the AAV9 binding polypeptides vh260-268. It may be seen that the alkaline stability is increased also for this target. The unstable polypeptides vh267 and vh268 are unstable for both bindings.

**Table 25**

| | SEQ ID NO | NaOH stability cycle with 50% binding AAV9 | NaOH stability cycle with 50% binding ZVH3 |
|---|---|---|---|
| vh260 | SEQ ID NO: 106 | 22 | 23 |
| vh261 | SEQ ID NO: 107 | 22 | 21 |
| vh262 | SEQ ID NO: 108 | 21 | 21 |
| vh263 | SEQ ID NO: 109 | 22 | 19 |
| vh264 | SEQ ID NO: 110 | 22 | 22 |
| vh265 | SEQ ID NO: 111 | 21 | 22 |
| vh266 | SEQ ID NO: 112 | 25 | 24 |
| vh267 | SEQ ID NO: 116 | 1 | 2 |
| vh268 | SEQ ID NO: 117 | 1 | 2 |

The alkaline stability was further evaluated for GFP, EGFR and AAV9 binding molecules, where target binding an ZVH3 binding was compared. The same constructs as in Example 8 above were used. For GFP and EGFR, the NaOH concentration was 0.1M and for AAV9 0.5M. the results are presented in Table 26 below.

**Table 26**

| Candidate | SEQ ID NO: | Target | NaOH stability (no. of exposure cycles with at least 50% target binding retained) | NaOH stability (no. of exposure cycles with at least 50% ZVH3 binding retained) |
|---|---|---|---|---|
| vh60 | 155 | GFP | 2 | 1 |
| vh113 | 156 | GFP | 16 | 17 |
| vh539 | 217 | GFP | 1 | N.A |
| vh166 | 157 | EGFR | 1 | 1 |
| vh167 | 158 | EGFR | 6 | 9 |
| vh541 | 218 | EGFR | 1 | N.A |
| vh342 | 152 | AAV9 | 2 | 3 |
| vh118 | 80 | AAV9 | 22 | 26 |
| vh540 | 216 | AAV9 | 6 | N.A |

### Example 12: Alkaline stabilization of scFv scaffolds

scFv, i.e. a fusion of two variable domains (VH and VL) with a flexible linker, is a suitable scaffold for interaction with targets, but lack some bioprocess related properties. Thus, the possibility to stabilize scFv scaffolds using the framework mutations of the present disclosure was assessed. A publicly accessible scFv fragment against human serum albumin (HSA) were used as a starting point.

### Materials and methods

Three constructs were designed for the initial evaluation of expression, coupling, binding and alkaline stability, i) a first construct using a native HSA scFv, ii) a second construct was engineered as a fusion protein of native HSA scFv (scFvHSA) and a polypeptide moiety according to SEQ ID NO: 160 (scFvHSA-Z), and iii) a third construct was engineered as a fusion protein of a stabilized HSA scFv (vh118 framework) and a polypeptide moiety according to SEQ ID NO: 160 (scFvHSAStab1-Z), i.e. the VH portion of the scFv scaffold was stabilized by introducing mutations into the preferred locations. The native HSA scFv was as defined in Adams R. Et al (MABS VOL. 8, NO. 7, 1336-1346 (2016)), and the modified constructs were designed by fusing with a polypeptide moiety according to SEQ ID NO: 160, and in one construct, the framework was modified as the framework of the vh118 variant described herein. The constructs were expressed in E. coli and purified on IMAC or PrismA, as in previous examples.

A first Biacore experiment was performed on an SA-chip with biotinylated affinity to compare ScFvHSA with and without fusion to the polypeptide moiety, where the binding was experiment using concentrations 0, 10, 50, 100, 500 and 1000 nM of HSA and the alkaline stability test used 1000 nM HSA and CIP condition 10 min and 0,1 M NaOH. Improved alkaline stability by fusion of the polypeptide moiety according to SEQ ID NO: 160 compared to native, and the stability could be further improved by framework mutation as shown in Fig. 14.

A similar experiment was performed on a CM5-chip with NHS coupling was used. Biacore evaluation of binding of purified affinity ligands (i) and (ii) on CM5-chip, using concentrations 0, 50, 100, 500 and 1000 nM of HAS were performed. Biacore evaluation of alkaline stability used 1000 nM HSA and CIP condition 10 min and 0,1 M NaOH. This example also showed improved alkaline stability by fusion of polypeptide moiety according to SEQ ID NO: 160 compared to native, and further improved by framework mutations (data not shown).

### Results

The expression and purification showed approximately a 2-fold increase in yield for the stabilized third construct scFvHSAStab1-Z compared to the other constructs. The experiments on the SA-chip showed improved stability for ScFvHSA-Z and even more for scFvHSAStab1-Z, showing that a stabilized VHH framework improved the stability even more. The alkaline stability results of the SA-chip experiment are shown in Fig. 14, which shows a normalized plot of the alkaline stability experiment. For the second Biacore experiment, a CM5-chip with NHS coupling was used and showed that constructs with the polypeptide moiety according to SEQ ID NO: 160 had good coupling efficiency compared with only native scFvHSA (data not shown).

### Example 13: Substituting position 71 in FW3

In this experiment it was investigated how substitutions in position 71 of the sdAb framework would affect the properties of the polypeptide, including the apparent binding affinity to target molecule, and the alkaline stability of the polypeptide. For each of the tested targets GFP, EGFR and AAV9 respectively, six different amino acids in position 71 were tested, where position 71 of the full sdAb sequence corresponds to position 13 in FW3. The experiment included the native amino acid R in position 71, as well as mutations where the R was substituted by one of F, H, K, Q or Y. The mutations were introduced in the previously generated stabilized sdAbs vh113 (GFP), vh167 (EGFR) and vh118 (AAV9). All the sdAb were tested as fusion proteins with Z127.

### Materials and methods

Six polypeptides (sdAbs) were tested for each target. Each sdAb was expressed as a fusion protein, fused C-terminally to an SpA domain Z variant (SEQ ID NO:160). All constructs had a C-terminal linker (GS) followed by a His₆ tag.

For GFP the fusion protein of vh113 with Z127 (SEQ ID NO: 167) was tested (position 71 being R), including 5 mutations in position 71 of vh113, for EGFR the fusion protein of vh167 with Z127 (SEQ ID NO: 169) was tested (position 71 being R), including mutations in position 71 of vh167, and for AAV9 the fusion protein of vh118 with Z127 (SEQ ID NO:162) was tested (position 71 being R), including mutations in position 71 of vh118, as shown in table 27 below. The constructs were produced and tested as described above, with transformation to BL21(DE3) E.coli cells and expression in 20ml shake flask, with purification on PrismA and finally the proteins were amine coupled onto CM5 chip for evaluation.

**Table 27**

| Candidate | SEQ ID NO: | Target | Amino acid in position 71 |
|---|---|---|---|
| vh113+Z127 | 167 | GFP | R |
| vh564+Z127 | 229 | GFP | F |
| vh565+Z127 | 230 | GFP | H |
| vh566+Z127 | 231 | GFP | K |
| vh567+Z127 | 232 | GFP | Q |
| vh568+Z127 | 233 | GFP | Y |
| vh167+Z127 | 169 | EGFR | R |
| vh571+Z127 | 234 | EGFR | F |
| vh572+Z127 | 235 | EGFR | H |
| vh573+Z127 | 236 | EGFR | K |
| vh574+Z127 | 237 | EGFR | Q |
| vh575+Z127 | 238 | EGFR | Y |
| vh118+Z127 | 162 | AAV9 | R |
| vh619+Z127 | 239 | AAV9 | F |
| vh620+Z127 | 240 | AAV9 | H |
| vh621+Z127 | 241 | AAV9 | K |
| vh622+Z127 | 242 | AAV9 | Q |
| vh623+Z127 | 243 | AAV9 | Y |

### Example 13A: Biacore analysis of binding affinity for GFP, EGFR and AAV9

To assess binding to the target molecule (GFP, EGFR, AAV9) candidate polypeptides were immobilized on a Biacore^{™} Series S CM5 chip as described above. To achieve similar coupling level between variants within each group the aim feature for coupling was for a good head-to-head evaluation.

Immobilization was performed using a standard method with aim for target level function in Biacore software with coupling of polypeptide variants in Flow Cell 2 (FC2) and activation/inactivation in Flow cell 1 (FC1). Polypeptide variants were diluted in Biacore^{™} Acetate buffer pH 5.0 (Cytiva^{™}) at a concentration of 25 µg/ml. The immobilization levels on CM5 chip ranged between about 1100 - 2100 RU for GFP, 2200-2500 RU for EGFR and 1800-2200 RU for AAV9.

Biacore^{™} method for binding analysis to AAV9: Running buffer: PBS-P+; Flow rate: 5 µl/min ; Sample injection: 2400 s/40 min over both Flow Cells (FC1 and FC2) ; Dissociation time: 2400 s/40 min; Regeneration: 10 mM Glycine-HCl pH 1.5, 30 µl/min, 2x30 s. Injections of analyte (AAV9) for each channel (cycles) were as follows: running buffer, 2E11 vp/ml, 5E11 vp/ml, 2E12 vp/ml.

Biacore^{™} method for binding analysis to GFP or EGFR: Running buffer: PBS-P+ ; Flow rate: 10 µl/min ; Sample injection: 600 s/10 min over both Flow Cells (FC1 and FC2) ; Dissociation time: 600 s/10 min; Regeneration: 10 mM Glycine-HCl pH 1.5, 30 µl/min, 2x30 s. Injections of analyte (GFP) for each channel immobilized with GFP binding polypeptides (cycles) were as follows: running buffer, 10 nM, 25 nM, 100 nM and 250 nM. Injections of analyte (EGFR) for each channel immobilized with EGFR binding polypeptides (cycles) were as follows: running buffer, 10 nM, 20 nM, 50 nM, 100 nM and 200 nM.

All sensorgrams were generated as reference subtracted and the responses as the difference between baseline before injection and signal just before end of injection.

### Example 13B: Biacore analysis of alkaline stability

After the binding analysis (see above) the same CM5 chips having immobilized polypeptides were subjected to repeated cycles of binding to the respective analyte followed by injection of NaOH (0.3 or 0.5 M) for the assessment of alkaline stability. The stability was measured with both binding to target molecule (CDR binding) and ZVH3 interaction against the framework. The mutations in pos71 for target molecule GFP result in almost abolished interaction and therefore alkaline stability with only ZVH3 was performed.

Biacore method for alkaline stability (per cycle): Running buffer: PBS-P+ ; Flow rate: 10 µl/min ; Sample injection 1 (AAV9 5E11 vp/ml, EGFR 75 nM or 50 nM ZVH3): 300 s over both flow cells ; Dissociation time 1: 30 s ; Sample injection 2 ( PBS-P+, 0.3 M NaOH (EGFR) or 0.5 M NaOH (AAV9, ZVH3): 600 s over both flow cells; Dissociation time 2: 0 s; Regeneration: 10 mM Glycine-HCl pH 1.5, 30 µl/min, 2x30 s. This cycle was repeated 40-100 times dependent on stability, to follow stability of target response values. Since only R binds for GFP, the experiments were not repeated for the alkaline stability, which is as shown in the examples above (50nM ZVH3 with 0.3 M NaOH).

All sensorgrams were generated as reference subtracted and the responses as the difference between baseline before injection and signal just before end of injection. For a reference binding each molecule (AAV9, EGFR and ZVH3) was injected followed with PBS-P+ instead of NaOH.

### Results

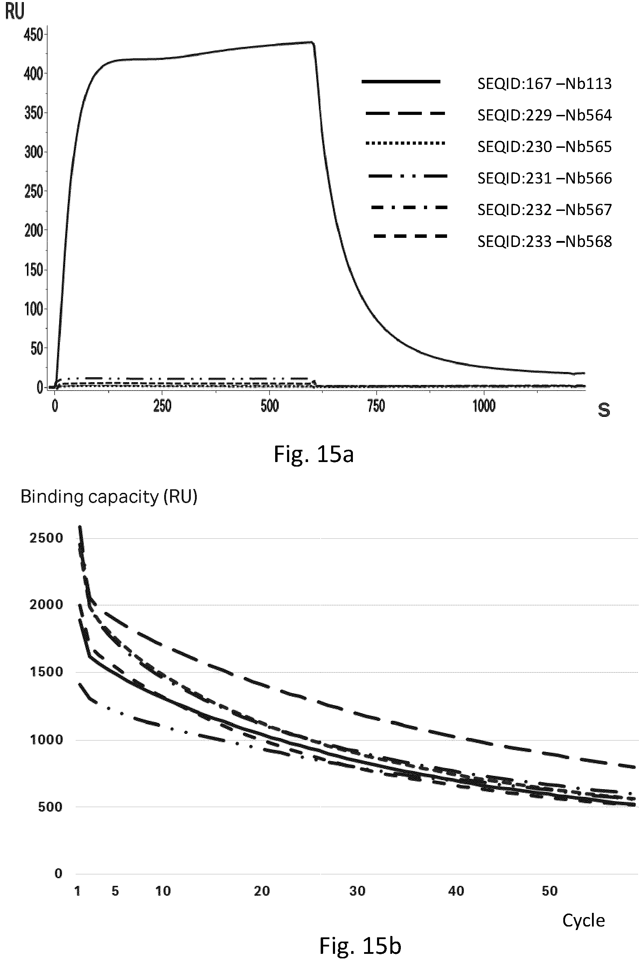

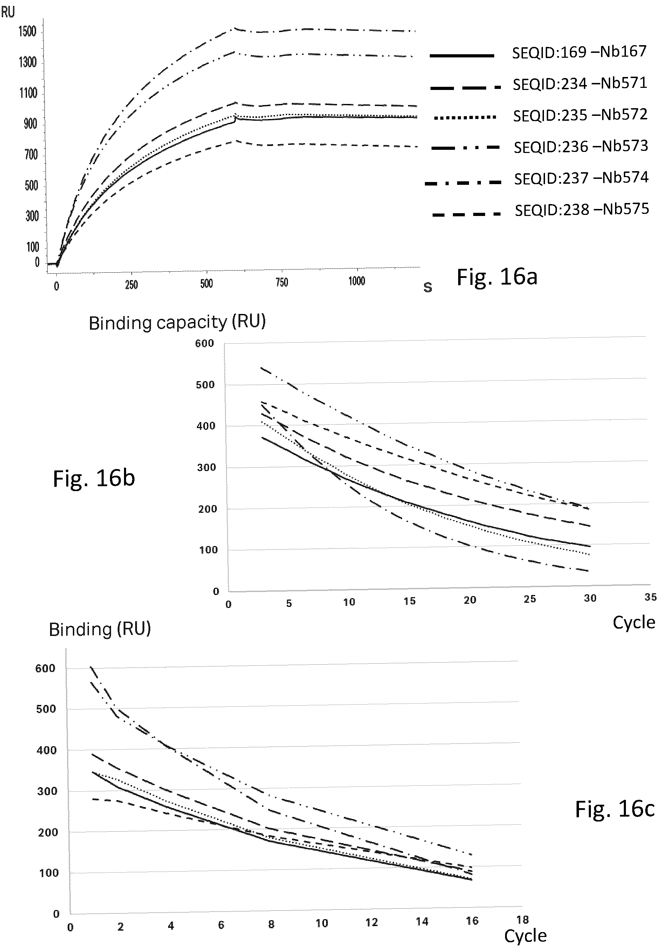

It was shown that varying the amino acid in position 71 greatly affect the binding interaction to the target for the fusion proteins, and that different results are obtained for different CDRs (different targets), indicating that the amino acid in this position either interact with one or several of the CDR(s) or the binding surface of the target, hence affecting the binding. The variation in position 71 can also affect the VH3 interaction and the alkaline stability. In Figs. 15-17 as described below, target binding is shown in (a), stability for ZVH3 binding is shown in (b) and for Figs. 16-17, stability for target binding is shown in (c).

For the GFP binding fusion proteins, only R in position 71 give rise to target binding (Fig. 15a). All other variants almost abolish the interaction to GFP with the current CDRs. However, from experiment B the binding ability for ZVH3 was significantly better for F and Q, better for H and Y than R (Fig. 15b). The ZVH3 binding ability significantly increased for F & Q and increased for H &Y compared to R (Fig. 15b). For alkaline stability for the framework then phenylalanine (F) is the most alkaline stable mutation for VHH for GFP (Fig. 15b).

For the EGFR binding fusion proteins, K or Q in position 71 give rise to best target binding, F and H are similar to R while Y is worse (Fig. 16a). The alkaline stability regarding EGFR binding ability there is no larger difference than Y have a slightly stable and Q have a slighter lower stability (Fig. 16b) in the group. Alkaline stability for the framework shows similar stability grouping as the target binding stability (Fig. 16c). Regarding the binding ability for the ZVH3 for the framework then all mutations (Y,F,Q,K, H) are better than R (Fig. 16b).

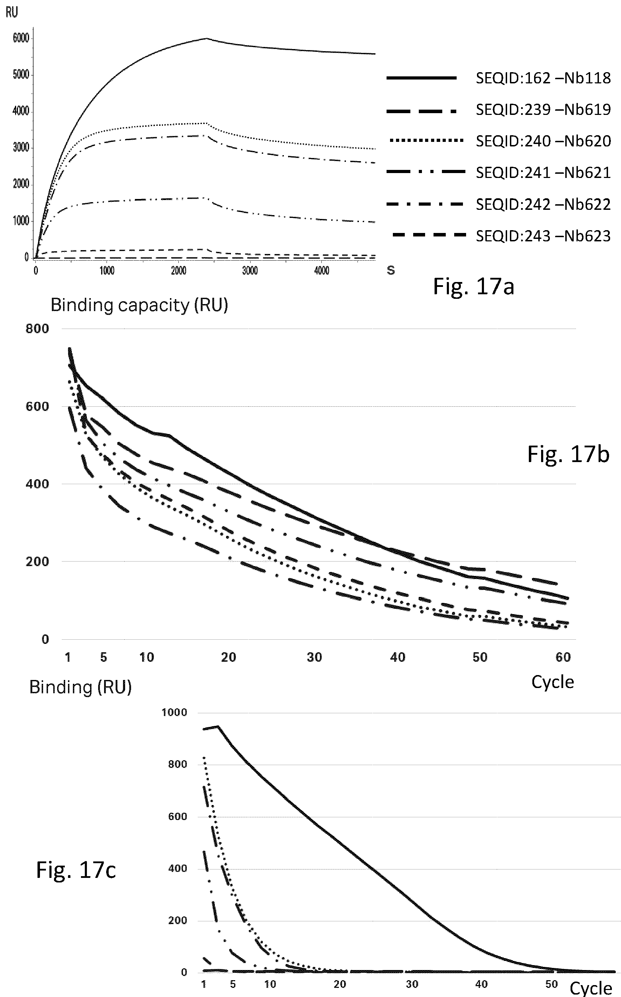

For the AAV9 binding fusion proteins, R in position 71 give rise to the best target binding, but also Q and H are adequate. The results are shown in Fig. 17. Mutation to K gives a low binding ability, and F and Y have almost abolished binding (Fig. 17a). However, binding ability for framework and alkaline stability via ZVH3 interaction are similar for all mutations but F and K have better stability than R (Fig. 17b). For alkaline stability for target molecule AAV9 then R is clearly the best amino acid in position 71 (Fig. 17c).

### LIST OF ITEMIZED EMBODIMENTS

1. A polypeptide comprising a heavy chain variable domain (VH) variant, such as a single domain antibody (sdAb) variant, comprising a plurality of antigen-binding regions and a framework comprising framework region 1 (FWR1), framework region 2 (FWR2) and framework region 3 (FWR3), wherein the VH variant has an amino acid sequence in which at least eight, at least nine, at least ten, such as all, of the following criteria i) to x) are fulfilled:
   i) the residue in Kabat position 19 is R, S, K or T;
   ii) the residue in Kabat position 23 is T, V, A or S, preferably T;
   iii) the residue in Kabat position 24 is I, V, A, R, N, D, Q, E, H, L, K, F, T, Y or S;
   iv) the residue in Kabat position 40 is selected from R, I, T or K, preferably R;
   v) the residue in Kabat position 43 is R, K, Q, E or G, preferably R or K;
   vi) the residue in Kabat position 44 is E, Q, A, D or G
   vii) the residue in Kabat position 45 is R, I or L, preferably R;
   viii) the residue in Kabat position 71 is F, H, K, Q, Y or R;
   ix) the residue in Kabat position 76 is N or Q;
   x) the residue in Kabat position 79 is Y, W or F; and
   xi) the residue in Kabat position 89 is V or I.
2. The polypeptide according to item 1, wherein the residue in Kabat position 71 is F, H, K, Q, or Y.
3. The polypeptide according to item 1 or 2, wherein the residue in Kabat position 24 is I, V, A, R, N, D, Q, E, H, L, K, F, T, Y or S;
4. The polypeptide according to items 1-3, wherein each of FWR1, FWR2 and FWR3 has at least 80 % identity to the amino acid sequence of a corresponding framework region of SEQ ID NO:80 wherein for the purpose of determining the sequence identity, the Kabat positions 14, 19, 23, 24, 40, 43, 44, 45,47, 60, 71, 76, 79, 82b and 89, and optionally Kabat position 6, are omitted.
5. A polypeptide comprising a VH variant, such as a single domain antibody (sdAb) variant, comprising a plurality of antigen-binding regions and a framework comprising framework region 1 (FWR1), framework region 2 (FWR2) and framework region 3 (FWR3), wherein the VH variant has an amino acid sequence in which at least five of the following criteria i) to vii) are fulfilled:
   i) the residue in Kabat position 24 is I, V, A, R, N, D, Q, E, H, L, K, F, T, Y or S;
   ii) the residue in Kabat position 40 is selected from R, I, K, or T, preferably R;
   iii) the residue in Kabat position 45 is R, I or L, preferably R;
   iv) the residue in Kabat position 71 is F, H, K, Q, Y or R;
   v) the residue in Kabat position 76 is N or Q;
   vi) the residue in Kabat position 79 is Y, W or F;
   vii) the residue in Kabat position 89 is V or I;
   and wherein each of FWR1, FWR2 and FWR3 has at least 80 % identity to the amino acid sequence of a corresponding framework region of SEQ ID NO: 80 wherein for the purpose of determining sequence identity, the Kabat positions 14, 19, 23, 24, 40, 43, 44, 45,47, 60, 71, 76, 79, 82b and 89, and optionally position 6, are omitted.
6. The polypeptide according to any one of items 1-4, which also fulfils at least five of the criteria i) to vii) according to item 5.
7.The polypeptide according to item 6, wherein all criteria i)-xi) of item 1 and all criteria i)-vii) of item 5 are fulfilled.
8. The polypeptide according to any one of the preceding items wherein the framework regions FWR1-3 together have at least 90 % sequence identity with the framework regions of SEQ ID NO: 80 wherein for the purpose of determining sequence identity, the Kabat positions 14, 19, 23, 24, 40, 43, 44, 45,47, 60, 71, 76, 79, 82b and 89, and optionally position 6, are omitted.
9. The polypeptide according to any one of the items 1-8, wherein for the purpose of determining the sequence identity, the Kabat position 6 is omitted.
10. The polypeptide according to any one of the items 1-8, wherein for the purpose of determining the sequence identity, the Kabat position 6 is not omitted.
11. The polypeptide according to any one of the preceding items, which is an antigen binding polypeptide.
12. The polypeptide according to any one of the preceding items, which is a single-chain polypeptide.
13. The polypeptide according to any one of the preceding items, wherein the polypeptide lacks antibody heavy chain constant domains.
14. The polypeptide according to any one of the preceding items, wherein said VH variant is a variable domain of a heavy chain of a heavy chain antibody (VHH) variant.
15. The polypeptide according to any one of the preceding items, wherein the amino acid sequence of the VH variant further comprises any one, such as more than one, such as all, of the following:
   the residue in Kabat position 6 is Q or E;
   the residue in Kabat position 14 is A, S or T;
   the residue in Kabat position 47 is F or L, preferably F;
   the residue in Kabat position 60 is A, T, N, Q or S, preferably A or S;
   the residue in Kabat position 82b is N, S, T or Q.
16. The polypeptide according to any one of the preceding items, wherein the residue in Kabat position 47 is F or L.
17. The polypeptide according to any one of the preceding items, wherein the residue in Kabat position 47 is not G, S, T or Y.
18. The polypeptide according to any one of the preceding items, wherein the residues in Kabat positions 24 and 40 are not both A.
19. The polypeptide according to any one of the preceding items, wherein the residue in Kabat position 40 is not A.
20. The polypeptide according to any one of the preceding items wherein the residue in Kabat position 76 is not T.
21. The polypeptide according to any one of the preceding items wherein the residue in Kabat position 76 is N or Q.
22. The polypeptide according to any one of the preceding items wherein the residue in Kabat position 89 is not E.
23. The polypeptide according to any one of the preceding items, wherein the residue in Kabat position 79 is Y, W or F, preferably Y or F, and the residue in Kabat position 89 is V or I.
24. The polypeptide according to any one of the preceding items, wherein FWR2 has at least 85 %, such as at least 88 %, sequence identity to Kabat position amino acids 36-49 (FWR2) of SEQ ID NO: 80 wherein for the purpose of determining sequence identity, the Kabat positions 40, 43, 44, 45 and 47 are omitted.
25. The polypeptide according to any one of the preceding items wherein FWR1 has at least 85 %, such as at least 90 %, sequence identity to Kabat position amino acids 1-26 (FWR1) of SEQ ID NO: 80 wherein for the purpose of determining sequence identity, the Kabat positions 6, 14, 19, 23 and 24 are omitted.
26. The polypeptide according to any one of the preceding items wherein the framework has at least 90 % sequence identity to the framework of SEQ ID NO: 80 wherein for the purpose of determining sequence identity, the Kabat positions 6, 14, 19, 23, 24, 40, 43, 44, 45,47, 60, 71, 76, 79, 82b and 89 are omitted.
27. The polypeptide according to any one of the preceding items, wherein framework region 1 (FWR1) has an amino acid sequence having at least 70 %, such as at least 75%, such as at least 80 %, such as at least 85 % identity to a sequence selected from the group consisting of SEQ ID Nos: 4-22 and 190.
28. The polypeptide according to item 27, wherein framework region 1 (FWR1) has an amino acid sequence selected from the group consisting of SEQ ID NOs: 4-22 and 190.
29. The polypeptide according to any one of the preceding items, wherein framework region 2 (FWR2) has an amino acid sequence having at least 70 %, such as at least 75%, such as at least 80 %, such as at least 85 % identity to a sequence selected from the group consisting of SEQ ID NOs:23-24 and 26-41.
30. The polypeptide according to item 29, wherein framework region 2 (FWR2) has an amino acid sequence selected from the group consisting of SEQ ID NOs: 23-24 and 26-41, such as the group consisting of SEQ ID NOs: 26, 32-39 and 41, such as the group consisting of SEQ ID NOs: 26, 38 and 41.
31. The polypeptide according to any one of the preceding items, wherein framework region 3 (FWR3) has an amino acid sequence having at least 70 %, such as at least 75%, such as at least 80 %, such as at least 85 % identity to a sequence selected from the group consisting of SEQ ID NOs: 45-49, 54-55, 58-63, 65, 67-70, 191 and 224-228.
32. The polypeptide according to item 31, wherein framework region 3 (FWR3) has an amino acid sequence selected from the group consisting of SEQ ID NOs: 45-49, 54-55, 58-63, 65, 67-70, 191 and 224-228.
33. The polypeptide according to any one of the preceding items, wherein the framework regions together have at least 92 %, such as at least 94 %, such as at least 97 %,sequence identity with the framework regions of SEQ ID NO:80, wherein for the purpose of establishing sequence identity, the Kabat positions 6, 14, 19, 23, 24, 40, 43, 44, 45,47, 60, 71, 76, 79, 82b and 89 are omitted.
34. The polypeptide according to any one of the preceding items, wherein the VH variant has a framework region 1 (FWR1) comprising an amino acid sequence according to
   X₁X₂QLX₅X₆SGGGX₁₁VQX₁₄GGSLX₁₉LSCX₂₃X₂₄SG (SEQ ID NO: 1)
   wherein, independently,
   X₁is Q, V, D or E, preferably Q or E;
   X₂ is V or D, preferably V;
   X₅ is Q, V or E, preferably Q;
   X₆ is Q or E, preferably Q;
   X₁₁ is S or L, preferably S;
   X₁₄ is A, S, or T, preferably A;
   X₁₉ is R, S, K or T, preferably R or T;
   X₂₃ is T, V, A or S, preferably T; and
   X₂₄ is I, V, A, R, N, D, Q, E, H, L, K, F, T, Y or S, preferably I.
35. The polypeptide according to any one of the preceding items, wherein the VH variant has a framework region 2 (FWR2) comprising an amino acid sequence according to
   WFRQX₅PGX₈X₉X₁₀EX₁₂VA (SEQ ID NO: 2)
   wherein, independently,
   X₅ is R, I, T or K, preferably R;
   X₈ is R, K, Q, E or G, preferably K or R;
   X₉ is E, Q, A, D or G;
   X₁₀ is R, I or L, preferably R; and
   X₁₂ is F or L, preferably F.
36. The polypeptide according to any one of the preceding items, wherein the VH variant has a framework region 3 (FWR3) comprising an amino acid sequence according to
   YX₂X₃X₄VX₆GRFTISX₁₃DNAKX₁₈TX₂₀X₂₁LQMNX₂₆LKPEDTAX₃₄YYCAA (SEQ ID NO: 3)
   wherein, independently,
   X₂ is A, T, N, Q or S, preferably A;
   X₃ is D or S, preferably D;
   X₄ is S or A, preferably S;
   X₆ is K or A, preferably K;
   X₁₃ is F, H, K, Q, Y or R;
   X₁₈ is N or Q, preferably N;
   X₂₀ is V or A, preferably V;
   X₂₁is Y, W or F, preferably Y or W;
   X₂₆ is N, S, T or Q, preferably S; and
   X₃₄ is V or I.
37. The polypeptide according to any one of the preceding items, wherein the VH variant comprises a framework region 4 (FWR4) comprising the amino acid sequence
   WGQGTQVTVSS (SEQ ID NO: 71)
   or an amino acid sequence having at least 70 %, such as at least 75 %, such as at least 80 % identity with SEQ ID NO: 71, with the proviso that the amino acid residue in position 1 is W.
38. The polypeptide according to any one of the preceding items wherein the VH variant has a framework corresponding to the framework of a VH variant selected from the group consisting of SEQ ID NOs: 72-78, 80-114, 118-121, 126-127,130-135,137, 139-151, 156,158 and 229-243.
39. The polypeptide according to item 38, wherein the VH variant has a framework corresponding to the framework of a VH selected from the group consisting of SEQ ID NOs: 76, 78, 80-114, 118-121, 126-127, 130-135, 137, 139-151, 156, 158 and 229-243.
40. The polypeptide according to any one of the preceding items, wherein said VH variant does not contain any histidine (H) residues.
41. The polypeptide according to any one of the preceding items, comprising a further amino acid sequence, optionally selected from the group consisting of: a leader peptide, a signal peptide, a purification tag, an affinity tag, a coupling peptide, a linker peptide, a spacer peptide.
42. The polypeptide according to item 40, wherein said further amino acid sequence is positioned N-terminally or C-terminally of the polypeptide.
43. The polypeptide according to item 41 or 42, wherein said further amino acid sequence is a leader peptide or signal peptide.
44. The polypeptide according to item 41, wherein said further amino acid sequence comprises a plurality of histidines, such as a (His)₆ sequence.
45. A multimeric polypeptide comprising at least two moieties, each moiety being a VH variant as defined in any one of the items 1-40, said moieties optionally being joined by a peptide linker.
46. The multimeric polypeptide according to item 45, comprising three VH variants.
47. The multimeric polypeptide according to item 45 or 46, wherein said VH variants are identical.
48. The multimeric polypeptide according to any one of items 45-47, wherein said VH variants have different CDRs.
49. A fusion protein comprising at least one polypeptide according to any one of the preceding items and at least one further polypeptide moiety.
50. The fusion protein according to item 49, wherein the further polypeptide moiety comprises an α-helix-containing domain.
51. The fusion protein according to item 50, wherein the further polypeptide moiety comprises an α-helix bundle domain.
52. The fusion protein according to item 51, wherein the further polypeptide moiety is derived from a protein domain of staphylococcal protein A (SpA).
53. The fusion protein according to item 51 or 52, wherein the further polypeptide moiety comprises a sequence having least 80 % identity such as at least 85 % or at least 90 % identity to SEQ ID NO: 159 or to SEQ ID NO: 160.
54. The fusion protein according to any one of items 49-53, wherein said further polypeptide moiety is positioned C-terminal of said VH variant.
55. The fusion protein according to any one of items 49-54, comprising two or more copies of said further polypeptide moiety.
56. The fusion protein according to any one of items 49-54, comprising the following structure
   ([A-L1]ₘ-[Z-L2]ₙ)ₚ
   wherein
   A represents a polypeptide as defined in any one of items 1-44,
   L1 for each occurrence may be present or absent, and where present, represents a linker or spacer,
   Z represents a further polypeptide moiety according to any one of items 50-53,
   L2 for each occurrence may be present or absent, and where present, represents a linker or spacer,
   m represents an integer of from 1 to 4,
   n represents an integer of from 1 to 4, or when m≥2, n represents 0 or an integer of from 1 to 4,
      and
   p represents an integer of from 1 to 4.
57. The fusion protein according to item 56 wherein m is 2 or 3, n is 1 and p is 1.
58. The fusion protein according to item 56 wherein m is 1, n is 1 and p is 2 or 3.
59. The fusion protein according to any one of items 49-58, comprising a C-terminal tag comprising a plurality of histidine residues, preferably at least 6 histidine residues.
60. The fusion protein according to item 59, wherein the fusion protein except for the tag does not contain any histidine residues.
61. An isolated nucleic acid encoding a polypeptide, a multimer or a fusion protein according to any one of items 1-60.
62. An expression vector comprising the nucleic acid of item 61.
63. A recombinant host cell for the production of the polypeptide, the multimer or the fusion protein of any one of items 1-60, said host cell comprising the expression vector of item 62.
64. The recombinant host cell of item 63, wherein the cells are prokaryotic cells, such as E. *coli* cells.
65. The recombinant host cell of item 63, wherein the cells are eukaryotic cells.
66. The recombinant host cell of item 65, wherein the eukaryotic cells are yeast cells, such as *S. cerevisiae* or *P. pastoris.*
67. The recombinant host cell of item 65, wherein the eukaryotic cells are insect cells.
68. The recombinant host cell of item 65, wherein the eukaryotic cells are animal cells, such as mammalian cells, such as Chinese hamster ovary (CHO) cells or human embryonic kidney (HEK) cells.
69. A method of producing the polypeptide, the multimer or the fusion protein of any one of items 1-60, comprising
   i. providing recombinant host cells according to any one of items 63-68;
   ii. culturing the host cell under conditions enabling expression of the polypeptide, multimer or fusion protein; and
   iii. isolating the polypeptide, multimer or fusion protein.
70. The method of any one of items 64-69, wherein step iii comprises purifying the polypeptide, multimer or fusion protein by affinity chromatography.
71. The method of item 70, wherein the affinity chromatography uses an affinity ligand that binds to a framework region of the VH variant.
72. Use of the polypeptide of any one of items 1-44, the multimer of any one of item 45-48, or the fusion protein of any one of items 49-60 as affinity ligand for capturing a target entity.
73. Use according to item 72 for the *in vitro* detection and/or purification of the target entity.
74. An adsorbent material comprising polypeptide of any one of items 1-44, the multimer of any one of items 45-48, or the fusion protein of any one of items 49-560coupled to a solid support.
75. The adsorbent material according to item 74, wherein the support material is a surface.
76. The adsorbent material according to item 74 or 75, wherein the support is selected from the group consisting of a particle, a bead, a fiber, a fibrous membrane, a filter, a sheet, a porous monolith, a chip, a plate, and a well.
77. The adsorbent material according to any one of items 74-76, wherein the support comprises a polymeric material.
78. The adsorbent material according to item 77, wherein the support comprises a polysaccharide-based material, such as cellulose or agarose and derivatives thereof.
79. The adsorbent material according to item 78, wherein the support comprises agar or agarose or a derivative thereof, such as crosslinked agarose.
80. The adsorbent material according to any one of items 74-78, wherein the support material is a fibrous material, such as a material comprising nanofibers.
81. The adsorbent material according to item 80, wherein the support material is a fibrous matrix, such as a nonwoven fibrous matrix.
82. The adsorbent material according to any one of items 74-81, wherein the support is a chromatography matrix.
83. The adsorbent material according to item 82, wherein the support material is a chromatography matrix selected from the group consisting of a fibrous matrix, a membrane, a filter and a monolith.
84. Use of the adsorbent material according to any one of items 74-83 for binding of the target entity.
85. Use according to item 84, for the detection of said target entity within a sample.
86. Use according to item 84, for separation of said target entity from other components of a sample.
87. Use according to item 86 for analytical separation of the target entity.
88. Use according to item 84 for preparative purification of the target entity.
89. A method of separation, comprising the steps of
   (a) Providing an adsorbent material according to any one of items 74-83 wherein the VH variant of the polypeptide has a binding affinity for a target entity,
   (b) Contacting the adsorbent material with a liquid sample comprising said target entity under conditions allowing the target entity to bind to the polypeptide,
   (c) Optionally washing the adsorbent material,
   (d) Eluting the target entity from the adsorbent material, and
   (e) Cleaning the adsorbent material with a cleaning liquid.
90. The method of item 89, wherein the cleaning liquid is alkaline, and preferably comprises from 0.05 to 0.5 M NaOH, such as 0.1-0.5 M NaOH.
91. The method of item 89 or 90 wherein steps (a)-(e) are repeated at least 10 times, such as at least 20 times.
92. The method of any one of items 89-91, wherein after 12 cycles of contact with alkaline cleaning liquid, the polypeptide or fusion protein retains at least 50 % such as at least 60 %, such as at least 80 %, such as at least 90 %, of the initial target entity binding capacity.
93. The method of item 92, wherein after 15 cycles of contact with alkaline cleaning liquid, the polypeptide or fusion protein retains at least 50 %, such as at least 60 %, such as at least 80 %, such as at least 90 %, of its initial target entity binding capacity.
94. The method of any one of items 89-91, wherein, after 12 cycles of contact with alkaline cleaning liquid, the polypeptide or the fusion protein retains at least 50 % such as at least 60 %, such as at least 80 %, such as at least 90 %, such as at least 95 %, such as at least 98 %, of the target entity binding capacity of the 2^{nd} cycle.
95. The method of item 94, wherein after 15 cycles of contact with alkaline cleaning liquid, the polypeptide or the fusion protein retains at least 50 %, such as at least 60 %, such as at least 80 %, such as at least 90 %, such as at least 95 %, of the target entity binding capacity of the 2^{nd} cycle.
96. A method of selecting a variant of a polypeptide, multimer or the fusion protein of any one of items 1-60 that is optimized for target entity binding, the method comprising:
   (a) providing a polypeptide, multimer or fusion protein of any one of items 1-60, the polypeptide, multimer or fusion protein comprising CDRs selected for binding a target entity;
   (b) providing variants of the polypeptide, multimer or fusion protein, wherein the residue in Kabat position 71 for each variant has been altered or selected to be one of F, H, K, Q, Y or R;
   (c) evaluating, for each variant, the binding affinity or binding selectivity for the target entity; and
   (d) selecting the variant that provides the highest binding affinity or binding selectivity for the target entity as the variant most optimized for target entity binding.
97. The method of item 96, wherein, for each variant of the polypeptide, multimer or fusion protein, the method comprises:
   (e) providing sub-variants of the variants, wherein the residue in Kabat position 24 for each sub-variant has been altered to be one of I, V, A, R, N, D, Q, E, H, L, K, F, T, Y or S;
   (f) evaluating, for each sub-variant, the binding affinity or binding selectivity for the target entity; and
   (g) selecting the sub-variant that provides the highest binding affinity or binding selectivity for the target entity as the sub-variant most optimized for target entity binding.
98. A method of providing an enhanced variant of a target entity binding polypeptide, multimer or the fusion protein of any one of items 1-60, the method comprising:
   (a) providing a polypeptide, multimer or fusion protein having a framework FW1, FW2 and FW3 of any one of items 1-60, wherein the residue in Kabat position 71 has been altered to one of F, H, K, Q, Y or R and/or the residue in Kabat position 24 for each sub-variant has been altered to be one of I, V, A, R, N, D, Q, E, H, L, K, F, T, Y or S;
   (b) screening a library of CDRs that bind the target entity;
   (c) selecting the CDRs that in combination with the framework provides the best binding affinity or binding selectivity for the target entity.

**Table 18. Exemplary amino acid sequences**

| **SEQ ID NO** | **Amino acid sequence** |
|---|---|
| 4 | QVQLQESGGGSVQAGGSLRLSCVASG |
| 5 | QVQLQESGGGSVQAGGSLTLSCVASG |
| 6 | QVQLQESGGGSVQAGGSLRLSCTASG |
| 7 | QVQLQESGGGSVQAGGSLTLSCTASG |
| 8 | QVQLQQSGGGSVQAGGSLRLSCTISG |
| 9 | QVQLQESGGGSVQAGGSLRLSCTISG |
| 10 | QVQLQQSGGGSVQAGGSLRLSCTVSG |
| 11 | QVQLQQSGGGSVQAGGSLTLSCTISG |
| 12 | QVQLVQSGGGSVQAGGSLRLSCTISG |
| 13 | QVQLEQSGGGSVQAGGSLRLSCTISG |
| 14 | QVQLQQSGGGSVQSGGSLRLSCTISG |
| 15 | QVQLQQSGGGSVQTGGSLRLSCTISG |
| 16 | QVQLQQSGGGSVQAGGSLKLSCTISG |
| 17 | QVQLQQSGGGSVQAGGSLSLSCTISG |
| 18 | QVQLQQSGGGSVQAGGSLRLSCVISG |
| 19 | QVQLQQSGGGSVQAGGSLRLSCSISG |
| 20 | QVQLQQSGGGSVQAGGSLRLSCAISG |
| 21 | QVQLQQSGGGSVQAGGSLRLSCTSSG |
| 22 | QVQLVESGGGSVQAGGSLSLSCTISG |
| 190 | QVQLQQSGGGSVQAGGSLTLSCTVSG |
| 23 | WFRQPPGKAREFVA |
| 24 | WFRQRPGKAREFVA |
| 26 | WFRQRPGKEREFVA |
| 27 | WFRQPPGKEREFVA |
| 28 | WFRQTPGKEREFVA |
| 29 | WFRQIPGKEREFVA |
| 30 | WFRQKPGKEREFVA |
| 31 | WFRQAPGKEREFVA |
| 32 | WFRQRPGGEREFVA |
| 33 | WFRQRPGQEREFVA |
| 34 | WFRQRPGREREFVA |
| 35 | WFRQRPGKQREFVA |
| 36 | WFRQRPGKDREFVA |
| 37 | WFRQRPGKGREFVA |
| 38 | WFRQRPGKELEFVA |
| 39 | WFRQRPGKEIEFVA |
| 40 | WFRQRPGKERELVA |
| 41 | WFRQRPGRAREFVA |
| 45 | YADSVKGRFTISRDNAKNTVYLQMNSLKPEDTAVYYCAA |
| 46 | YNDSVKGRFTISRDNAKNTVYLQMNSLKPEDTAVYYCAA |
| 47 | YQOSVKGRFTISRONAKNTVYLQMNSLKPEDTAVYYCAA |
| 48 | YTDSVKGRFTISRDNAKNTVYLQMNSLKPEDTAVYYCAA |
| 49 | YSOSVKGRFTISRONAKNTVYLQMNSLKPEDTAVYYCAA |
| 54 | YADSVKGRFTISRDNAKQTVYLQMNSLKPEDTAVYYCAA |
| 55 | YADSVKGRFTISRDNAKDTVYLQMNSLKPEDTAVYYCAA |
| 58 | YADSVKGRFTISRDNAKNTVALQMNSLKPEDTAVYYCAA |
| 59 | YADSVKGRFTISRDNAKNTVFLQMNSLKPEDTAVYYCAA |
| 60 | YADSVKGRFTISRDNAKNTVWLQMNSLKPEDTAVYYCAA |
| 61 | YADSVKGRFTISRDNAKNTVYLQMNNLKPEDTAVYYCAA |
| 62 | YADSVKGRFTISRDNAKNTVYLQMNTLKPEDTAVYYCAA |
| 63 | YADSVKGRFTISRDNAKNTVYLQMNQLKPEDTAVYYCAA |
| 65 | YADSVKGRFTISRDNAKNTVYLQMNSLKPEDTAAYYCAA |
| 67 | YADSVKGRFTISRDNAKNTVYLQMNSLKPEDTAIYYCAA |
| 68 | YADSVKGRFTISRDNAKNTVYLQMNSLKPEDTALYYCAA |
| 69 | YTDSVKGRFTISRDNAKNTVFLQMQSLKPEDTAIYYCAA |
| 70 | YTDSVKGRFTISRDNAKNTVYLQMQSLKPEDTAVYYCAA |
| 191 | YADSVKGRFTISRDNAKNTVFLOMNSLKPEDTALYYCAA |
| 71 | WGQGTQVTVSS |
| 72 | QVQLQESGGGSVQAGGSLRLSCVASGRTLSDYTMGWFRQPPGKAREFVAAISWSGAYTKYADS VKGRFTISRDNAKNTVYLQMNSLKPEDTAVYYCAAGPTGLLSKKATPADYDYWGQGTQVTVSS |
| 73 | QVQLQESGGGSVQAGGSLRLSCVASGRTLSDYTMGWFRQPPGKAREFVAAISWSGAYTYYADS VKGRFTISRDNAKNTVYLQMNSLKPEDTAVYYCAAGPTGLLSKKATPADYDYWGQGTQVTVSS |
| 74 | QVQLQESGGGSVQAGGSLTLSCVASGRTLSDYTMGWFRQPPGKAREFVAAISWSGAYTYYADS VKGRFTISRDNAKNTVYLQMNSLKPEDTAVYYCAAGPTGLLSKKATPADYDYWGQGTQVTVSS |
| 75 | QVQLQESGGGSVQAGGSLRLSCTASGRTLSDYTMGWFRQPPGKAREFVAAISWSGAYTYYADS VKGRFTISRDNAKNTVYLQMNSLKPEDTAVYYCAAGPTGLLSKKATPADYDYWGQGTQVTVSS |
| 76 | QVQLQESGGGSVQAGGSLRLSCVASGRTLSDYTMGWFRQRPGKAREFVAAISWSGAYTYYADS VKGRFTISRDNAKNTVYLQMNSLKPEDTAVYYCAAGPTGLLSKKATPADYDYWGQGTQVTVSS |
| 77 | QVQLQESGGGSVQAGGSLTLSCTASGRTLSDYTMGWFRQPPGKAREFVAAISWSGAYTYYADS VKGRFTISRDNAKNTVYLQMNSLKPEDTAVYYCAAGPTGLLSKKATPADYDYWGQGTQVTVSS |
| 78 | QVQLQESGGGSVQAGGSLTLSCTASGRTLSDYTMGWFRQRPGKAREFVAAISWSGAYTYYADS VKGRFTISRDNAKNTVYLQMNSLKPEDTAVYYCAAGPTGLLSKKATPADYDYWGQGTQVTVSS |
| 80 | QVQLQQSGGGSVQAGGSLRLSCTISGRTLSDYTMGWFRQRPGKEREFVAAISWSGAYTKYADS VKGRFTISRDNAKNTVYLQMNSLKPEDTAVYYCAAGPTGLLSKKATPADYDYWGQGTQVTVSS |
| 81 | QVQLQESGGGSVQAGGSLRLSCTISGRTLSDYTMGWFRQRPGKEREFVAAISWSGAYTKYADSV KGRFTISRDNAKNTVYLQMNSLKPEDTAVYYCAAGPTGLLSKKATPADYDYWGQGTQVTVSS |
| 82 | QVQLQQSGGGSVQAGGSLRLSCTVSGRTLSDYTMGWFRQRPGKEREFVAAISWSGAYTKYADS VKGRFTISRDNAKNTVYLQMNSLKPEDTAVYYCAAGPTGLLSKKATPADYDYWGQGTQVTVSS |
| 83 | QVQLQQSGGGSVQAGGSLRLSCTISGRTLSDYTMGWFRQPPGKEREFVAAISWSGAYTKYADS VKGRFTISRDNAKNTVYLQMNSLKPEDTAVYYCAAGPTGLLSKKATPADYDYWGQGTQVTVSS |
| 84 | QVQLQQSGGGSVQAGGSLRLSCTISGRTLSDYTMGWFRQRPGKAREFVAAISWSGAYTKYADS VKGRFTISRDNAKNTVYLQMNSLKPEDTAVYYCAAGPTGLLSKKATPADYDYWGQGTQVTVSS |
| 85 | QVQLQESGGGSVQAGGSLRLSCTISGRTLSDYTMGWFRQRPGKAREFVAAISWSGAYTKYADSV KGRFTISRDNAKNTVYLQMNSLKPEDTAVYYCAAGPTGLLSKKATPADYDYWGQGTQVTVSS |
| 86 | QVQLQQSGGGSVQAGGSLRLSCTVSGRTLSDYTMGWFRQRPGKAREFVAAISWSGAYTKYADS VKGRFTISRDNAKNTVYLQMNSLKPEDTAVYYCAAGPTGLLSKKATPADYDYWGQGTQVTVSS |
| 87 | QVQLQQSGGGSVQAGGSLRLSCTISGRTLSDYTMGWFRQPPGKAREFVAAISWSGAYTKYADS VKGRFTISRDNAKNTVYLQMNSLKPEDTAVYYCAAGPTGLLSKKATPADYDYWGQGTQVTVSS |
| 88 | QVQLQQSGGGSVQAGGSLTLSCTISGRTLSDYTMGWFRQRPGKEREFVAAISWSGAYTKYADS VKGRFTISRDNAKNTVYLQMNSLKPEDTAVYYCAAGPTGLLSKKATPADYDYWGQGTQVTVSS |
| 89 | QVQLVQSGGGSVQAGGSLRLSCTISGRTLSDYTMGWFRQRPGKEREFVAAISWSGAYTKYADSV KGRFTISRDNAKNTVYLQMNSLKPEDTAVYYCAAGPTGLLSKKATPADYDYWGQGTQVTVSS |
| 90 | QVQLEQSGGGSVQAGGSLRLSCTISGRTLSDYTMGWFRQRPGKEREFVAAISWSGAYTKYADSV KGRFTISRDNAKNTVYLQMNSLKPEDTAVYYCAAGPTGLLSKKATPADYDYWGQGTQVTVSS |
| 91 | QVQLQESGGGSVQAGGSLRLSCTISGRTLSDYTMGWFRQRPGKEREFVAAISWSGAYTKYADSV KGRFTISRDNAKNTVYLQMNSLKPEDTAVYYCAAGPTGLLSKKATPADYDYWGQGTQVTVSS |
| 92 | QVQLQQSGGGSVQSGGSLRLSCTISGRTLSDYTMGWFRQRPGKEREFVAAISWSGAYTKYADSV KGRFTISRDNAKNTVYLQMNSLKPEDTAVYYCAAGPTGLLSKKATPADYDYWGQGTQVTVSS |
| 93 | QVQLQQSGGGSVQTGGSLRLSCTISGRTLSDYTMGWFRQRPGKEREFVAAISWSGAYTKYADSV KGRFTISRDNAKNTVYLQMNSLKPEDTAVYYCAAGPTGLLSKKATPADYDYWGQGTQVTVSS |
| 94 | QVQLQQSGGGSVQAGGSLKLSCTISGRTLSDYTMGWFRQRPGKEREFVAAISWSGAYTKYADS VKGRFTISRDNAKNTVYLQMNSLKPEDTAVYYCAAGPTGLLSKKATPADYDYWGQGTQVTVSS |
| 95 | QVQLQQSGGGSVQAGGSLSLSCTISGRTLSDYTMGWFRQRPGKEREFVAAISWSGAYTKYADSV KGRFTISRDNAKNTVYLQMNSLKPEDTAVYYCAAGPTGLLSKKATPADYDYWGQGTQVTVSS |
| 96 | QVQLQQSGGGSVQAGGSLRLSCVISGRTLSDYTMGWFRQRPGKEREFVAAISWSGAYTKYADS VKGRFTISRDNAKNTVYLQMNSLKPEDTAVYYCAAGPTGLLSKKATPADYDYWGQGTQVTVSS |
| 97 | QVQLQQSGGGSVQAGGSLRLSCSISGRTLSDYTMGWFRQRPGKEREFVAAISWSGAYTKYADS VKGRFTISRDNAKNTVYLQMNSLKPEDTAVYYCAAGPTGLLSKKATPADYDYWGQGTQVTVSS |
| 98 | QVQLQQSGGGSVQAGGSLRLSCAISGRTLSDYTMGWFRQRPGKEREFVAAISWSGAYTKYADS VKGRFTISRDNAKNTVYLQMNSLKPEDTAIYYCAAGPTGLLSKKATPADYDYWGQGTQVTVSS |
| 99 | QVQLQQSGGGSVQAGGSLRLSCTSSGRTLSDYTMGWFRQRPGKEREFVAAISWSGAYTKYADS VKGRFTISRDNAKNTVYLQMNSLKPEDTAVYYCAAGPTGLLSKKATPADYDYWGQGTQVTVSS |
| 100 | QVQLQQSGGGSVQAGGSLRLSCTVSGRTLSDYTMGWFRQRPGKEREFVAAISWSGAYTKYADS VKGRFTISRDNAKNTVYLQMNSLKPEDTAVYYCAAGPTGLLSKKATPADYDYWGQGTQVTVSS |
| 101 | QVQLQQSGGGSVQAGGSLRLSCTISGRTLSDYTMGWFRQTPGKEREFVAAISWSGAYTKYADS VKGRFTISRDNAKNTVYLQMNSLKPEDTAVYYCAAGPTGLLSKKATPADYDYWGQGTQVTVSS |
| 102 | QVQLQQSGGGSVQAGGSLRLSCTISGRTLSDYTMGWFRQIPGKEREFVAAISWSGAYTKYADSV KGRFTISRDNAKNTVYLQMNSLKPEDTAVYYCAAGPTGLLSKKATPADYDYWGQGTQVTVSS |
| 103 | QVQLQQSGGGSVQAGGSLRLSCTISGRTLSDYTMGWFRQKPGKEREFVAAISWSGAYTKYADS VKGRFTISRDNAKNTVYLQMNSLKPEDTAVYYCAAGPTGLLSKKATPADYDYWGQGTQVTVSS |
| 104 | QVQLQQSGGGSVQAGGSLRLSCTISGRTLSDYTMGWFRQAPGKEREFVAAISWSGAYTKYADS VKGRFTISRDNAKNTVYLQMNSLKPEDTAIYYCAAGPTGLLSKKATPADYDYWGQGTQVTVSS |
| 105 | QVQLQQSGGGSVQAGGSLRLSCTISGRTLSDYTMGWFRQRPGGEREFVAAISWSGAYTKYADS VKGRFTISRDNAKNTVYLQMNSLKPEDTAVYYCAAGPTGLLSKKATPADYDYWGQGTQVTVSS |
| 106 | QVQLQQSGGGSVQAGGSLRLSCTISGRTLSDYTMGWFRQRPGQEREFVAAISWSGAYTKYADS VKGRFTISRDNAKNTVYLQMNSLKPEDTAVYYCAAGPTGLLSKKATPADYDYWGQGTQVTVSS |
| 107 | QVQLQQSGGGSVQAGGSLRLSCTISGRTLSDYTMGWFRQRPGREREFVAAISWSGAYTKYADS VKGRFTISRDNAKNTVYLQMNSLKPEDTAVYYCAAGPTGLLSKKATPADYDYWGQGTQVTVSS |
| 108 | QVQLQQSGGGSVQAGGSLRLSCTISGRTLSDYTMGWFRQRPGKQREFVAAISWSGAYTKYADS VKGRFTISRDNAKNTVYLQMNSLKPEDTAVYYCAAGPTGLLSKKATPADYDYWGQGTQVTVSS |
| 109 | QVQLQQSGGGSVQAGGSLRLSCTISGRTLSDYTMGWFRQRPGKAREFVAAISWSGAYTKYADS VKGRFTISRDNAKNTVYLQMNSLKPEDTAVYYCAAGPTGLLSKKATPADYDYWGQGTQVTVSS |
| 110 | QVQLQQSGGGSVQAGGSLRLSCTISGRTLSDYTMGWFRQRPGKDREFVAAISWSGAYTKYADS VKGRFTISRDNAKNTVYLQMNSLKPEDTAVYYCAAGPTGLLSKKATPADYDYWGQGTQVTVSS |
| 111 | QVQLQQSGGGSVQAGGSLRLSCTISGRTLSDYTMGWFRQRPGKGREFVAAISWSGAYTKYADS VKGRFTISRDNAKNTVYLQMNSLKPEDTAVYYCAAGPTGLLSKKATPADYDYWGQGTQVTVSS |
| 112 | QVQLQQSGGGSVQAGGSLRLSCTISGRTLSDYTMGWFRQRPGKELEFVAAISWSGAYTKYADSV KGRFTISRDNAKNTVYLQMNSLKPEDTAVYYCAAGPTGLLSKKATPADYDYWGQGTQVTVSS |
| 113 | QVQLQQSGGGSVQAGGSLRLSCTISGRTLSDYTMGWFRQRPGKEIEFVAAISWSGAYTKYADSV KGRFTISRDNAKNTVYLQMNSLKPEDTAVYYCAAGPTGLLSKKATPADYDYWGQGTQVTVSS |
| 114 | QVQLQQSGGGSVQAGGSLRLSCTISGRTLSDYTMGWFRQRPGKERELVAAISWSGAYTKYADS VKGRFTISRDNAKNTVYLQMNSLKPEDTAIYYCAAGPTGLLSKKATPADYDYWGQGTQVTVSS |
| 118 | QVQLQQSGGGSVQAGGSLRLSCTISGRTLSDYTMGWFRQRPGKEREFVAAISWSGAYTKYNDS VKGRFTISRDNAKNTVYLQMNSLKPEDTAVYYCAAGPTGLLSKKATPADYDYWGQGTQVTVSS |
| 119 | QVQLQQSGGGSVQAGGSLRLSCTISGRTLSDYTMGWFRQRPGKEREFVAAISWSGAYTKYQDS VKGRFTISRDNAKNTVYLQMNSLKPEDTAVYYCAAGPTGLLSKKATPADYDYWGQGTQVTVSS |
| 120 | QVQLQQSGGGSVQAGGSLRLSCTISGRTLSDYTMGWFRQRPGKEREFVAAISWSGAYTKYTDSV KGRFTISRDNAKNTVYLQMNSLKPEDTAVYYCAAGPTGLLSKKATPADYDYWGQGTQVTVSS |
| 121 | QVQLQQSGGGSVQAGGSLRLSCTISGRTLSDYTMGWFRQRPGKEREFVAAISWSGAYTKYSDSV KGRFTISRDNAKNTVYLQMNSLKPEDTAVYYCAAGPTGLLSKKATPADYDYWGQGTQVTVSS |
| 126 | QVQLQQSGGGSVQAGGSLRLSCTISGRTLSDYTMGWFRQRPGKEREFVAAISWSGAYTKYADS VKGRFTISRDNAKQTVYLQMNSLKPEDTAVYYCAAGPTGLLSKKATPADYDYWGQGTQVTVSS |
| 127 | QVQLQQSGGGSVQAGGSLRLSCTISGRTLSDYTMGWFRQRPGKEREFVAAISWSGAYTKYADS VKGRFTISRDNAKDTVYLQMNSLKPEDTAVYYCAAGPTGLLSKKATPADYDYWGQGTQVTVSS |
| 130 | QVQLQQSGGGSVQAGGSLRLSCTISGRTLSDYTMGWFRQRPGKEREFVAAISWSGAYTKYADS VKGRFTISRDNAKNTVALQMNSLKPEDTAVYYCAAGPTGLLSKKATPADYDYWGQGTQVTVSS |
| 131 | QVQLQQSGGGSVQAGGSLRLSCTISGRTLSDYTMGWFRQRPGKEREFVAAISWSGAYTKYADS VKGRFTISRDNAKNTVFLQMNSLKPEDTAVYYCAAGPTGLLSKKATPADYDYWGQGTQVTVSS |
| 132 | QVQLQQSGGGSVQAGGSLRLSCTISGRTLSDYTMGWFRQRPGKEREFVAAISWSGAYTKYADS VKGRFTISRDNAKNTVWLQMNSLKPEDTAVYYCAAGPTGLLSKKATPADYDYWGQGTQVTVSS |
| 133 | QVQLQQSGGGSVQAGGSLRLSCTISGRTLSDYTMGWFRQRPGKEREFVAAISWSGAYTKYADS VKGRFTISRDNAKNTVYLQMNNLKPEDTAVYYCAAGPTGLLSKKATPADYDYWGQGTQVTVSS |
| 134 | QVQLQQSGGGSVQAGGSLRLSCTISGRTLSDYTMGWFRQRPGKEREFVAAISWSGAYTKYADS VKGRFTISRDNAKNTVYLQMNTLKPEDTAVYYCAAGPTGLLSKKATPADYDYWGQGTQVTVSS |
| 135 | QVQLQQSGGGSVQAGGSLRLSCTISGRTLSDYTMGWFRQRPGKEREFVAAISWSGAYTKYADS VKGRFTISRDNAKNTVYLQMNQLKPEDTAVYYCAAGPTGLLSKKATPADYDYWGQGTQVTVSS |
| 137 | QVQLQQSGGGSVQAGGSLRLSCTISGRTLSDYTMGWFRQRPGKEREFVAAISWSGAYTKYADS VKGRFTISRDNAKNTVYLQMNSLKPEDTAAYYCAAGPTGLLSKKATPADYDYWGQGTQVTVSS |
| 139 | QVQLQQSGGGSVQAGGSLRLSCTISGRTLSDYTMGWFRQRPGKEREFVAAISWSGAYTKYADS VKGRFTISRDNAKNTVYLQMNSLKPEDTAIYYCAAGPTGLLSKKATPADYDYWGQGTQVTVSS |
| 140 | QVQLQQSGGGSVQAGGSLRLSCTISGRTLSDYTMGWFRQRPGKEREFVAAISWSGAYTKYADS VKGRFTISRDNAKNTVYLQMNSLKPEDTALYYCAAGPTGLLSKKATPADYDYWGQGTQVTVSS |
| 141 | QVQLQQSGGGSVQAGGSLRLSCAISGRTLSDYTMGWFRQRPGKEREFVAAISWSGAYTKYADS VKGRFTISRDNAKNTVYLQMNSLKPEDTAIYYCAAGPTGLLSKKATPADYDYWGQGTQVTVSS |
| 142 | QVQLQQSGGGSVQAGGSLRLSCTISGRTLSDYTMGWFRQAPGKEREFVAAISWSGAYTKYADS VKGRFTISRDNAKNTVYLQMNSLKPEDTAIYYCAAGPTGLLSKKATPADYDYWGQGTQVTVSS |
| 143 | QVQLQQSGGGSVQAGGSLRLSCTISGRTLSDYTMGWFRQRPGKERELVAAISWSGAYTKYADS VKGRFTISRDNAKNTVYLQMNSLKPEDTAIYYCAAGPTGLLSKKATPADYDYWGQGTQVTVSS |
| 144 | QVQLQQSGGGSVQAGGSLRLSCTSSGRTLSDFTMAWFRQRPGRAREFVAAISWSGAYTYYTDS VKGRFTISRDNAKNTVFLQMQSLKPEDTAIYYCAAGPTGLLSRKTTPADYQYWGQGTQVTVSS |
| 145 | QVQLVESGGGSVQAGGSLSLSCTISGRTLSDFTMAWFRQRPGKEREFVAAISWSGAYTYYTDSV KGRFTISRDNAKNTVYLQMQSLKPEDTAVYYCAAGPTGLLSRKTTPADYQYWGQGTQVTVSS |
| 146 | QVQLQQSGGGSVQAGGSLRLSCTISGRTLSDFTMAWFRQRPGKEREFVAAISWSGAYTYYADSV KGRFTISRDNAKNTVYLQMNSLKPEDTAVYYCAAGPTGLLSRKTTPADYQYWGQGTQVTVSS |
| 147 | QVQLQQSGGGSVQAGGSLRLSCTISGRTLSDFTMAWFRQRPGKEREFVAAVSWSGAYTKYADS VKGRFTISRDNAKNTVYLQMNSLKPEDTAVYYCAAGPTGLLSKKATPPDYDYWGQGTQVTVSS |
| 148 | QVQLQQSGGGSVQAGGSLRLSCTISGRTLSDFTMGWFRQRPGKEREFVAAISWSGAYTKYADS VKGRFTISRDNAKNTVYLQMNSLKPEDTAVYYCAAGPTGLLSKKTTPADYDYWGQGTQVTVSS |
| 149 | QVQLQQSGGGSVQAGGSLTLSCTVSGRTLSDFTMGWFRQRPGKELEFVAAISWSGAYTKYADS VKGRFTISRDNAKNTVWLQMNSLKPEDTAVYYCAAGPTGPLSKKTTPADYDYWGQGTQVTVSS |
| 150 | QVQLQQSGGGSVQAGGSLRLSCTSSGRTLSDFTMGWFRQRPGKEREFVAAISWSGAYTKYADS VKGRFTISRDNAKNTVWLQMNSLKPEDTAVYYCAAGPTGLLSKKTTPADYDYWGQGTQVTVSS |
| 151 | QVQLQQSGGGSVQAGGSLRLSCTVSGRTLSDFTMGWFRQRPGKEREFVAAISWSGAYTKYADS VKGRFTISRDNAKNTVFLQMNSLKPEDTALYYCAAGPTGLLSKKTTPADYDYWGQGTQVTVSS |
| 156 | QVQLQQSGGGSVQAGGSLRLSCTISGRTSINPYMAWFRQRPGKEREFVAAISSGGVYTYYADSV KGRFTISRDNAKNTVYLQMNSLKPEDTAVYYCAADFRRGGNWNVDPFRYDYDYWGQGTQVTV SS |
| 158 | QVQLQQSGGGSVQAGGSLRLSCTISGRTFSSYAMGWFRQRPGKEREFVAAINWSSGSTYYADS VKGRFTISRDNAKNTVYLQMNSLKPEDTAVYYCAAGYQINSGNYNFKDYEYDYWGQGTQVTVS S |
| 159 | VDAKFDKEAQEAFYEILHLPNLTEEQRNAFIQSLKDDPSGSKAILAEAKKVNDAQAPK |
| 160 | VDAKFDKEAYRALAEIARLPNLTEEQRAAFIQSLKDDPSGSKAILAEAKKVNDAQAPK |
| 194 | EVQLQQSGGGSVQAGGSLRLSCTISG |
| 195 | EVQLQQSGGGSVQAGGSLRLSCTISGRTLSDYTMGWFRQRPGKEREFVAAISWSGAYTKYADSV KGRFTISRDNAKNTVYLQMNSLKPEDTAVYYCAAGPTGLLSKKATPADYDYWGQGTQVTVSS |
| 196 | GPTGLLSQKTTPADYDY |
| 197 | QVQLQQSGGGSVQAGGSLRLSCTISGRTLSDFTMGWFRQRPGKEREFVAAISWSGAYTKYADS VKGRFTISRDNAKNTVYLQMNSLKPEDTAVYYCAAGPTGLLSQKTTPADYDYWGQGTQVTVSS |
| 198 | EVQLQQSGGGSVQAGGSLRLSCTISGRTLSDFTMGWFRQRPGKEREFVAAISWSGAYTKYADSV KGRFTISRDNAKNTVYLQMNSLKPEDTAVYYCAAGPTGLLSQKTTPADYDYWGQGTQVTVSS |
| 200 | QVQLQQSGGGSVQAGGSLRLSCTSSGRTLSDFTMGWFRQIPGKEREFVAAISWSGAYTKYADSV KGRFTISRDNAKQTVWLQMNSLKPEDTAVYYCAAGPTGLLSKKTTPADYDYWGQGTQVTVSS |
| 201 | QVQLQQSGGGSVQAGGSLRLSCTSSGRTLSDFTMGWFRQRPGKDLEFVAAISWSGAYTKYADS VKGRFTISRDNAKNTVWLQMNSLKPEDTAIYYCAAGPTGLLSKKTTPADYDYWGQGTQVTVSS |
| 202 | QVQLQQSGGGSVQAGGSLRLSCTVSGRTLSDFTMGWFRQRPGKELEFVAAISWSGAYTKYADS VKGRFTISRDNAKNTVWLQMNSLKPEDTAVYYCAAGPTGLLSKKTTPADYDYWGQGTQVTVSS |
| 203 | QVQLQQSGGGSVQAGGSLTLSCTVSGRTLSDFTMGWFRQRPGKELEFVAAISWSGAYTKYADS VKGRFTISRDNAKNTVWLQMNSLKPEDTAVYYCAAGPTGLLSKKTTPADYDYWGQGTQVTVSS |
| 204 | QVQLQQSGGGSVQAGGSLTLSCTVSGRTLSDFTMGWFRQRPGKELEFVAAISWSGAYTKYADS VKGRFTISRDNAKNTVWLQMNSLKPEDTAVYYCAAGPTGLLSQKTTPADYDYWGQGTQVTVSS |
| 205 | QVQLQQSGGGSVQAGGSLRLSCTSSGRTLSDFTMGWFRQIPGKEREFVAAISWSGAYTKYADSV KGRFTISRDNAKQTVWLQMNSLKPEDTAVYYCAAGPTGLLSKKTTPADYDYWGQGTQVTVSS |
| 219 | EVQLQQSGGGSVQAGGSLRLSCTISGRTLSDFTMGWFRQRPGKEREFVAAISWSGAYTKYADSV KGRFTISRDNAKNTVYLQMNSLKPEDTAVYYCAAGPTGLLSKKTTPADYDYWGQGTQVTVSS |
| 221 | EVQLQQSGGGSVQAGGSLTLSCTVSGRTLSDFTMGWFRQRPGKELEFVAAISWSGAYTKYADSV KGRFTISRDNAKNTVWLQMNSLKPEDTAVYYCAAGPTGPLSKKTTPADYDYWGQGTQVTVSS |
| 224 | YADSVKGRFTISFDNAKNTVYLQMNSLKPEDTAVYYCAA |
| 225 | YADSVKGRFTISHDNAKNTVYLQMNSLKPEDTAVYYCAA |
| 226 | YADSVKGRFTISKDNAKNTVYLQMNSLKPEDTAVYYCAA |
| 227 | YADSVKGRFTISQDNAKNTVYLQM NSLKPEDT A VYYCAA |
| 228 | YADSVKGRFTISYDNAKNTVYLQMNSLKPEDTAVYYCAA |
| 229 | YADSVKGRFTISFDNAKNTVYLQMNSLKPEDTAVYYCAA |
| 230 | YADSVKGRFTISHDNAKNTVYLQMNSLKPEDTAVYYCAA |
| 231 | YADSVKGRFTISKDNAKNTVYLQMNSLKPEDTAVYYCAA |
| 232 | YADSVKGRFTISQDNAKNTVYLQM NSLKPEDT A VYYCAA |
| 233 | Y ADSVKG RFTISYD NAKNTVYLQM NSLKPEDT A VYYCAA |
| 234 | YADSVKGRFTISFDNAKNTVYLQMNSLKPEDTAVYYCAA |
| 235 | YADSVKGRFTISHDNAKNTVYLQMNSLKPEDTAVYYCAA |
| 236 | YADSVKGRFTISKDNAKNTVYLQMNSLKPEDTAVYYCAA |
| 237 | YADSVKGRFTISQDNAKNTVYLQM NSLKPEDT A VYYCAA |
| 238 | YADSVKGRFTISYDNAKNTVYLQMNSLKPEDTAVYYCAA |
| 239 | YADSVKGRFTISFDNAKNTVYLQMNSLKPEDTAVYYCAA |
| 240 | YADSVKGRFTISHDNAKNTVYLQMNSLKPEDTAVYYCAA |
| 241 | YADSVKGRFTISKDNAKNTVYLQMNSLKPEDTAVYYCAA |
| 242 | YADSVKGRFTISQDNAKNTVYLQM NSLKPEDT A VYYCAA |
| 243 | Y ADSVKG RFTISYD NAKNTVYLQM NSLKPEDT A VYYCAA |

### REFERENCES

Thompson et al, Nucleic Acids Research, 22: 4673-4680 (1994)
Kabat et al., J. Immunol. 147(5), 1709-1719 (1991)
WO2003080655A1
WO2016079033A1
S Hjerten, Biochim Biophys Acta 79(2), 393-398 (1964)
US6602990
US7396467
WO2019137869A1
WO2018011600A1
Schmitz et al, Structure 21, 1214-1224 (2013)
Fleetwood et al., Cell. Mol. Life Sci. 70:1081-1093 (2013)
Mitchell & Colwell, Proteins 86, 697-706 (2018)
Adams R. Et al, MABS VOL. 8, NO. 7, 1336-1346 (2016)
WO2023174900A1

## Claims

1. A polypeptide comprising a heavy chain variable domain (VH) variant, such as a single domain antibody (sdAb) variant, comprising a plurality of antigen-binding regions and a framework comprising framework region 1 (FWR1), framework region 2 (FWR2) and framework region 3 (FWR3), wherein the VH variant has an amino acid sequence comprising a plurality of mutations compared to i) a native camelid framework as defined in any one of SEQ ID NO: 152, SEQ ID NO: 155 or SEQ ID NO: 157, or ii) a synthetic framework as defined in any one of SEQ ID NO: 72 or SEQ ID NO:73, in which viii and at least eight of the other following criteria i) to xi) are fulfilled:
i) the residue in Kabat position 19 is R, S, K or T;
ii) the residue in Kabat position 23 is T, V, A or S, preferably T;
iii) the residue in Kabat position 24 is I, V, A, R, N, D, Q, E, H, L, K, F, T, Y or S;
iv) the residue in Kabat position 40 is selected from R, I, T or K, preferably R;
v) the residue in Kabat position 43 is R, K, Q, E or G, preferably R or K;
vi) the residue in Kabat position 44 is E, Q, A, D or G;
vii) the residue in Kabat position 45 is R, I or L, preferably R;
viii) the residue in Kabat position 71 is F, H, K, Q, or Y;
ix) the residue in Kabat position 76 is N or Q;
x) the residue in Kabat position 79 is Y, W or F; and
xi) the residue in Kabat position 89 V or I.

2. The polypeptide according to any one of the preceding claims, wherein the VH variant has a framework region 1 (FWR1) comprising an amino acid sequence according to
X₁X₂QLX₅X₆SGGGX₁₁VQX₁₄GGSLX₁₉LSCX₂₃X₂₄SG (SEQ ID NO: 1)
wherein, independently,
X₁ is Q, V, D or E, preferably Q or E;
X₂ is V or D, preferably V;
X₈ is Q, V or E, preferably Q;
X₆ is Q or E, preferably Q;
X₁₁ is S or L, preferably S;
X₁₄ is A, S, or T, preferably A;
X₁₉ is R, S, K or T, preferably R or T;
X₂₃ is T, V, A or S, preferably T; and
X₂₄ is I, V, A, R, N, D, Q, E, H, L, K, F, T, Y or S, preferably I;
a framework region 2 (FWR2) comprising an amino acid sequence according to
WFRQX_{S}PGX₈X₉X₁₀EX₁₂VA (SEQ ID NO: 2)
wherein, independently,
X₈ is R, I, T or K, preferably R;
X₈ is R, K, Q, E or G, preferably K or R;
X₉ is E, Q, A, D or G;
X₁₀ is R, I or L, preferably R; and
X₁₂ is F or L, preferably F; and
a framework region 3 (FWR3) comprising an amino acid sequence according to
YX₂X₃X₄VX₆GRFTIS X₁₃DNAKX₁₈TX₂₀X₂₁LQMNX₂₆LKPEDTAX₃₄YYCAA (SEQ ID NO: 3)
wherein, independently,
X₂ is A, T, N, Q or S, preferably A;
X₃ is D or S, preferably D;
X₄ is S or A, preferably S;
X₆ is K or A, preferably K;
X₁₃ is F, H, K, Q, or Y;
X₁₈ is N or Q, preferably N;
X₂₀ is V or A, preferably V;
X₂₁ is Y, F or W;
X₂₆ is N, S, T or Q, preferably S; and
X₃₄ is V or I.

3. The polypeptide according to any one of any one of the preceding claims, wherein the VH variant comprises a framework region 4 (FWR4) comprising the amino acid sequence
WGQGTQVTVSS (SEQ ID NO: 71).

4. The polypeptide according to any one of the claims 1-2, wherein each of FWR1, FWR2 and FWR3 has at least 80 %, such as at least 85%, 90% or 95%, identity to the amino acid sequence of a corresponding framework region of SEQ ID NO: 80, wherein for the purpose of determining sequence identity, the Kabat positions 14, 19, 23, 24, 40, 43, 44, 45,47, 60, 71, 76, 79, 82b and 89, and optionally position 6, are omitted.

5. The polypeptide according to any one of the claims 1-4, wherein the amino acid sequence of the VH variant further fulfils any one, such as more than one, such as all, of the following:
the residue in Kabat position 6 is Q or E;
the residue in Kabat position 14 is A, S or T;
the residue in Kabat position 47 is F or L, preferably F;
the residue in Kabat position 60 is A, T, N, Q or S, preferably A or S;
the residue in Kabat position 82b is N, S, T or Q.

6. The polypeptide according to any one of the preceding claims, wherein framework region 1 (FWR1) has an amino acid sequence having at least 70 %, such as at least 75%, such as at least 80 %, such as at least 85 % identity to a sequence selected from the group consisting of SEQ ID NOs:4-22, and/or framework region 2 (FWR2) has an amino acid sequence having at least 70 %, such as at least 75%, such as at least 80 %, such as at least 85 % identity to a sequence selected from the group consisting of SEQ ID NOs:23-24 and 26-41 and/or framework region 3 (FWR3) has an amino acid sequence having at least 70 %, such as at least 75%, such as at least 80 %, such as at least 85 % identity to a sequence selected from the group consisting of SEQ ID NOs:45-70 and 224-228.

7. The polypeptide according to any one of the preceding claims, which is an antigen binding polypeptide, such as a single-chain polypeptide, and wherein said VH variant is a variable domain of a heavy chain of a heavy chain antibody (VHH) variant.

8. The polypeptide according to any one of the preceding claims, wherein the VH variant has a framework corresponding to the framework of a VH variant selected from the group consisting of SEQ ID NOs: 76, 78, 80-114, 118-121, 126-127, 130-135, 137, 139-151, 156, 158 and 229-243.

9. The polypeptide according to any one of the preceding claims, wherein the polypeptide antigen-binding regions comprises three CDRs, CDR1, CDR2 and CDR3, which bind a target entity, and wherein the residue in Kabat position 71 has been selected to enhance target entity binding of the CDRs, wherein the residue in Kabat position 71 has been selected as F, H, K, Q, or Y.

10. A multimeric polypeptide comprising at least two moieties, each moiety being a VH variant as defined in any one of the preceding claims, said moieties optionally being joined by a peptide linker.

11. A fusion protein comprising at least one polypeptide according to any one of claims 1-8 or a multimer according to claim 10, and at least one further polypeptide moiety.

12. The fusion protein according to claim 113, wherein the further polypeptide moiety comprises a sequence having least 80 % identity such as at least 85 % or at least 90 % identity to SEQ ID NO: 159 or to SEQ ID NO: 160.

13. An adsorbent material comprising the polypeptide according to any one of claims 1-8, the multimer of claim 10 or the fusion protein of claims 11-12 coupled to a solid support, wherein the support material is optionally selected from a particle, a bead, a fiber, a fibrous membrane, a filter, a sheet, a porous monolith, a chip, a plate, and a well, such as a nonwoven fibrous matrix, or comprises agar or agarose or a derivative thereof, such as crosslinked agarose, , and wherein the support optionally is a chromatography matrix.

14. A method of separation, comprising the steps of
(a) providing an adsorbent material according to claim 12, wherein the VH variant of the polypeptide has a binding affinity for a target entity,
(b) contacting the adsorbent material with a liquid sample comprising said target entity under conditions allowing the target entity to bind to the polypeptide,
(c) optionally washing the adsorbent material,
(d) eluting the target entity from the adsorbent material, and
(e) cleaning the adsorbent material with a cleaning liquid, such as an alkaline cleaning liquid, wherein the cleaning liquid preferably comprises from 0.05 to 0.5 M NaOH, such as 0.1-0.5 M NaOH and wherein steps (a)-(e) are repeated at least 10 times.

15. A method for selecting a variant of a polypeptide, multimer or the fusion protein of any one of claims 1-12 that is optimized for target entity binding, the method comprising:
(a) providing a polypeptide, multimer or fusion protein of any one of claims 1-12, the polypeptide, multimer or fusion protein comprising antigen-binding regions consisting of three CDRs, CDR1, CDR2 and CDR3, which bind a target entity;
(c) providing variants of the polypeptide, multimer or fusion protein, wherein the residue in Kabat position 71 for each variant has been altered or selected to be one of F, H, K, Q, Y or R;
(c) evaluating, for each variant, the binding affinity or binding selectivity for the target entity; and
(d) selecting the variant that provides the highest binding affinity or binding selectivity for the target entity as the variant optimized for target entity binding.
